(19) Europäisches Patentamt
European Patent Office
Office européen des brevets

(11) EP 2 925 323 B1

# (12) EUROPEAN PATENT SPECIFICATION

(45) Date of publication and mention of the grant of the patent:
**17.05.2017 Bulletin 2017/20**

(21) Application number: **13798637.8**

(22) Date of filing: **27.11.2013**

(51) Int Cl.:
***C07D 401/14*** *(2006.01)* ***A61K 31/4725*** *(2006.01)*
***A61P 11/06*** *(2006.01)* ***A61K 9/14*** *(2006.01)*

(86) International application number:
**PCT/EP2013/074803**

(87) International publication number:
**WO 2014/083026 (05.06.2014 Gazette 2014/23)**

## (54) AN INHIBITOR OF BRUTON'S TYROSINE KINASE

HEMMER DER BRUTON-TYROSINKINASE

INHIBITEUR DE TYROSINE KINASE DE BRUTON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2012 US 201261731510 P**

(43) Date of publication of application:
**07.10.2015 Bulletin 2015/41**

(73) Proprietor: **F. Hoffmann-La Roche AG**
**4070 Basel (CH)**

(72) Inventors:
- **IYER, Raman Mahadevan**
  **Piscataway, New Jersey 08854 (US)**
- **LAINE, Dramane Ibrahim**
  **Hoboken, New Jersey 07030 (US)**
- **LOPEZ-TAPIA, Francisco Javier**
  **Ewa Beach, Hawaii 96706 (US)**
- **PHILLIPS, Jonathan E.**
  **Simi Valley,**
  **California 93065 (US)**
- **STEVENSON, Christopher**
  **Lindfield**
  **West Sussex RH16 2DA (GB)**

(74) Representative: **Sauer, Frank et al**
**F. Hoffmann-La Roche AG**
**Patent Department**
**Grenzacherstrasse 124**
**4070 Basel (CH)**

(56) References cited:
**WO-A1-2013/024078 US-A1- 2012 040 949**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 925 323 B1

**Description**

**FIELD OF THE INVENTION**

The present application relates to the use of a novel compound which inhibits Btk and is useful for the treatment of auto-immune and inflammatory diseases caused by aberrant B-cell activation.

**BACKGROUND OF THE INVENTION**

Protein kinases constitute one of the largest families of human enzymes and regulate many different signaling processes by adding phosphate groups to proteins (T. Hunter, Cell 1987 50:823-829). Specifically, tyrosine kinases phosphorylate proteins on the phenolic moiety of tyrosine residues. The tyrosine kinase family includes members that control cell growth, migration, and differentiation. Abnormal kinase activity has been implicated in a variety of human diseases including cancers, autoimmune and inflammatory diseases. Since protein kinases are among the key regulators of cell signaling they provide a target to modulate cellular function with small molecular kinase inhibitors and thus make good drug design targets. In addition to treatment of kinase-mediated disease processes, selective and efficacious inhibitors of kinase activity are also useful for investigation of cell signaling processes and identification of other cellular targets of therapeutic interest.

There is good evidence that B-cells play a key role in the pathogenesis of autoimmune and/or inflammatory disease. Protein-based therapeutics that deplete B cells such as Rituxan are effective against autoantibody-driven inflammatory diseases such as rheumatoid arthritis (Rastetter et al. Annu Rev Med 2004 55:477). Therefore inhibitors of the protein kinases that play a role in B-cell activation should be useful therapeutics for B-cell mediated disease pathology such as autoantibody production.

Signaling through the B-cell receptor (BCR) controls a range of B-cell responses including proliferation and differentiation into mature antibody producing cells. The BCR is a key regulatory point for B-cell activity and aberrant signaling can cause deregulated B-cell proliferation and formation of pathogenic autoantibodies that lead to multiple autoimmune and/or inflammatory diseases. Bruton's Tyrosine Kinase (Btk) is a non-BCR associated kinase that is membrane proximal and immediately downstream from BCR. Lack of Btk has been shown to block BCR signaling and therefore inhibition of Btk could be a useful therapeutic approach to block B-cell mediated disease processes.

Btk is a member of the Tec family of tyrosine kinases, and has been shown to be a critical regulator of early B-cell development and mature B-cell activation and survival (Khan et al. Immunity 1995 3:283; Ellmeier et al. J. Exp. Med. 2000 192:1611). Mutation of Btk in humans leads to the condition X-linked agammaglobulinemia (XLA) (reviewed in Rosen et al. New Eng. J. Med. 1995 333:431 and Lindvall et al. Immunol. Rev. 2005 203:200). These patients are immunocompromised and show impaired maturation of B-cells, decreased immunoglobulin and peripheral B-cell levels, diminished T-cell independent immune responses as well as attenuated calcium mobilization following BCR stimulation.

Evidence for a role for Btk in autoimmune and inflammatory diseases has also been provided by Btk-deficient mouse models. In preclinical murine models of systemic lupus erythematosus (SLE), Btk-deficient mice show marked amelioration of disease progression. In addition, Btk-deficient mice are resistant to collagen-induced arthritis (Jansson and Holmdahl Clin. Exp. Immunol. 1993 94:459). A selective Btk inhibitor has been demonstrated dose-dependent efficacy in a mouse arthritis model (Z. Pan et al., Chem. Med Chem. 2007 2:58-61).

Btk is also expressed by cells other than B-cells that may be involved in disease processes. For example, Btk is expressed by mast cells and Btk-deficient bone marrow derived mast cells demonstrate impaired antigen induced degranulation (Iwaki et al. J. Biol. Chem. 2005 280:40261). Studies have highlighted the pivotal role of Btk in the regulation of other cell types critically involved in the development of allergic asthma. In particular, the absence of Btk severely impairs FcεRI- dependent mast cell responses with regard to the production of allergic cytokines and degranulation (Iyer et al. J. Biol. Chem. 2011 286:9503-13; Hata et al. J. Exp. Med. 1998 187:1235-47). It has also been shown that Btk was required for IgE-mediated activation of human basophils (MacGlashan et al. Int. Immunopharmacol 2011 11:475-9). This shows Btk could be useful to treat pathological mast cells responses such as allergy and asthma.

Innate immune signaling is being increasingly recognized as playing an important role in the natural history of asthma both in terms of its possible role in the onset of asthma (Wu et al., Am J Respir Crit Care Med 2008; 178:1123-1129) and in acute exacerbations of the disease (Johnston et al., Br. Med. J. 1995; 310:1225-1229). Pattern recognition receptors such as Toll-like receptors (TLRs) have been shown to play a role in allergen sensitization as well as airway inflammation, remodeling, and hyper-responsiveness (Hammad et al Nat Med. 2009;15(4):410-6.). Btk is involved in innate signaling downstream of these receptors. For instance, Btk has been shown to be required for the full activation of TLR signaling in vitro (Liu et al., Nat Immunol. 2011; 12(5):416-24). *In vivo,* Btk has been shown to play a critical role in initiating TLR3 signaling (Lee et al., Proc Natl Acad Sci U S A. 2012; 109(15):5791-6). These data indicate that Btk could be useful to treat TLR, and in particular TLR3, mediated innate immune response disease processes.

Also monocytes from XLA patients, in which Btk activity is absent, show decreased TNF alpha production

following stimulation (Horwood et al. J Exp Med 197:1603, 2003). Therefore TNF alpha mediated inflammation could be modulated by small molecular Btk inhibitors. Also, Btk has been reported to play a role in apoptosis (Islam and Smith Immunol. Rev. 2000 178:49) and thus Btk inhibitors would be useful for the treatment of certain B-cell lymphomas and leukemias (Feldhahn et al. J. Exp. Med. 2005 201:1837).

## SUMMARY OF THE INVENTION

The present invention is defined in the appended claims. Subject matters which are not encompassed by the scope of the claims do not form part of the present invention. This invention discloses the Btk inhibitor compound 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one, formulations thereof, and uses for the treatment of asthma by inhalation therewith, as described herein below.

The application provides 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for use in treating or ameliorating asthma, or a related condition in a mammal by inhalation.

The invention provides a formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose.

The application provides a compound of Formula I,

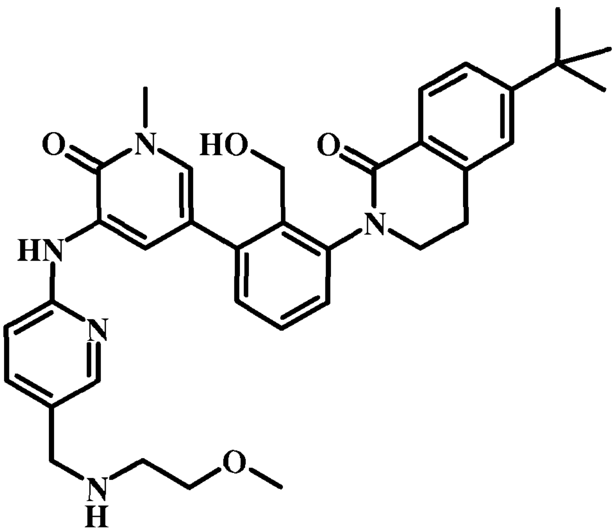

I

or a pharmaceutically acceptable salt thereof.

## IN THE DRAWINGS

**Figure 1:** Powder X-ray diffraction patterns before and after micronization. The top curve is after micronization. The bottom curve is before micronization.

**Figure 2:** Particle size distribution of reference compound 1 for mOVA36.

| | | | |
|---|---|---|---|
| **Particle Name:** | **Accessory Name:** | **Analysis model:** | **Sensitivity:** |
| Fraunholer | Hydro 2000S (A) | General purpose | Normal |
| **Particle RI:** | **Absorption:** | **Size range:** | **Obscuration:** |
| 0.000 | 0 | 0.020 to 2000.000 um | 9.39 % |
| **Dispersant Name:** | **Dispersant RI:** | **Weighted Residual:** | **Result Emulation:** |
| Water | 1.330 | 0.390 % | Off |
| **Concentration:** | **Span :** | **Uniformity:** | **Result units:** |
| 0,0028 %Vol | 1.496 | 0.466 | Volume |

(continued)

| Specific Surface Aren: | Surface Weighted Mean D[3,2]: | Vol. Weighted Mean D[4,3]: | |
|---|---|---|---|
| 2.98 $m^2/g$ | 2.014 um | 3.162 um | |
| d(0.1): 1.226 um | d(0.5): 2.877 um | | d(0.9): 5.530 um |

| Size (µm) | Volume in% | Size (µm) | Volume in % | Size (µm) | Volume in % | Size (µm) | Volume in % | Size (µm) | Volume in % | Size (µm) | Volume in% |
|---|---|---|---|---|---|---|---|---|---|---|---|
| 0.010 | 0.00 | 0.105 | 0.00 | 1.096 | 2.54 | 11.482 | 0.00 | 120.226 | 0.00 | 1258.925 | 0.00 |
| 0.011 | 0.00 | 0.120 | 0.00 | 1.259 | 3.54 | 13.183 | 0.00 | 138.038 | 0.00 | 1445.440 | 0.00 |
| 0.013 | 0.00 | 0.138 | 0.00 | 1.445 | 4.73 | 15.136 | 0.00 | 158.489 | 0.00 | 1659.587 | 0.00 |
| 0.015 | 0.00 | 0.153 | 0.06 | 1.660 | 6.04 | 17.378 | 0.00 | 181.970 | 0.00 | 1305.461 | 0.00 |
| 0.017 | 0.00 | 0.182 | 0.17 | 1.905 | 7.37 | 19.953 | 0.00 | 208.930 | 0.00 | 2187.762 | 0.00 |
| 0.020 | 0.00 | 0.209 | 0.30 | 2.188 | 8.54 | 22.909 | 0.00 | 239.883 | 0.00 | 2511.386 | 0.00 |
| 0.023 | 0.00 | 0.240 | 0.38 | 2.512 | 9.40 | 26.303 | 0.00 | 275.423 | 0.00 | 2894.032 | 0.00 |
| 0.026 | 0.00 | 0.275 | 0.43 | 2.884 | 9.77 | 30.200 | 0.00 | 316.228 | 0.00 | 3311.311 | 0.00 |
| 0.030 | 0.00 | 0.316 | 0.45 | 3.311 | 9.56 | 34.674 | 0.00 | 363.078 | 0.00 | 3801.894 | 0.00 |
| 0.035 | 0.00 | 0.363 | 0.45 | 3.802 | 8.75 | 39.811 | 0.00 | 416.869 | 0.00 | 4365.158 | 0.00 |
| 0.040 | 0.00 | 0.417 | 0.44 | 4.365 | 7.43 | 45.709 | 0.00 | 478.630 | 0.00 | 5011.872 | 0.00 |
| 0.046 | 0.00 | 0.479 | 0.45 | 5.012 | 5.82 | 52.481 | 0.00 | 549.541 | 0.00 | 5754.399 | 0.00 |
| 0.052 | 0.00 | 0.550 | 0.49 | 5.754 | 4.11 | 60.256 | 0.00 | 630.957 | 0.00 | 6606.934 | 0.00 |
| 0.060 | 0.00 | 0.631 | 0.60 | 6.607 | 2.56 | 69.183 | 0.00 | 724.436 | 0.00 | 7585.776 | 0.00 |
| 0.069 | 0.00 | 0.724 | 0.82 | 7.586 | 1.34 | 79.433 | 0.00 | 831.764 | 0.00 | 8709.636 | 0.00 |
| 0.079 | 0.00 | 0.832 | 1.20 | 8.710 | 0.49 | 91.201 | 0.00 | 954.993 | 0.00 | 10000.000 | |
| 0.091 | 0.00 | 0.955 | 1.76 | 10.000 | 0.00 | 104.713 | 0.00 | 1096.478 | 0.00 | | |
| 0.105 | | 1.096 | | 11.482 | | 120.226 | | 1258.925 | | | |

**Figure 3:** Particle size distribution of reference compound 1 for mOVA40

| | | | |
|---|---|---|---|
| **Partide Name:** Fraunhofer | **Accessory Name:** Hydro 20005 (A) | **Analysis model:** General purpose | **Sensitivity:** Normal |
| **Particle RI:** 0.000 | **Absorption:** 0 | **Size range:** 0.020 to 2000.000 um | **Obscuration:** 12.00 % |
| **Dispersant Name:** Water | **Dispersant RI:** 1.330 | **Weighted Residual:** 0.402 % | **Result Emulation.** Off |
| **Concentration:** 0.0039 %viol | **Span:** 1.582 | **Uniformity:** 0.492 | **Result units:** Volume |
| **Specific Surface Area:** 2.52 $m^2$/g | **Surface Weighted Mean D[3,2]:** 2.129 um | **Vol. Weighted Mean D[4,3]:** 3.386 um | |
| d(0,1): 1.205 um | d(0.5): 3.053 um | | d(0,9):6.035 um |

| Size (µm) | Volume in % | Size (µm) | Volume in % | Size (µm) | Volume in % | Size (µm) | Volume in % | Size (µm) | Volume in % | Size (µm) | Volume in % |
|---|---|---|---|---|---|---|---|---|---|---|---|
| 0.010 | 0.00 | 0.105 | 0.00 | 1.096 | 2.41 | 11.482 | 0.01 | 120.226 | 0.00 | 1258.925 | 0.00 |
| 0.011 | 0.00 | 0.120 | 0.00 | 1.259 | 3.22 | 13.183 | 0.00 | 138.038 | 0.00 | 1445.440 | 0.00 |
| 0.013 | 0.00 | 0.138 | 0.00 | 1.445 | 4.19 | 15.136 | 0.00 | 158.489 | 0.00 | 1659.597 | 0.00 |
| 0.015 | 0.00 | 0.158 | 0.00 | 1.660 | 5.31 | 17.378 | 0.00 | 181.970 | 0,00 | 1905.461 | 0.00 |
| 0.017 | 0.00 | 0.182 | 0.05 | 1.905 | 6.49 | 19.953 | 0.00 | 208.930 | 0.00 | 2187.762 | 0.00 |
| 0.020 | 0.00 | 0.209 | 0.17 | 2.188 | 7.63 | 22.909 | 0.00 | 335.883 | 0.00 | 2511.886 | 0.00 |
| 0.023 | 0.00 | 0.240 | 0.27 | 2.512 | 6.58 | 26.303 | 0.00 | 275.423 | 0.00 | 2884.032 | 0.00 |
| 0.025 | 0.00 | 0.275 | 0.35 | 2.884 | 9.18 | 30.200 | 0.00 | 316.228 | 0.00 | 3311.311 | 0.00 |
| 0.030 | 0.00 | 0.316 | 0.41 | 3.311 | 9.30 | 34.674 | 0.00 | 363.078 | 0.00 | 3801.894 | 0.00 |
| 0.035 | 0.00 | 0.363 | 0.47 | 3.802 | 8.87 | 39.611 | 0.00 | 410.309 | 0.00 | 4365.158 | 0.00 |
| 0.040 | 0.00 | 0.417 | 0.52 | 4.365 | 7.92 | 45.709 | 0.00 | 478.630 | 0.00 | 5011.872 | 0.00 |
| 0.045 | 0.00 | 0.479 | 0.56 | 5.012 | 6.56 | 52.431 | 0.00 | 545.541 | 0.00 | 5754.359 | 0.00 |
| 0.052 | 0.00 | 0.550 | 0.67 | 5.754 | 4.98 | 60.256 | 0.00 | 630.957 | 0.00 | 6006.934 | 0.00 |
| 0.060 | 0.00 | 0.631 | 0.80 | 6.607 | 3.43 | 69.183 | 0.00 | 724.436 | 0.00 | 7535.776 | 0.00 |
| 0.060 | 0.00 | 0.724 | 1.02 | 7.586 | 2.06 | 79.433 | 0.00 | 831.764 | 0.00 | 8709.636 | 0.00 |
| 0.079 | 0.00 | 0.832 | 1.34 | 8.710 | 1.04 | 91.201 | 0.00 | 954.993 | 0.00 | 10000.000 | |
| 0.091 | 0.00 | 0.955 | 1.80 | 10.000 | 0.37 | 104.713 | 0.00 | 1096.473 | 0.00 | | |
| 0.105 | | 1.096 | | 11.482 | | 120.226 | | 1258.925 | | | |

## DETAILED DESCRIPTION OF THE INVENTION

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

When any variable occurs more than one time in any moiety or formula depicting and describing compounds employed or claimed in the present application, its definition on each occurrence is independent of its definition at every other occurrence. Also, combinations of substituents and/or variables are permissible only if such compounds result in stable compounds.

The symbols "*" at the end of a bond or "------" drawn through a bond each refer to the point of attachment of a functional group or other chemical moiety to the rest of the molecule of which it is a part. Thus, for example:

MeC(=O)OR$^4$ wherein R$^4$ = 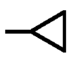 or 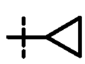 MeC(=O)O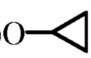 .

A bond drawn into ring system (as opposed to connected at a distinct vertex) indicates that the bond may be attached to any of the suitable ring atoms.

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen atom or a substituent.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Technical and scientific terms used herein have the meaning commonly understood by one of skill in the art to which the present application pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. Materials, reagents and the like to which reference are made in the following description and examples are obtainable from commercial sources, unless otherwise noted.

### Inhibitors of Btk

This application discloses the Btk inhibitor compound 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one (compound 3), formulations thereof, methods of treatment of asthma by inhalation therewith, and uses for the treatment of asthma by inhalation therewith, as described herein.

The present application concerns compound 3 for use in the treatment of asthma of whatever type, etiology or pathogenesis and in particular asthma that is a member selected from the group consisting of atopic asthma, non-atopic asthma, allergic asthma, non-allergic asthma, atopic bronchial IgE-mediated asthma, bronchial asthma, essential asthma, true asthma, intrinsic asthma, extrinsic asthma, bronchitic asthma, emphysematous asthma, exercise-induced asthma, allergen-induced asthma, cold-air induced asthma, occupational asthma, nocturnal asthma, seasonal asthma, cough-variant asthma, chronic asthma, intermittent asthma, mild persistent asthma, moderate persistent asthma, severe persistent asthma, neutrophilic asthma, eosinophilic asthma, mixed asthma, paucigranulocytic asthma, Th2-high asthma,

Th2-low asthma, childhood asthma, pathogen-induced asthma caused by bacterial, fungal, protozoal or viral infection, incipient asthma, wheezy baby syndrome and bronchiolitis.

The invention provides the use of 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for treating or ameliorating asthma, or a related condition in a mammal by inhalation.

The application provides the above compound for use, wherein 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one is administered by inhalation as a dry powder.

The application provides 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for use in treating or ameliorating asthma, or a related condition in a mammal by inhalation , wherein the compound is administered by inhalation as a dry powder.

The application provides the compound 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for use in treating or ameliorating asthma, or a related condition in a mammal by inhalation as a dry powder.

The application provides the use of 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for the preparation of a medicament for treating or ameliorating asthma, or a related condition in a mammal by inhalation as a dry powder.

The application provides the use of the compound 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one for the treatment of asthma by inhalation as a dry powder.

The application provides a formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose.

The application provides a formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and Lactohale LH 300.

The application provides a formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and Respitose ML 006.

The application provides a formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose; micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and Lactohale LH 300; or micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and Respitose ML 006.

The application provides micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose for use in treating or ameliorating asthma, or a related condition in a mammal by inhalation.

The application provides the use of the formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose for treating or ameliorating asthma, or a related condition in a mammal by inhalation of a pharmacologically effective amount of the formulation.

The application provides a compound of Formula I,

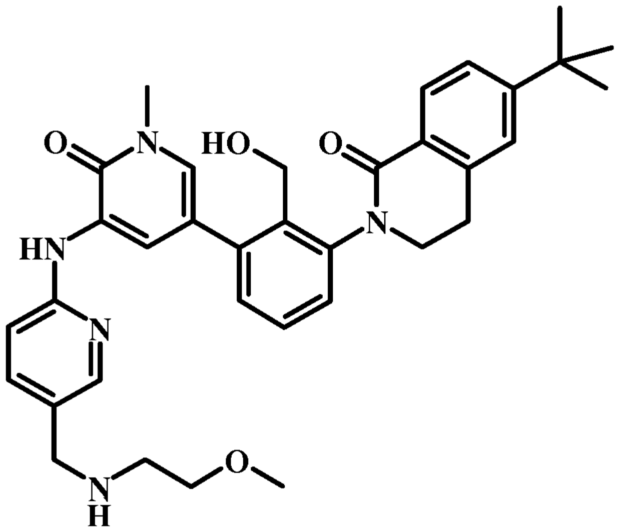

I

or a pharmaceutically acceptable salt thereof.

The application provides a combination of the compound 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one, or formulations thereof, and any one or more of the therapeutic agents selected from the group consisting of:

(a) 5-Lipoxygenase (5-LO) inhibitors or 5-lipoxygenase activating protein (FLAP) antagonists,
(b) Leukotriene antagonists (LTRAs) including antagonists of $LTB_4$, $LTC_4$, $LTD_4$, and $LTE_4$,
(c) Histamine receptor antagonists including H1 and H3 antagonists,
(d) $\alpha_1$- and $\alpha_2$-adrenoceptor agonist vasoconstrictor sympathomimetic agents for decongestant use,
(e) short or long acting $\beta_2$ agonists,
(f) PDE inhibitors, e.g. PDE3, PDE4 and PDE5 inhibitors
(g) Theophylline
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-$\alpha$) agents,
(m) Adhesion molecule inhibitors including VLA-4 antagonists,
(n) Kinin-$B_1$- and $B_2$-receptor antagonists,
(o) Immunosuppressive agents,
(p) Inhibitors of matrix metalloproteases (MMPs),
(q) Tachykinin $NK_1$, $NK_2$ and $NK_3$ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NF$\kappa$B pathway, e.g. IKK inhibitors,
(w) modulators of cytokine signaling pathways such as p38 MAP kinase or syk kinase,
(x) Agents that can be classed as mucolytics or anti-tussive,
(y) Antibiotics,
(z) HDAC inhibitors,
(aa) PI3 kinase inhibitors,
(bb) CXCR2 antagonists. and
(cc) muscarinic antagonists.

The application provides a use of any of the above compound or formulations in the manufacture of a medicament for the treatment of an inflammatory disorder.

The application provides a use of any of the above compound or formulations in the manufacture of a medicament

for the treatment of an autoimmune disorder.

The application provides a use of any of the above compounds or formulations in the manufacture of a medicament for the treatment of asthma by inhalation.

The application provides the above compound or formulations for use in the treatment of an inflammatory disorder.

The application provides the above compound or formulations for use in the treatment of an autoimmune disorder.

The application provides the above compound or formulations for the treatment of asthma by inhalation.

Compounds and Preparation

Examples of representative compounds encompassed by the present application and within the scope of the application are provided in the following Table. These examples and preparations which follow are provided to enable those skilled in the art to more clearly understand and to practice the present invention.

In general, the nomenclature used in this Application is based on AUTONOMTM v.4.0, a Beilstein Institute computerized system for the generation of IUPAC systematic nomenclature. If there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

**Discovery of Compounds 1, 2 and 3**

The design of inhaled drugs exerting their actions topically in the lung generally requires the compounds to be potent and to have adequate pharmacokinetic properties (i.e. high clearance and low availability) to minimize systemic exposure (Tayab et al, Expert Opin. Drug Deliv. (2005), 2(3), 519-532). For drugs intended to be delivered with a dry-powder inhaler (DPI), a suitable stable and crystalline solid form needs to be identified (Selby et al, Future Med. Chem. (2011), 3(13), 1679-1701). In addition, poorly soluble compounds have the potential to be associated with unwanted adverse effects in toxicity studies (Forbes et al, Adv Drug Del. Rev (2011), 63, 69-87). With these concepts in mind, the Roche collection of Btk inhibitors was screened to identify suitable candidates for inhalation.

Firstly, a set of 33 compounds was selected from a Roche collection of 1918 Btk compounds according to their high potency (FRET $IC_{50}$ < 50 nM and/or HWB $IC_{50}$ < 1 μM) and high solubility (LYSA sol > 200 μg/mL). Compound with low clearance (Rat Cl< 40 mL/min/kg or Rat microsomal CLint < 25 μl/min/mg protein), a flag in the GSH assay or low availability in the compound bank (<10 mg) were also excluded.

Secondly, after acquiring additional data on the set of 33 compounds (e.g. missing rat PK data, crystallinity information, solubility in stimulated lung fluid), a sub-set of 11 compounds was further selected mainly according to their high in-vivo clearance in the rat (Rat Cl> 40 mL/min/kg).

Finally, based on their crystallinity and high potency in in-vitro assays relevant to asthma, reference compounds 1, or 2 and compound 3 were selected as potential inhaled candidates

TABLE I depicts examples of compounds disclosed in the present application:

TABLE I.

| Compound | Nomenclature | Structure |
|---|---|---|
| 1 (reference) | 6-tert-Butyl-8-fluoro-2-{3-hydroxymethyl-4-[1-methyl-5-(1'-methyl-1',2',3',4',5',6'-hexahydro-[3,4']bipyridinyl-6-ylamino)-6-oxo-1,6-dihydro-pyridazin-3-yl]-pyridin-2-yl}-2H-phthalazin-1-one | 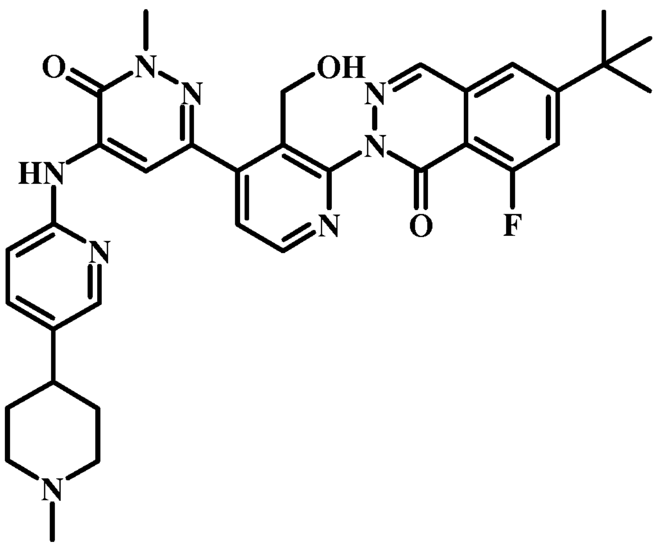 |

(continued)

| Compound | Nomenclature | Structure |
|---|---|---|
| 2 (reference) | 2-(2-{3-[5-(5-Azetidin-1-ylmethyl-1-methyl-1H-pyrazol-3-ylamino)-1-methyl-6-oxo-1,6-dihydro-pyridazin-3-yl]-2-hydroxymethylphenyl}-8-fluoro-1-oxo-1,2-dihydro-isoquinolin-6-yl)-2-methyl-propionitrile | 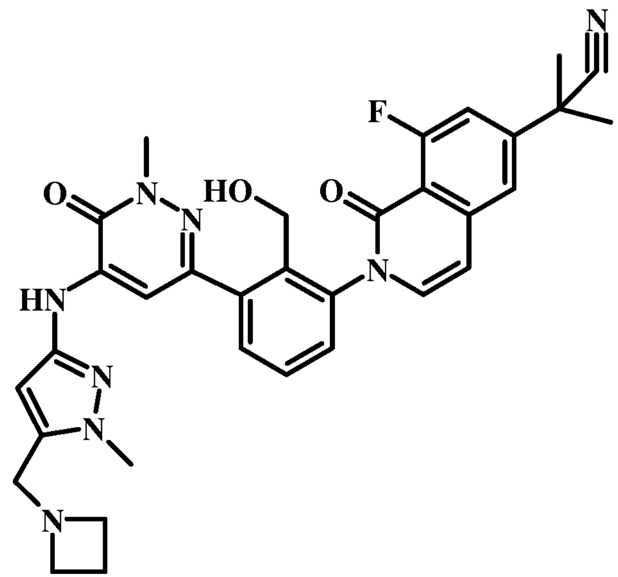 |
| 3 | 6-tert-Butyl-2-[2-hydroxymethyl-3-(5-{5-[(2-methoxy-ethylamino)-methyl]-pyridin-2-ylamino}-1-methyl-6-oxo-1,6-dihydro-pyridin-3-yl)-phenyl]-3,4-dihydro-2H-isoquinolin-1-one | 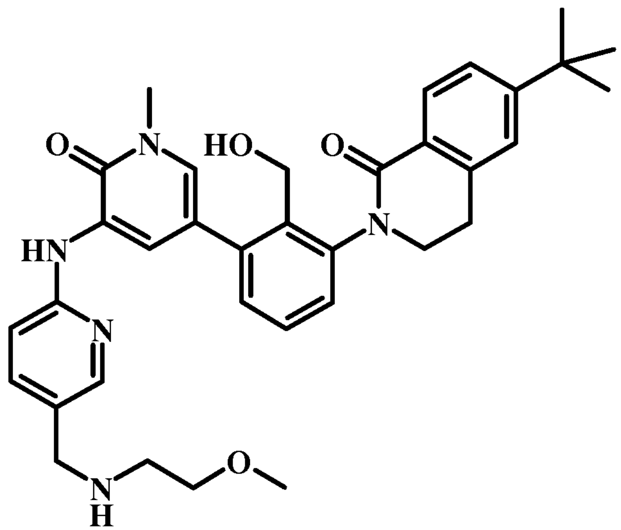 |

General Synthetic Schemes

The compounds of the present application may be prepared by any conventional means. Suitable processes for synthesizing these compounds are provided in the examples. Generally, compounds of the application may be prepared according to the schemes below.

Reference Compound 1 was prepared in seven steps from commercially available starting materials as shown in Scheme 1.

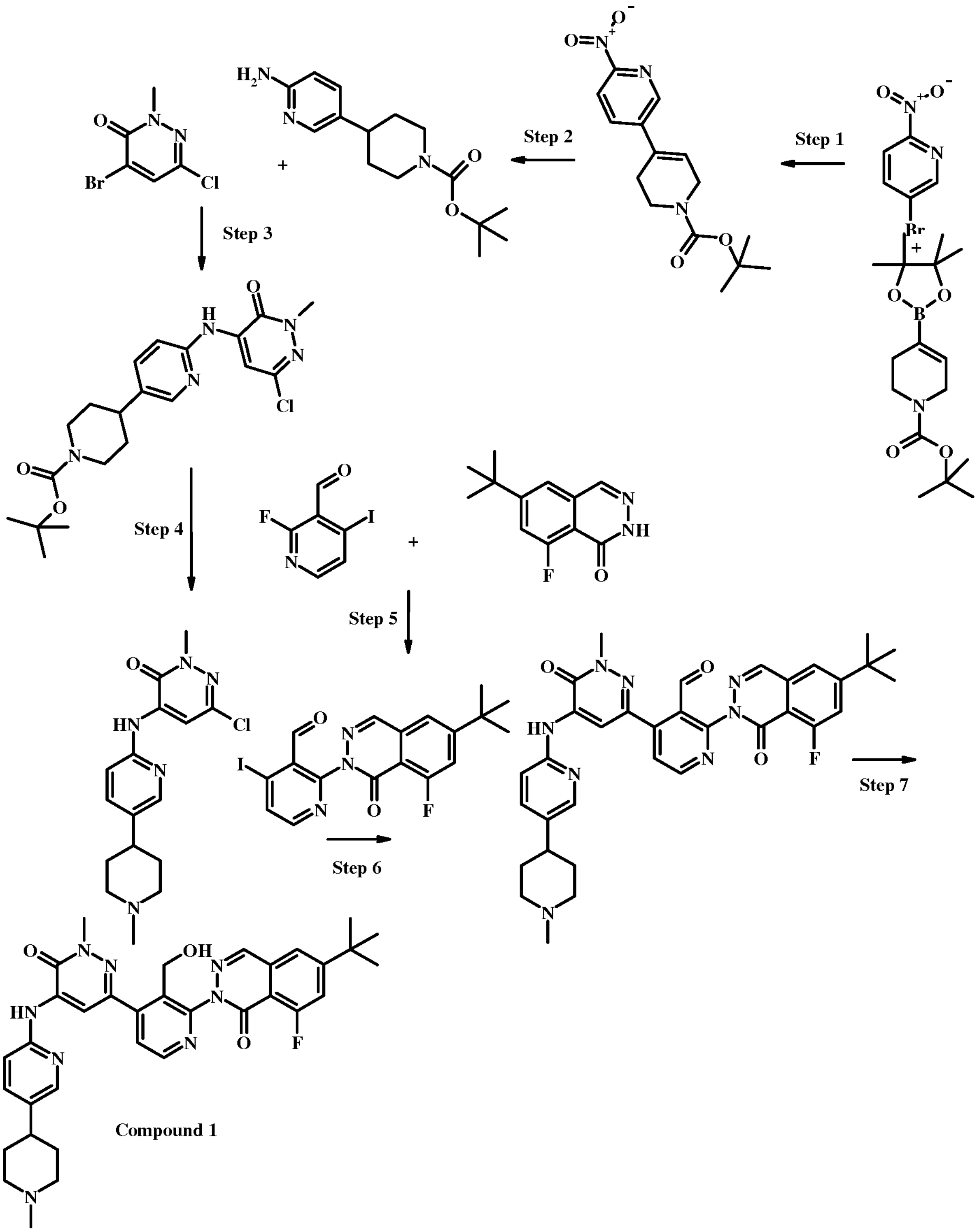


Compound 1

**Scheme 1**: Preparation of reference Compound 1

Reference Compound 2 was prepared in nineteen steps from commercially available starting materials as shown in Schemes 2, 3 and 4.

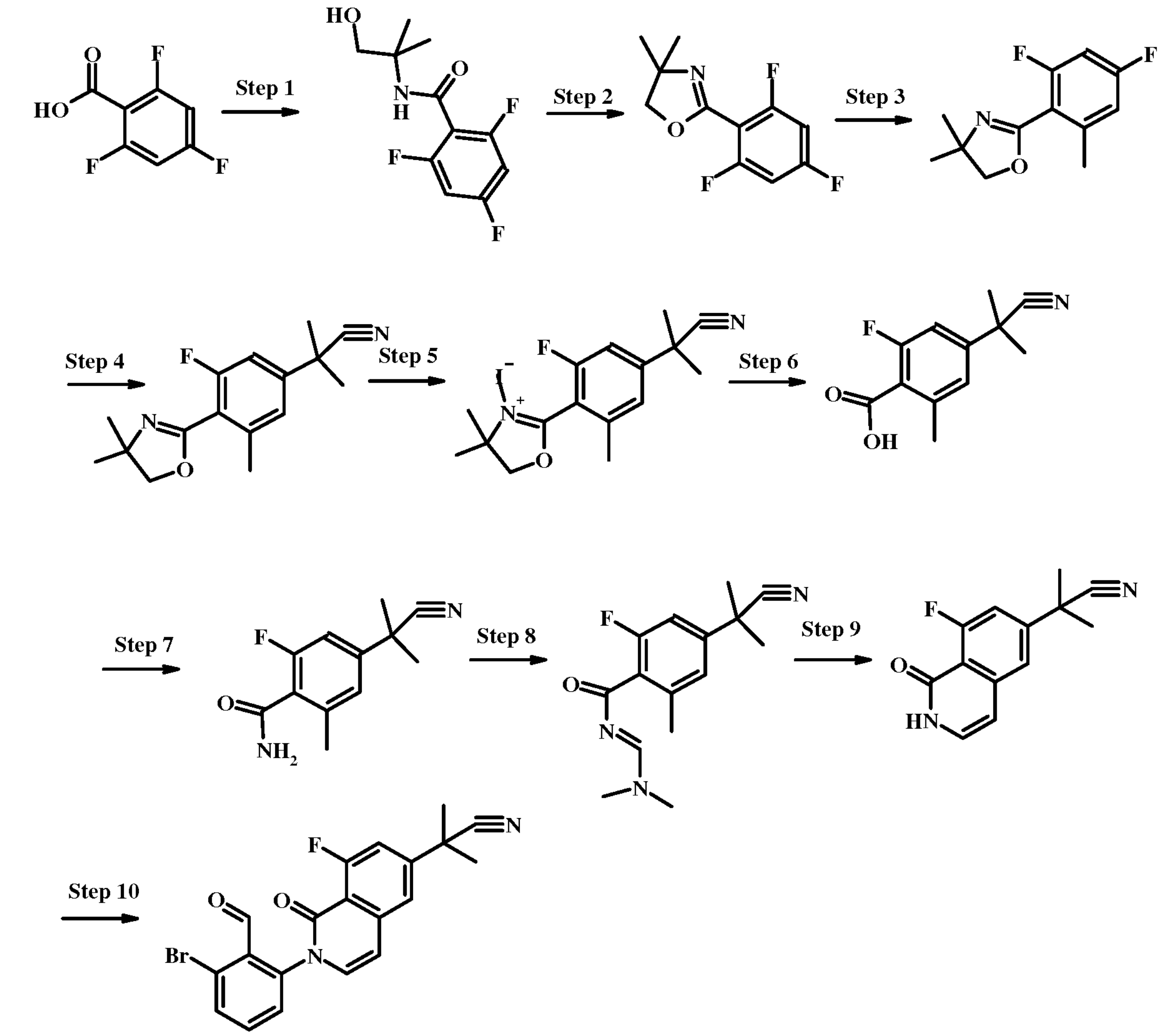


**Scheme 2**: Preparation of reference compound 2 (steps 1-10)

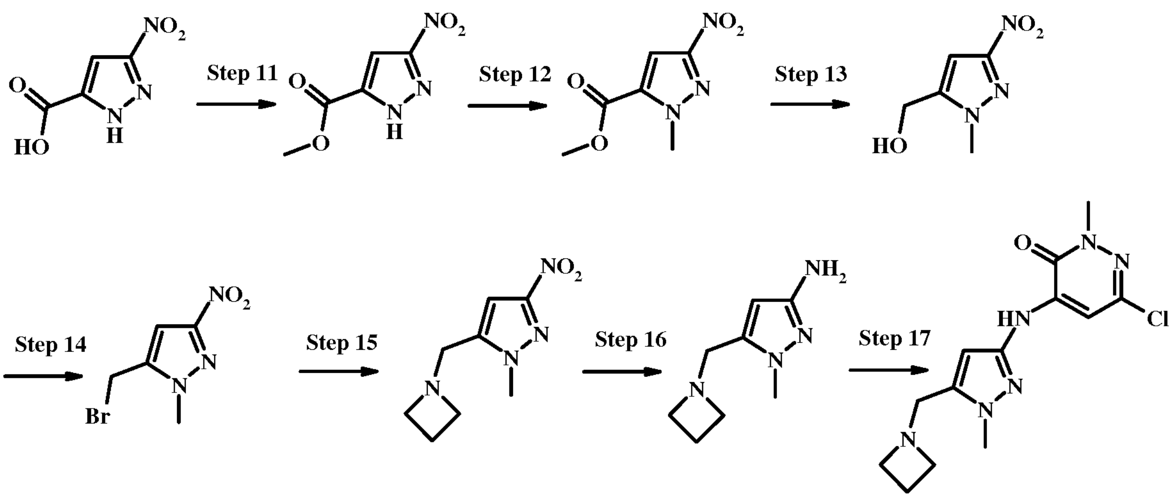

**Scheme 3**: Preparation of reference compound 2 (steps 11-17)

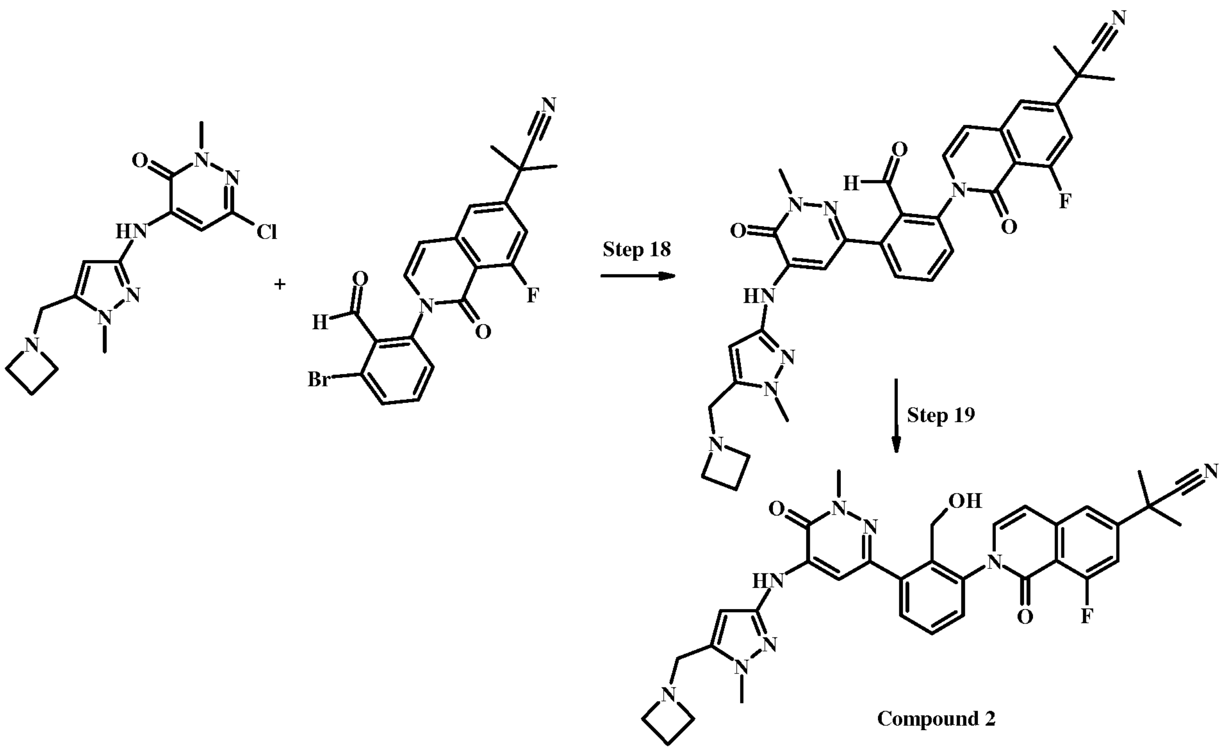


Compound 2

**Scheme 4**: Preparation of reference compound 2 (steps 18-19)

Compound 3 was prepared in 10 steps from compound A and commercially available starting materials as shown in Schemes 5, 6 and 7. The preparation of 5-bromo-l-methyl-3-(5-(morpholine-4-carbonyl)pyridin-2-ylamino)pyridin-2(1H)-one A has been described in the literature (WO2011140488(A1)).

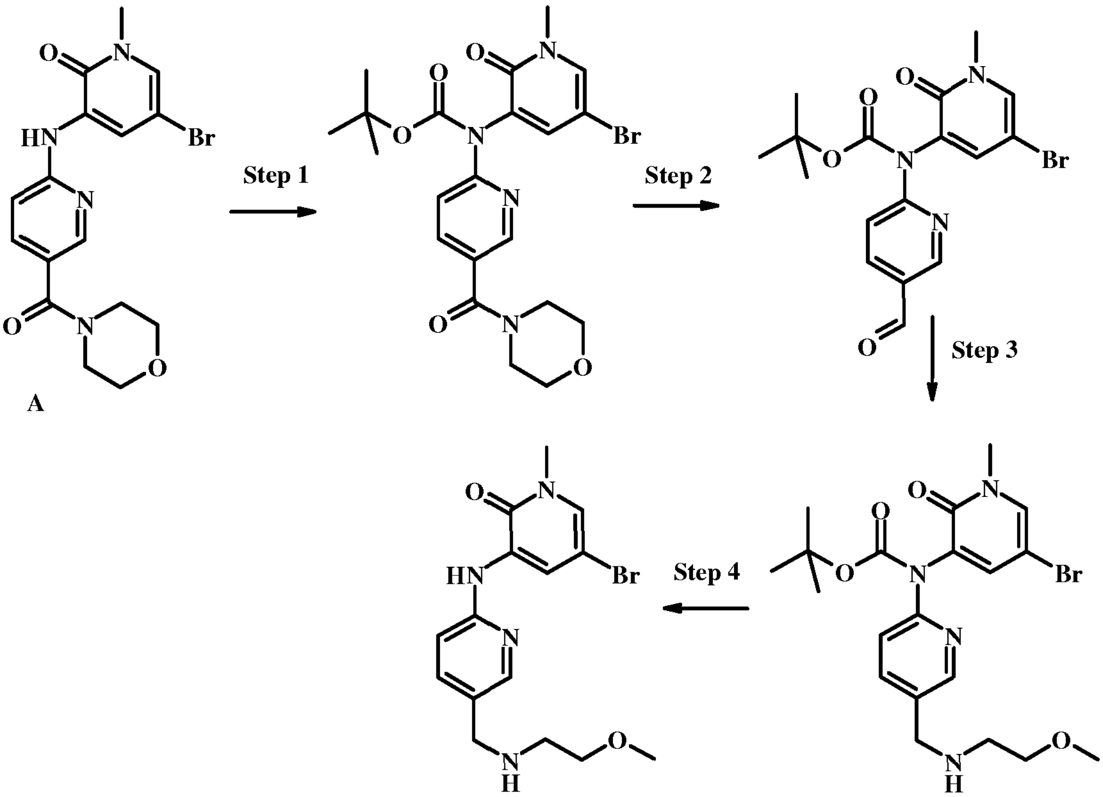


**Scheme 5**: Preparation of Compound 3 (steps 1-5)

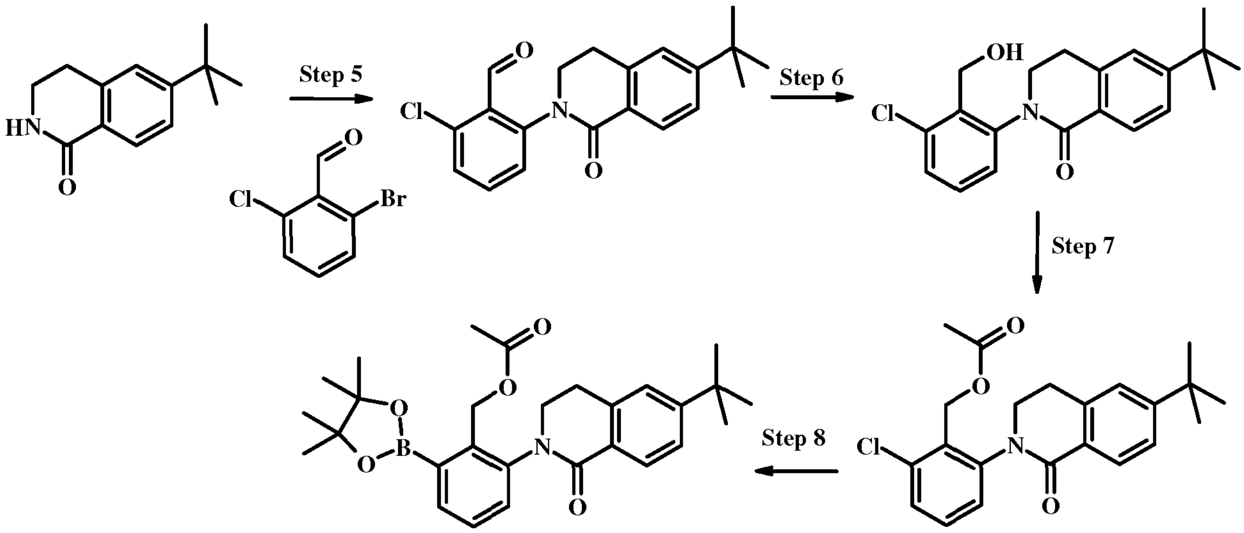


**Scheme 6:** Preparation of Compound 3 (steps 5-8)

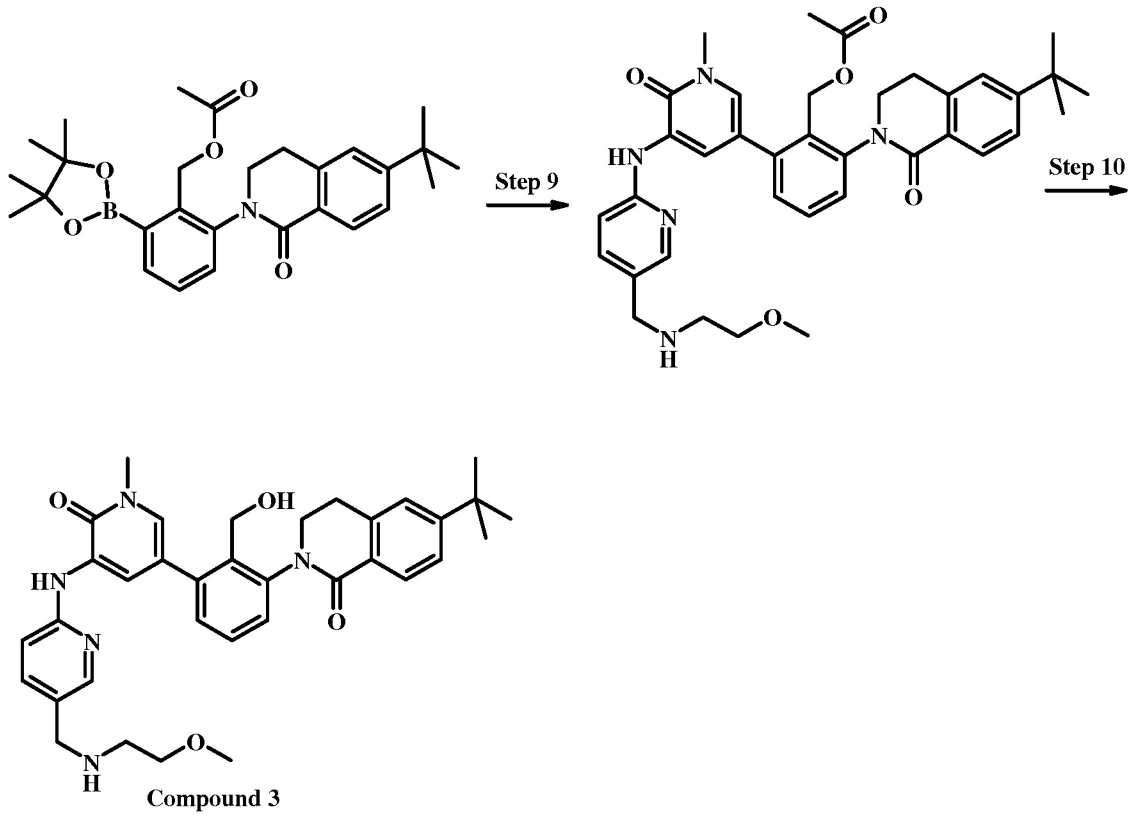


Compound 3

**Scheme 7**: Preparation of Compound 3 (steps 9-10)

Pharmaceutical Compositions and Administration

The compounds of the present application may be formulated in a wide variety of administration dosage forms and carriers suitable for delivery by inhalation. The preferred manner of administration is via inhalation using a convenient daily dosing regimen which can be adjusted according to the degree of affliction and the patient's response to the active ingredient.

A compound or compounds of the present application, as well as their pharmaceutically useable salts, together with one or more conventional excipients, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as powders

The term "excipient" as used herein refers to a compound that is useful in preparing a pharmaceutical composition, generally safe, non-toxic and neither biologically nor otherwise undesirable, and includes excipients that are acceptable for veterinary use as well as human pharmaceutical use. The compounds of this application can be administered alone but will generally be administered in admixture with one or more suitable pharmaceutical excipients, diluents or carriers selected with regard to the intended route of administration and standard pharmaceutical practice.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

A "pharmaceutically acceptable salt" form of an active ingredient may also initially confer a desirable pharmacokinetic property on the active ingredient which were absent in the non-salt form, and may even positively affect the pharmacodynamics of the active ingredient with respect to its therapeutic activity in the body. The phrase "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, and sulfuric acid; or formed with organic acids such as acetic acid, fumaric acid, succinic acid, maleic acid, tartaric acid, L(+)-tartaric acid, D(-)-tartaric acid, citric acid, 1-hydroxy-2-naphtoic acid,

propionic acid, p-toluensulfonic acid, cinnamic acid, 1,2-dibenzenacrylic acid, pamoic acid, pyromellitic acid, sebacic acid, mesitylene sulfonic acid, biphenyldisulfonic acid, 2-naphthalenesulfonic acid salicylic acid, stearic acid, muconic acid, stearic acid, lauric acid (+)-camphoric acid, ascorbic acid, and glutaric acid; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, and N-methylglucamine.

In another aspect, the application is directed to a dosage form adapted for administration to a patient by inhalation, for example as a dry powder, an aerosol, a suspension, or a solution composition. In one embodiment, the application is directed to a dosage form adapted for administration to a patient by inhalation as a dry powder. In a further embodiment, the application is directed to a dosage form adapted for administration to a patient by inhalation via a nebulizer.

Dry powder compositions for delivery to the lung by inhalation typically comprise a polymorph or salt of the application as a finely divided powder together with one or more pharmaceutically-acceptable excipients as finely divided powders. Pharmaceutically- acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (e.g. micronized) compound can be defined by a $D(_{50})$ value of about 1 to about 10 microns (for example as measured using laser diffraction).

The dry powder may be administered to the patient via a reservoir dry powder inhaler (RDPI) having a reservoir suitable for storing multiple (un-metered doses) of medicament in dry powder form. RDPIs typically include a means for metering each medicament dose from the reservoir to a delivery position. For example, the metering means may comprise a metering cup, which is movable from a first position where the cup may be filled with medicament from the reservoir to a second position where the metered medicament dose is made available to the patient for inhalation.

Alternatively, the dry powder may be presented in capsules (e.g. gelatin or plastic), cartridges, or blister packs for use in a multi-dose dry powder inhaler (MDPI). MDPIs are inhalers wherein the medicament is comprised within a multi-dose pack containing (or otherwise carrying) multiple defined doses (or parts thereof) of medicament. When the dry powder is presented as a blister pack, it comprises multiple blisters for containment of the medicament in dry powder form. The blisters are typically arranged in regular fashion for ease of release of the medicament therefrom. For example, the blisters may be arranged in a generally circular fashion on a disc-form blister pack, or the blisters may be elongate in form, for example comprising a strip or a tape. Each capsule, cartridge, or blister may, for example, contain between 20μg-10mg of the polymorph or salt of the application.

Aerosols may be formed by suspending or dissolving a polymorph or salt of the application in a liquefied propellant. Suitable propellants include halocarbons, hydrocarbons, and other liquified gases. Representative propellants include: trichlorofluoromethane (propellant 11), dichlorofluoromethane (propellant 12), dichlorotetratluoroethave (propellant 114), tetrafluoroethane (H FA- 134a), 1, 1-ditluoroethane (HFA-152a), difluoromethane (HFA-32), pentafluoroethane (HFA-12), heptafluoropropane (HFA-227a), perfluoropropane, perfluorobutane, perfluoropentane, butane, isobutane, and pentane. Aerosols comprising a polymorph or salt of the application will typically be administered to a patient via a metered dose inhaler (MDI). Such devices are known to those skilled in the art.

The aerosol may contain additional pharmaceutically-acceptable excipients typically used with MDIs such as surfactants, lubricants, cosolvents and other excipients to improve the physical stability of the formulation, to improve valve performance, to improve solubility, or to improve taste.

There is thus provided as a further aspect of the application a pharmaceutical aerosol formulation comprising a polymorph or salt of the application and a fluorocarbon or hydrogen-containing chlorofluorocarbon as propellant, optionally in combination with a surfactant and/or a cosolvent.

According to another aspect of the application, there is provided a pharmaceutical aerosol formulation wherein the propellant is selected from 1, 1, 1, 2-tetrafluoroethane, 1, 1, 1, 2, 3, 3, 3-heptafluoro-n-propane and mixtures thereof.

The formulations of the application may be buffered by the addition of suitable buffering agents.

Capsules and cartridges for use in an inhaler or insufflator, of for example gelatin, may be formulated containing a powder mix for inhalation of a polymorph or salt of the application and a suitable powder base such as lactose or starch. Each capsule or cartridge may generally contain from 20μg to 10mg of the polymorph or salt of the application. Alternatively, the polymorph or salt of the application may be presented without excipients such as lactose.

The proportion of the active polymorph or salt in the local compositions according to the application depends on the precise type of formulation to be prepared but will generally be within the range of from 0.001 to 10% by weight. Generally, for most types of preparations, the proportion used will be within the range of from 0.005 to 1 %, for example from 0.01 to 0.5%. However, in powders for inhalation or insufflation the proportion used will normally be within the range of from 0.1 to 5%.

Aerosol formulations are preferably arranged so that each metered dose or "puff" of aerosol contains from 20μg to 10mg, preferably from 20μg to 2000μg, more preferably from about 20μg to 500μg of a polymorph or salt of the application. Administration may be once daily or several times daily, for example 2, 3, 4 or 8 times, giving for example

1, 2 or 3 doses each time. The overall daily dose with an aerosol will be within the range from 10 μg to 10mg, preferably from 20μg to 2000 μg. The overall daily dose and the metered dose delivered by capsules and cartridges in an inhaler or insufflator will generally be double that delivered with aerosol formulations.

In the case of suspension aerosol formulations, the particle size of the particulate (e.g., micronized) drug should be such as to permit inhalation of substantially all the drug into the lungs upon administration of the aerosol formulation and will thus be less than 100 microns, desirably less than 20 microns, and in particular in the range of from 1 to 10 microns, such as from 1 to 5 microns, more preferably from 2 to 3 microns.

The formulations of the application may be prepared by dispersal or dissolution of the medicament and a polymorph or salt of the application in the selected propellant in an appropriate container, for example, with the aid of sonication or a high-shear mixer. The process is desirably carried out under controlled humidity conditions.

The chemical and physical stability and the pharmaceutical acceptability of the aerosol formulations according to the application may be determined by techniques well known to those skilled in the art. Thus, for example, the chemical stability of the components may be determined by HPLC assay, for example, after prolonged storage of the product. Physical stability data may be gained from other conventional analytical techniques such as, for example, by leak testing, by valve delivery assay (average shot weights per actuation), by dose reproducibility assay (active ingredient per actuation) and spray distribution analysis.

The stability of the suspension aerosol formulations according to the application may be measured by conventional techniques, for example, by measuring flocculation size distribution using a back light scattering instrument or by measuring particle size distribution by cascade impaction or by the "twin impinger" analytical process. As used herein reference to the "twin impinger" assay means "Determination of the deposition of the emitted dose in pressurized inhalations using apparatus A" as defined in British Pharmacopaeia 1988, pages A204-207, Appendix XVII C. Such techniques enable the "respirable fraction" of the aerosol formulations to be calculated. One method used to calculate the "respirable fraction" is by reference to "fine particle fraction" which is the amount of active ingredient collected in the lower impingement chamber per actuation expressed as a percentage of the total amount of active ingredient delivered per actuation using the twin impinger method described above.

The term "metered dose inhaler" or MDI means a unit comprising a can, a secured cap covering the can and a formulation metering valve situated in the cap. MDI system includes a suitable channeling device. Suitable channeling devices comprise for example, a valve actuator and a cylindrical or cone-like passage through which medicament may be delivered from the filled canister via the metering valve to the nose or mouth of a patient such as a mouthpiece actuator. MDI canisters generally comprise a container capable of withstanding the vapor pressure of the propellant used such as a plastic or plastic-coated glass bottle or preferably a metal can, for example, aluminum or an alloy thereof which may optionally be anodized, lacquer-coated and/or plastic-coated (for example WO96/32099 wherein part or all of the internal surfaces are coated with one or more fluorocarbon polymers optionally in combination with one or more non-fluorocarbon polymers), which container is closed with a metering valve. The cap may be secured onto the can via ultrasonic welding, screw fitting or crimping. MDIs taught herein may be prepared by methods of the art (e.g. see Byron, above and WO96/32099). Preferably the canister is fitted with a cap assembly, wherein a drug-metering valve is situated in the cap, and said cap is crimped in place.

In one embodiment of the application the metallic internal surface of the can is coated with a fluoropolymer, more preferably blended with a non-fluoropolymer. In another embodiment of the application the metallic internal surface of the can is coated with a polymer blend of polytetrafluoroethylene (PTFE) and polyethersulfone (PES). In a further embodiment of the application the whole of the metallic internal surface of the can is coated with a polymer blend of polytetrafluoroethylene (PTFE) and polyethersulfone (PES). The metering valves are designed to deliver a metered amount of the formulation per actuation and incorporate a gasket to prevent leakage of propellant through the valve. The gasket may comprise any suitable elastomeric material such as, for example, low density polyethylene, chlorobutyl, bromobutyl, EPDM, black and white butadiene- acrylonitrile rubbers, butyl rubber and neoprene. Suitable valves are commercially available from manufacturers well known in the aerosol industry, for example, from Valois, France (e.g. DF10, DF30, DF60), Bespak pic, UK (e.g. BK300, BK357) and 3M-Neotechnic Ltd, UK (e.g. Spraymiser™). In various embodiments, the MDIs may also be used in conjunction with other structures such as, without limitation, overwrap packages for storing and containing the MDIs, including those described in U.S. Patent Nos. 6, 119,853; 6, 179, 118; 6,315, 112; 6,352, 152; 6,390,291 ; and 6,679,374, as well as dose counter units such as, those described in U.S. Patent Nos. 6,360,739 and 6,431,168.

Conventional bulk manufacturing methods and machinery well known to those skilled in the art of pharmaceutical aerosol manufacture may be employed for the preparation of large-scale batches for the commercial production of filled canisters. Thus, for example, in one bulk manufacturing method for preparing suspension aerosol formulations a metering valve is crimped onto an aluminum can to form an empty canister. The particulate medicament is added to a charge vessel and liquefied propellant together with the optional excipients is pressure filled through the charge vessel into a manufacturing vessel. The drug suspension is mixed before recirculation to a filling machine and an aliquot of the drug suspension is then filled through the metering valve into the canister. In one example bulk manufacturing method for

preparing solution aerosol formulations a metering valve is crimped onto an aluminum can to form an empty canister. The liquefied propellant together with the optional excipients and the dissolved medicament is pressure filled through the charge vessel into a manufacturing vessel.

In an alternative process, an aliquot of the liquefied formulation is added to an open canister under conditions which are sufficiently cold to ensure the formulation does not vaporize, and then a metering valve crimped onto the canister.

Typically, in batches prepared for pharmaceutical use, each filled canister is check- weighed, coded with a batch number and packed into a tray for storage before release testing. Suspensions and solutions comprising a polymorph or salt of the application may also be administered to a patient via a nebulizer. The solvent or suspension agent utilized for nebulization may be any pharmaceutically-acceptable liquid such as water, aqueous saline, alcohols or glycols, e.g., ethanol, isopropyl alcohol, glycerol, propylene glycol, polyethylene glycol, etc. or mixtures thereof. Saline solutions utilize salts which display little or no pharmacological activity after administration. Both organic salts, such as alkali metal or ammonium halogen salts, e.g., sodium chloride, potassium chloride or organic salts, such as potassium, sodium and ammonium salts or organic acids, e.g., ascorbic acid, citric acid, acetic acid, tartaric acid, etc. may be used for this purpose.

Other pharmaceutically-acceptable excipients may be added to the suspension or solution. The polymorph or salt of the application may be stabilized by the addition of an inorganic acid, e.g., hydrochloric acid, nitric acid, sulphuric acid and/or phosphoric acid; an organic acid, e.g., ascorbic acid, citric acid, acetic acid, and tartaric acid, etc., a complexing agent such as EDTA or citric acid and salts thereof; or an antioxidant such as antioxidant such as vitamin E or ascorbic acid. These may be used alone or together to stabilize the polymorph or salt of the application. Preservatives may be added such as benzalkonium chloride or benzoic acid and salts thereof. Surfactant may be added particularly to improve the physical stability of suspensions. These include lecithin, disodium dioctylsulphosuccinate, oleic acid and sorbitan esters.

## Indications and Methods of Use

As described herein, the reference compounds 1, and 2, and compound 3 inhibit Bruton's tyrosine kinase (Btk). Activation of Btk by upstream kinases results in activation of phospholipase-Cγ which, in turn, stimulates release of pro-inflammatory mediators. Reference compounds 1, and 2, and compound 3 are useful in the treatment of asthma. The present application further discloses pharmaceutical compositions or formulations containing the reference compounds 1, and 2, or compound 3, admixed with a pharmaceutically acceptable carrier, excipients, or diluents useful in the treatment of asthma for delivery by inhalation.

The application provides a method of treating a patient having asthma responsive to inhibition of Btk activity via inhalation of reference compounds 1, and 2, or compound 3, as described herein.

The application provides the use of reference compounds 1, 2, or compound 3, as described herein, for treating a patient having asthma responsive to inhibition of Btk activity via inhalation. The application provides the use of reference compounds 1, and 2, or compound 3, as described herein, for the manufacture of a medicament for treating a patient having asthma responsive to inhibition of Btk activity via inhalation.

The application provides the reference compounds 1, and 2, or compound 3, as described herein, for use in treating a patient having asthma responsive to inhibition of Btk activity via inhalation.

## Combination Treatment

For the avoidance of doubt, references herein to "treatment" include references to curative, palliative and prophylactic treatment.

According to another embodiment of the present application, reference compounds 1, and 2 or compound 3 of the application or compositions thereof, can also be used as a combination with one or more additional therapeutic agents to be co-administered to a patient to obtain some particularly desired therapeutic end result such as the treatment of pathophysiologically-relevant disease processes including (i) bronchoconstriction, (ii) inflammation, (iii) allergy, (iv) airway remodeling, (v) signs and symptoms such as breathlessness, cough.

As used herein, the terms "co-administration", "co-administered" and "in combination with", referring reference compounds 1, and 2 or compound 3 of the application and one or more other therapeutic agents, is intended to mean, and does refer to and include the following:

- simultaneous administration of such combination of reference compounds 1, and 2 or compound 3 of the application and therapeutic agent(s) to a patient in need of treatment, when such components are formulated together into a single dosage form which releases said components at substantially the same time to said patient,

- substantially simultaneous administration of such combination of reference compounds 1, and 2 or compound 3 of the application and therapeutic agent(s) to a patient in need of treatment, when such components are formulated apart from each other into separate dosage forms which are taken at substantially the same time by said patient, whereupon said components are released at substantially the same time to said patient,
- sequential administration of such combination of reference compounds 1, and 2 or compound 3 of the application and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of reference compounds 1, and 2 or compound 3 of the application and therapeutic agent(s) to a patient in need of treatment, when such components are formulated together into a single dosage form which releases said components in a controlled manner whereupon they are concurrently, consecutively, and/or overlapingly administered at the same and/or different times by said patient, where each part may be administered by either the same or different route.

Suitable examples of other therapeutic agents which may be used in combination with reference compounds 1, and 2, or compound 3, or pharmaceutically acceptable salts thereof, derived forms or compositions thereof, include:

(a) 5-Lipoxygenase (5-LO) inhibitors or 5-lipoxygenase activating protein (FLAP) antagonists,
(b) Leukotriene antagonists (LTRAs) including antagonists of $LTB_4$, $LTC_4$, $LTD_4$, and $LTE_4$,
(c) Histamine receptor antagonists including H1 and H3 antagonists,
(d) $\alpha_1$- and $\alpha_2$-adrenoceptor agonist vasoconstrictor sympathomimetic agents for decongestant use,
(e) short or long acting $\beta_2$ agonists,
(f) PDE inhibitors, e.g. PDE3, PDE4 and PDE5 inhibitors
(g) Theophylline
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-$\alpha$) agents,
(m) Adhesion molecule inhibitors including VLA-4 antagonists,
(n) Kinin-$B_1$- and $B_2$-receptor antagonists,
(o) Immunosuppressive agents,
(p) Inhibitors of matrix metalloproteases (MMPs),
(q) Tachykinin $NK_1$, $NK_2$ and $NK_3$ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NF$\kappa$B pathway, e.g. IKK inhibitors,
(w) modulators of cytokine signaling pathways such as p38 MAP kinase or syk kinase,
(x) Agents that can be classed as mucolytics or anti-tussive,
(y) Antibiotics,
(z) HDAC inhibitors,
(aa) PI3 kinase inhibitors,
(bb) CXCR2 antagonists. and
(cc) muscarinic antagonists.

The application provides the combination of the reference compounds 1, and 2, or compound 3 with :

- H3 antagonists,
- $\beta_2$ agonists,
- Muscarinic antagonists
- PDE4 inhibitors,
- Steroids, especially glucocorticosteroids,
- Adenosine A2a receptor agonists,

- Modulators of cytokine signaling pathways such as p38 MAP kinase or syk kinase, or,
- Leukotriene antagonists (LTRAs) including antagonists of $LTB_4$, $LTC_4$, $LTD_4$, and $LTE_4$.

The application provides the combination of the reference compounds 1, 2, or compound 3 with:

- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, or
- $\beta_2$ agonists including in particular salbutamol, terbutaline, bambuterol, fenoterol, salmeterol, formoterol, tulobuterol and their salts, or
- Muscarinic antagonists including tiotropium bromide, ipratropium bromide, umeclidinium bromide, glycopyrrolate, oxitropium bromide, aclidium bromide, darotropium bromide and PF-3635659.

The application provides the combinations as described above for use in the treatment of asthma.
The application provides the use of the combinations as described above for the treatment of asthma.

## EXAMPLES

General Abbreviations

Commonly used abbreviations include: acetyl (Ac), azo-*bis*-isobutyrylnitrile (AIBN), atmospheres (Atm), 9-borabicyclo[3.3.1]nonane (9-BBN or BBN), 2,2'-bis(diphenylphosphino)-1,1'-binaphthyl (BINAP), *tert*-butoxycarbonyl (Boc), di-tert-butyl pyrocarbonate or boc anhydride ($BOC_2O$), benzyl (Bn), butyl (Bu), Chemical Abstracts Registration Number (CASRN), benzyloxycarbonyl (CBZ or Z), carbonyl diimidazole (CDI), 1,4-diazabicyclo[2.2.2]octane (DABCO), diethylaminosulfur trifluoride (DAST), dibenzylideneacetone (dba), 1,5-diazabicyclo[4.3.0]non-5-ene (DBN), 1,8-diazabicyclo[5.4.0]undec-7-ene (DBU), N,N'-dicyclohexylcarbodiimide (DCC), 1,2-dichloroethane (DCE), dichloromethane (DCM), 2,3-Dichloro-5,6-dicyano-1,4-benzoquinone (DDQ), diethyl azodicarboxylate (DEAD), di-*iso*-propylazodicarboxylate (DIAD), di-*iso*-butylaluminumhydride (DIBAL or DIBAL-H), di-iso-propylethylamine (DIPEA), N,N-dimethyl acetamide (DMA), 4-N,N-dimethylaminopyridine (DMAP), N,N-dimethylformamide (DMF), dimethyl sulfoxide (DMSO), 1,1'-*bis*-(diphenylphosphino)ethane (dppe), 1,1'-*bis*-(diphenylphosphino)ferrocene (dppf), 1-(3-dimethylaminopropyl)-3-ethylcarbodiimide hydrochloride (EDCI), 2-ethoxy-1-ethoxycarbonyl-1,2-dihydroquinoline (EEDQ), ethyl (Et), ethyl acetate (EtOAc), ethanol (EtOH), 2-ethoxy-2*H*-quinoline-1-carboxylic acid ethyl ester (EEDQ), diethyl ether ($Et_2O$) ethyl isopropyl ether (EtOiPr), O-(7-azabenzotriazole-1-yl)-N, N,N'N'-tetramethyluronium hexafluorophosphate acetic acid (HATU), acetic acid (HOAc), 1-N-hydroxybenzotriazole (HOBt), high pressure liquid chromatography (HPLC), *iso*-propanol (IPA), isopropylmagnesium chloride (iPrMgCl), hexamethyl disilazane (HMDS), liquid chromatography mass spectrometry (LCMS), lithium hexamethyl disilazane (LiHMDS), meta-chloroperoxybenzoic acid (m-CPBA), methanol (MeOH), melting point (mp), $MeSO_2$-(mesyl or Ms), methyl (Me), acetonitrile (MeCN), m-chloroperbenzoic acid (MCPBA), mass spectrum (ms), methyl *t*-butyl ether (MTBE), methyl tetrahydrofuran (MeTHF), N-bromosuccinimide (NBS), n-Butyllithium (nBuLi), N-carboxyanhydride (NCA), N-chlorosuccinimide (NCS), N-methylmorpholine (NMM), N-methylpyrrolidone (NMP), pyridinium chlorochromate (PCC), Dichloro-((bis-diphenylphosphino)ferrocenyl) palladium(II) ($Pd(dppf)Cl_2$), palladium(II) acetate ($Pd(OAc)_2$), tris(dibenzylideneacetone)dipalladium(O) ($Pd_2(dba)_3$), pyridinium dichromate (PDC), phenyl (Ph), propyl (Pr), *iso*-propyl (*i*-Pr), pounds per square inch (psi), pyridine (pyr), 1,2,3,4,5-Pentaphenyl-1'-(di-tert-butylphosphino)ferrocene (Q-Phos), room temperature (ambient temperature, rt or RT), sec-Butyllithium (sBuLi), *tert*-butyldimethylsilyl or *t*-$BuMe_2Si$ (TBDMS), tetra-n-butylammonium fluoride (TBAF), triethylamine (TEA or $Et_3N$), 2,2,6,6-tetramethylpiperidine 1-oxyl (TEMPO), trimethylsilylethoxymethyl (SEM), triflate or $CF_3SO_2$-(Tf), trifluoroacetic acid (TFA), 1,1'-*bis*-2,2,6,6-tetramethylheptane-2,6-dione (TMHD), O-benzotriazol-1-yl-N,N,N',N'-tetramethyluronium tetrafluoroborate (TBTU), thin layer chromatography (TLC), tetrahydrofuran (THF), trimethylsilyl or $Me_3Si$ (TMS), p-toluenesulfonic acid monohydrate (TsOH or pTsOH), 4-Me-$C_6H_4SO_2$- or tosyl (Ts), and N-urethane-N-carboxyanhydride (UNCA). Conventional nomenclature including the prefixes *normal (n), iso* (*i*-), *secondary (sec-), tertiary (tert-)* and *neo* have their customary meaning when used with an alkyl moiety. (J. Rigaudy and D. P. Klesney, Nomenclature in Organic Chemistry, IUPAC 1979 Pergamon Press, Oxford.).

General Conditions

Compounds of the present application can be prepared beginning with the commercially available starting materials by utilizing general synthetic techniques and procedures known to those skilled in the art. Outlines below are reaction schemes suitable for preparing such compounds. Further exemplification can be found in the specific examples.

Specific Abbreviations

| | |
|---|---|
| boc | tert-butoxycarbonyl |
| $CH_2Cl_2$ | dichloromethane |
| $Cs_2CO_3$ | cesium carbonate |
| DCM | Dichloromethane |
| DMF | N,N-dimethylformamide |
| DMSO | Dimethylsulfoxide |
| EtOAc | ethyl acetate |
| HATU | O-(7-azabenzotriazol-1-yl)-N,N,N',N'-tetramethyluroniumhexafluorophosphate |
| Hunig's Base | N,N-diisopropylethylamine |
| HCl | hydrogen chloride |
| LC-MS | liquid chromatography mass spectrometry |
| HPLC | high pressure liquid chromatography |
| MeOH | methyl alcohol |
| $MgSO_4$ | magnesium sulfate |
| nBuLi | n-butyl lithium |
| NaCl | sodium chloride |
| $Na_2CO_3$ | sodium carbonate |
| NaOMe | sodium methoxide |
| $Na_2SO_4$ | sodium sulfate |
| $NH_4OH$ | ammonium hydroxide |
| NMP | 1-methyl-2-pyrrolidinone |
| NMR | nuclear magnetic resonance |
| $Pd(OAc)_2$ | palladium(II) acetate |
| TFA | trifluoroacetic acid |
| THF | tetrahydrofuran |
| TLC | thin layer chromatography |
| TMSCl | trimethylsilyl chloride |

General Experimental Details

Reagents were purchased from Aldrich, Oakwood, Matrix or other suppliers and used without further purification. Reactions using microwave irradiation for heating were conducted using either a Personal Chemistry Emrys Optimizer System or a CEM Discovery System. The purification of multi-milligram to multi-gram scale was conducted by methods known know to those skilled in the art such as elution of silica gel flash column; preparative flash column purifications were also effected in some cases by use of disposal pre-packed multigram silica gel columns (RediSep) eluted with a CombiFlash system. Biotage™ and ISCO™ are also flash column instruments that may have been used in this application for purification of intermediates.

For the purpose of judging compound identity and purity, LC/MS (liquid chromatography/mass spectroscopy) spectra were recorded using the following system. For measurement of mass spectra, the system consists of a Micromass Platform II spectrometer: ES Ionization in positive mode (mass range: 150 -1200). The simultaneous chromatographic separation was achieved with the following HPLC system: ES Industries Chromegabond WR C-18 3u 120Å (3.2 x 30mm) column cartridge; Mobile Phase A: Water (0.02% TFA) and Phase B: Acetonitrile (0.02% TFA); gradient 10% B to 90% B in 3 minutes; equilibration time of 1 minute; flow rate of 2 mL/minute. Compounds described below were also purified by reversed phased HPLC, using methods well known to those skilled in the art. In some cases, preparative HPLC purification was conducted using PE Sciex 150 EX Mass Spec controlling a Gilson 215 collector attached to a Shimadzu preparative HPLC system and a Leap autoinjector. Compounds were collected from the elution stream using LC/MS detection in the positive ion detection: The elution of compounds from C-18 columns (2.0 X 10 cm eluting at 20 mL/min) was effected using appropriate linear gradation mode over 10 minutes of Solvent (A) 0.05% TFA/$H_2O$ and Solvent (B) 0.035% TFA/acetonitrile. For injection on to HPLC systems, the crude samples were dissolved in mixtures of methanol, acetonitrile and DMSO.

Compounds were characterized either by $^1$H-NMR using a Bruker 400 MHz NMR Spectrometer or LCMS.

The compounds of the present application may be synthesized according to known techniques. The following examples and references are provided to aid the understanding of the present application. The examples are not intended, however, to limit the application, the true scope of which is set forth in the appended claims. The names of the final

products in the examples were generated using Isis AutoNom 2000.

Preparative Examples

Preparation of reference Compound 1

Step 1. Preparation of 6-nitro-3',6'-dihydro-2'H-[3,4']bipyridinyl-1'-carboxylic acid tert-butyl ester.

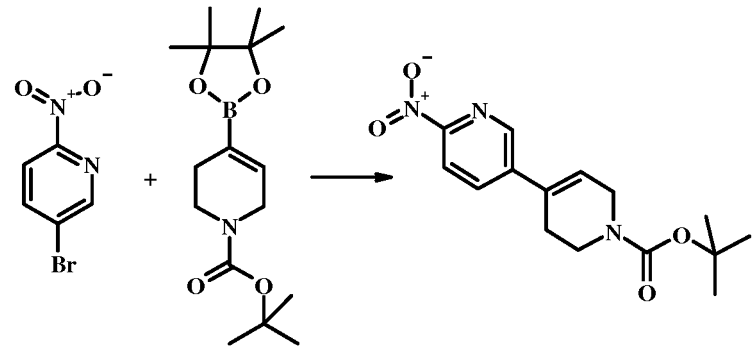

Method AIn a 500 mL round-bottomed flask, 5-bromo-2-nitropyridine (6.56 g, 32.3 mmol) and tert-butyl 4-(4,4,5,5-tetramethyl-1,3,2-dioxaborolan-2-yl)-5,6-dihydropyridine-1(2H)-carboxylate (10 g, 32.3 mmol) were combined with dioxane (160 ml) to give a light yellow solution. $Cs_2CO_3$ (21.1 g, 64.7 mmol) and water (6 ml) were added. The reaction mixture was degassed with argon before bis(triphenylphosphine)palladium(II) dichloride (2.27 g, 3.23 mmol) was added. The reaction mixture was heated to 80 °C and stirred for 15 h. The reaction mixture was poured into 500 mL $H_2O$ and extracted with EtOAc (3 x 200 mL). The combined extracts were washed with brine, dried over $MgSO_4$, filtered and concentrated under vacuum. The crude material was purified by flash chromatography (silica gel, 220 g, 10% to 40% EtOAc in hexanes) to afford a pink solid. The resulting solid was triturated with ether to afford the desired product as a solid (4.8 g). The filtrate from the trituration and mixed fractions from the first chromatography were combined and purified by flash chromatography (silica gel, 220 g, 20% to 40% EtOAc in hexanes) affording additional product (2.2 g). $^1H$ NMR (400 MHz, $CHCl_3$-*d*) δ ppm 1.52 (s, 9 H) 2.59 (d, *J*=1.52 Hz, 2 H) 3.72 (t, *J*=5.56 Hz, 2 H) 4.19 (d, *J*=3.03 Hz, 2 H) 6.35 (br. s., 1 H) 7.97 (dd, *J*=8.46, 2.40 Hz, 1 H) 8.27 (d, *J*=8.34 Hz, 1 H) 8.67 (d, *J*=2.27 Hz, 1 H).

Method B

A 12.0 L three neck flask equipped with mechanical stirrer, heating mantle, condenser, thermometer was charged with 5-bromo-2-nitropyridine (322 g, 1.59 mol), potassium carbonate (658 g, 4.76 mol), tert-butyl 4-(4,4,5,5-tetramethyl-1,3,2-dioxaborolan-2-yl)-5,6-dihydropyridine-1(2H)-carboxylate (500 g, 1.62 mol), 1,4-dioxane (3.24 kg, 3.14 1) and water (314 g, 314 ml). The solution was degassed thrice by pulling vacuum then releasing with $N_2$. Under a strong $N_2$ flow, bis(triphenylphosphine)palladium(ii) dichloride (11.1 g, 15.9 mmol) was added. The reaction mixture was stirred at 80 °C for 5 hours. The heat was turned off and the reaction mixture stirred at ambient temperature, overnight. The mixture was heated to 40 °C, and then charcoal (200 g, 16.7 mol) was added. The mixture stirred at 40 °C for 1 hour. The mixture was cooled to 30 °C, then filtered through Celite™ and washed with plenty of dioxane. To the combined filtrate and wash was added hydrogen peroxide (50.0 g, 45.0 ml, 441 mmol) and the mixture was stirred at ambient temperature for 1 hour. The reddish solution was stored at room temperature for 48 hours then concentrated under vacuum at 50 °C to a low volume solution (weight ca 3226 g). The solution was transferred into a 12L three neck flask and stored at room temperature under $N_2$, overnight. The solution was heated to 40 °C, then added slowly to water (3.38 kg, 3.381, 188 mol). A solid crystallized out. The heat was turned off and the mixture was allowed to cool to room temperature. The mixture was cooled to 8 °C and stirred for 2 hours. The solids were filtered and washed with 1:2 dioxane/water, followed by water. The solid was dried by vacuum for 30 minutes. The solid was transferred into a drying tray then dried in a vacuum oven at 50 °C/26 inches Hg with a $N_2$ bleed to a constant weight to afford 460 g (95%) of the desired product. $^1H$ NMR (300 MHz, DMSO-$d_6$) δ ppm 1.43 (s, 9 H) 2.55 (d, *J*=1.89 Hz, 2 H) 3.47 - 3.73 (m, 2 H) 4.08 (d, *J*=2.64 Hz, 2 H) 6.57 (br. s., 1 H) 8.20 - 8.26 (m, 1 H) 8.27 - 8.33 (m, 1 H) 8.77 (d, *J*=1.89 Hz, 1 H).

Step 2. Preparation of 6-Amino-3',4',5',6'-tetrahydro-2'H-[3,4']bipyridinyl-1'-carboxylic acid tert-butyl ester

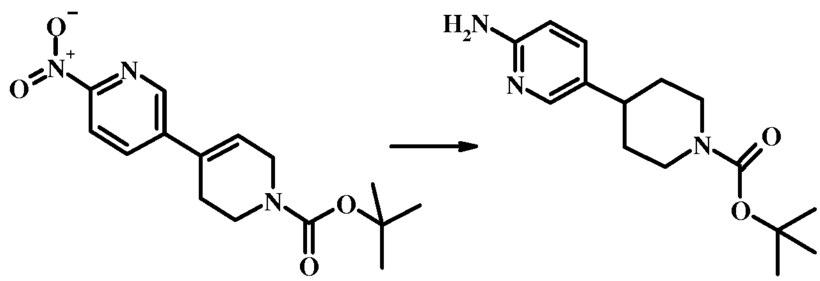

Method A

In a 500 mL round-bottomed flask, tert-butyl 4-(6-nitropyridin-3-yl)-5,6-dihydropyridine-1(2H)-carboxylate (4.9 g, 16.0 mmol) in EtOH (300 ml) and ethyl acetate (75 ml) was combined with palladium on carbon (1.32 g, 1.24 mmol). The reaction mixture was evacuated twice with hydrogen and then stirred with a hydrogen-filled balloon overnight. LC/MS analysis showed that the reaction was complete. The reaction mixture was purged with nitrogen and filtered through celite. The celite cake was washed several times with EtOAc. To the colorless combined filtrate and washes was added $CH_2Cl_2$ and the solution was evaporated to dryness. $CH_2Cl_2$ was added again and the solution was concentrated under vacuum to afford quantitative yield of the desired product. $(M+H)^+$ = 278 m/e.

Method B

Reactor Pretreatment: The 20L hydrogenation reactor was charged with 10 g of Pd/C and 8 L of ethyl acetate. The mixture stirred at 60 °C for 4 hours. The heat was turned off, and suspension was cooled to room temperature. The suspension was drained. The 20L hydrogenation reactor was charged with a suspension of tert-butyl 4-(6-nitropyridin-3-yl)-5,6-dihydropyridine-1(2H)-carboxylate (460 g, 1.51 mol) in ethyl acetate (2.3 kg, 2.56 1). EtOH (2.02 kg, 2.56 1) was used to rinse the residue from the flask containing the suspension into the hydrogenation reactor. Then Pd/C (160 g, 75.4 mmol) was added. After degassing the suspension with $N_2$ three times, and with $H_2$ three times, the reaction was stirred under 500 psi of hydrogen at 60 °C for 3 hours. The content of the reactor was drained, filtered thru Celite™, and washed with plenty of ethyl acetate. The combined filtrate and wash were stored in the cold room under $N_2$ overnight and then concentrated under vacuum at 40 °C to dryness to give a gray solid. MTBE (1.53 kg, 2.06 1, 17.3 mol) was added and the solution was concentrated under vacuum to a low volume to remove 700 ml of solvent. N-heptane (705 g, 1.03 1, 7.04 mol) was added and the solution was concentrated under vacuum to remove 400 ml of solvent. N-heptane (705 g, 1.03 1, 7.04 mol) was added again and the solution was concentrated under vacuum to get a movable slurry. The slurry was stirred at room temperature for 30 minutes, and then cooled to 9 °C where it stirred for 45 minutes. The suspension was then filtered and washed with cold heptane. The off-white solids were transferred into a drying tray and dried in a vacuum oven at 30 °C/26 in Hg with a $N_2$ bleed over the weekend. The filtrate afforded 381 g (91%) of the desired product. $^1H$ NMR (300 MHz, DMSO-$d_6$) δ ppm 1.18 - 1.53 (m, 11 H) 1.66 (d, *J*=11.33 Hz, 2 H) 2.76 (br. s., 2 H) 4.04 (d, *J*=12.46 Hz, 2 H) 5.67 (s, 2 H) 6.38 (d, *J*=8.69 Hz, 1 H) 7.25 (dd, *J*=8.50, 2.45 Hz, 1 H) 7.76 (d, *J*=2.27 Hz, 1 H).

Step 3. Preparation of 6-(6-chloro-2-methyl-3-oxo-2,3-dihydro-pyridazin-4-ylamino)-3',4',5',6'-tetrahydro-2'H-[3,4']bipyridinyl-1'-carboxylic acid tert-butyl ester.

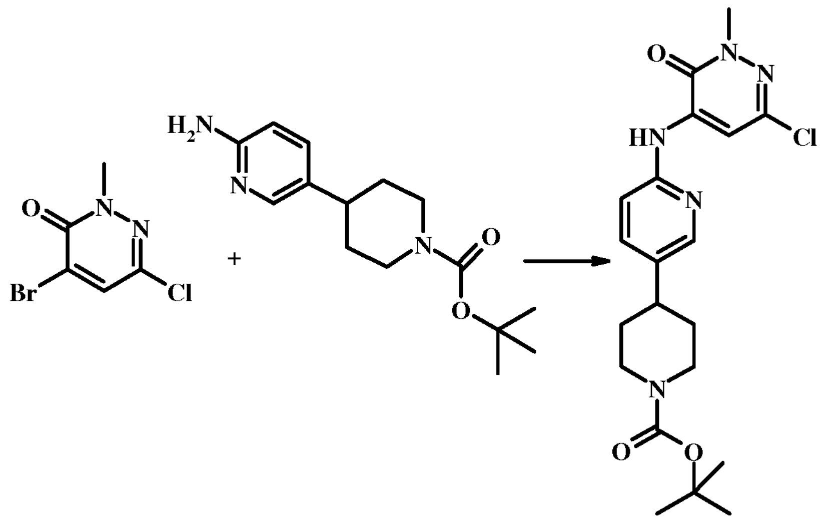

Method A

4-Bromo-6-chloro-2-methylpyridazin-3(2H)-one (3.59 g, 16.0 mmol), tert-butyl 4-(6-aminopyridin-3-yl)piperidine-1-carboxylate (4.45 g, 16.0 mmol), 4,5-bis(diphenylphosphino)-9,9-dimethylxanthene (696 mg, 1.2 mmol) and cesium carbonate (18.3 g, 56.2 mmol) were suspended in dioxane (150 ml) under an argon atmosphere. Finally tris(dibenzylideneacetone)dipalladium(0) (551 mg, 602 μmol) was added. The reaction mixture was heated at 90 °C overnight. The reaction mixture was filtered over celite, and the celite cake was washed with dioxane several times. The combined filtrate and washes were concentrated under vacuum. The resultant solid was triturated with EtOAc, washed with ether and dried in a vacuum oven overnight at 50 °C to afford 4.57 g of the desired product as a white solid. The combined filtrate and washes were evaporated to dryness and dissolved in $CH_2Cl_2$ (4 ml) and then purified by flash chromatography (silica gel, 120 g Analogix column, 20% to 50% EtOAc in hexanes over 20 min) to afford an additional 582 mg. Total yield (5.15 g, 12.3 mmol, 76.4 % yield). $(M+H)^+$ =420 m/e; $^1H$ NMR (400 MHz, $CHCl_3$-*d*) δ ppm 1.50 (s, 9 H) 1.54 - 1.69 (m, 3 H) 1.83 (d, *J*=13.64 Hz, 2 H) 2.67 (tt, *J*=12.38, 3.66 Hz, 1 H) 2.83 (t, *J*=13.14 Hz, 2 H) 3.82 (s, 3 H) 6.89 (d, *J*=8.59 Hz, 1 H) 7.51 (dd, *J*=8.46, 2.40 Hz, 1 H) 8.25 (d, *J*=2.27 Hz, 1 H) 8.27 (br. s., 1 H) 8.30 (s, 1 H).

Method B

A 12L three neck flask equipped with mechanical stirrer, $N_2$ bubbler, and thermometer was charged with tert-butyl 4-(6-aminopyridin-3-yl)piperidine-1-carboxylate (381 g, 1.37 mol), followed by THF (2.35 kg, 2.67 1). The reaction mixture was stirred until the reagent went into solution. Sodium tert-pentoxide, 2.5M in THF (577 ml, 1.44 mol) was added to the solution dropwise. The temperature went from 22°C to 25°C. The reaction mixture stirred for 30 min. A solution of 4-bromo-6-chloro-2-methyl-2H-pyridazin-3-one (322 g, 1.44 mol) in THF (1.17 kg, 1.33 1) for a total volume of 1640 mL was prepared. 820 mL of the 4-bromo-6-chloro-2-methyl-2H-pyridazin-3-one solution (0.5 eq.) was slowly added over 30 min to the tert-butyl 4-(6-aminopyridin-3-yl)piperidine-1-carboxylate; keeping the temperature of the reaction below 30 °C by adding some ice to the water bath. The reaction mixture was stirred for 30 minutes more. Sodium tert-pentoxide, 2.5M in THF (275 ml, 687 mmol) was added to the reaction mixture, then the mixture was stirred for an additional 20 min. 410 mL more of the 4-bromo-6-chloro-2-methyl-2H-pyridazin-3-one solution (0.25 eq.) was slowly added, keeping the temperature below 30 °C, then the reaction mixture was stirred for 20 min. Sodium tert-pentoxide, 2.5M in THF (137 ml, 343 mmol) was added to the reaction mixture, and then the mixture was stirred for an additional 20 min. The final 410 mL of the 4-bromo-6-chloro-2-methyl-2H-pyridazin-3-one solution (0.25 eq.) were added, keeping the temperature below 30 °C, then the reaction mixture was stirred for another 20 min. Sodium tert-pentoxide, 2.5M in THF (137 ml, 343 mmol) was added to the reaction mixture, and then the red mixture was stirred for an additional hour and 20 min. A 600 ml of a solution of citric acid prepared by dissolving citric acid(264 g, 1.37 mol) in water (1.14 kg, 1.141) (~20% solution) was slowly added to the reaction, maintaining the temperature below 30°C, bringing the pH of the reaction to 4.6. The reaction mixture turned to yellow suspension. Water (800 ml) was added. The mixture was heated to 50 °C and was stirred for 2 hours. The reaction mixture was cooled to room temperature overnight, giving a nice, yellow suspension amenable to stirring. The suspension was transferred into a 20 L Buchi flask and concentrated under vacuum at 40 °C to remove about 3.2 L of solvent. The slurry was stirred at room temperature for 3 hours, then the solids were filtered using table top funnel and washed with water. The off-white solids were dried by suction for 1 hour. The solid was dried in a vacuum oven at 60°C/26 in Hg with $N_2$ bleed to a constant weight to give 488 g (84%) of

the desired product. $^1H$ NMR (300 MHz, DMSO-$d_6$) δ ppm 1.42 (s, 9 H) 1.50 (dd, *J*=12.65, 3.59 Hz, 2 H) 1.74 (d, *J*=11.33 Hz, 2 H) 2.58 - 2.96 (m, 3 H) 3.68 (s, 3 H) 4.07 (d, *J*=12.09 Hz, 2 H) 7.48 (d, *J*=8.69 Hz, 1 H) 7.67 (dd, *J*=8.50, 2.45 Hz, 1 H) 8.27 (d, *J*=2.27 Hz, 1 H) 8.33 (s, 1 H) 9.62 (s, 1H).

Step 4. Preparation of 6-chloro-2-methyl-4-(1'-methyl-1',2',3',4',5',6'-hexahydro-[3,4']bipyridinyl-6-ylamino)-2H-pyridazin-3-one.

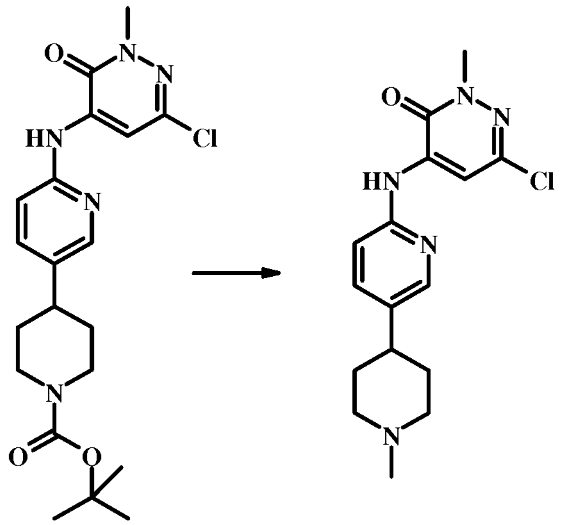

Method A

Tert-butyl 4-(6-(6-chloro-2-methyl-3-oxo-2,3-dihydropyridazin-4-ylamino)pyridin-3-yl)piperidine-1-carboxylate (2.0 g, 4.76 mmol) was dissolved in a solvent mixture of formic acid (40.0 ml) and formaldehyde, 37% (80.0 ml). The reaction mixture was stirred at 70 °C overnight until the reaction was complete as determined by LCMS analysis, and then cooled to ambient temperature. Water was added and the resultant aqueous mixture was washed with $CH_2Cl_2$ and the $CH_2Cl_2$ layer was discarded. The pH of the aqueous layer was carefully adjusted to pH = 12 with solid $K_2CO_3$, which resulted in precipitation of a solid. The solid was collected by filtration, washed with water and dried in vacuum oven at 50 °C over 72 h to afford 1.4 g of the desired product. $(M+H)^+$ = 334 m/e. $^1H$ NMR (300 MHz, $CHCl_3$-*d*) δ ppm 1.84 (dd, *J*=8.31, 3.02 Hz, 4 H) 1.99 - 2.19 (m, 2 H) 2.35 (s, 3 H) 2.42 - 2.68 (m, 1 H) 3.02 (d, *J*=12.09 Hz, 2 H) 3.81 (s, 3 H) 6.86 (d, *J*=8.31 Hz, 1 H) 7.52 (dd, *J*=8.50, 2.46 Hz, 1 H) 8.16 - 8.33 (m, 3 H).

Method B

A 5.0 L three neck flask equipped with mechanical stirrer, $N_2$ bubbler, thermometer, condenser and heating mantle was charged with tert-butyl 4-(6-(6-chloro-2-methyl-3-oxo-2,3-dihydropyridazin-4-ylamino)pyridin-3-yl)piperidine-1-carboxylate (488 g, 1.16 mol). To this flask was added formic acid (2.34 kg, 1.95 1). The reaction mixture became a dark solution. The solution was heated to 55 °C and stirred for 1hr. The deprotection was complete as judged by HPLC. Formaldehyde, 37% aqueous solution (472 g, 433 ml, 5.81 mol) was added to the mixture. The reaction mixture was heated to 85 °C and stirred for 3 hours. The heat was turned off and the reaction mixture was allowed to cool to room temperature overnight then concentrated under vacuum at 70°C to get a dark oil. The oil was transferred into a 12L three neck flask, to which was added IPA (593 g, 761 ml). Solids crystallized out. To the solids was added a 20% aqueous $K_2CO_3$ solution (total 2650 ml to make pH 8.3) and during addition of the base, the mixture became a solution, and then at pH 7, became a white suspension again. The suspension was heated at 65 °C for 3 hrs, then slowly cooled down to ambient temperature overnight. The solid was collected by filtration using a table-top funnel. The white solids were washed with water. The solids were washed with heptane and dried by suction and then further dried in a vacuum oven at 75 °C/26 in Hg with $N_2$ bleed overnight to afford 367 g (94%) of the desired product. $^1H$ NMR (300 MHz, DMSO-$d_6$) δppm 1.57 - 1.76 (m, 4 H) 1.94 (td, *J*=11.24, 3.59 Hz, 2 H) 2.18 (s, 3 H) 2.38 - 2.47 (m, 1 H) 2.85 (d, *J*=11.33 Hz, 2 H) 3.68 (s, 3 H) 7.48 (d, *J*=8.69 Hz, 1 H) 7.58 - 7.71 (m, 1 H) 8.26 (d, *J*=2.64 Hz, 1 H) 8.33 (s, 1 H) 9.61 (s, 1 H).

Step 5. Preparation of 2-(6-tert-butyl-8-fluoro-1-oxo-1H-phthalazin-2-yl)-4-iodo-pyridine-3-carbaldehyde

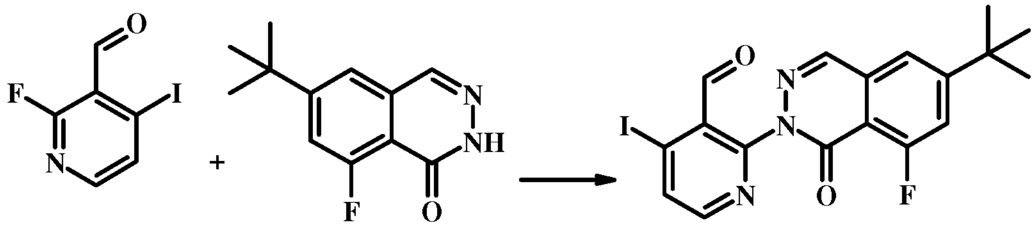

Method A

In a 1 L round-bottomed flask, 6-tert-butyl-8-fluorophthalazin-1(2H)-one (5.6 g, 25.4 mmol) was combined with THF (300 ml) to give a colorless solution. Sodium hydride (1.12 g, 28.0 mmol) was added. The reaction mixture was stirred at ambient temperature for 10 min. 2-Fluoro-4-iodonicotinaldehyde (7.02 g, 28.0 mmol) was added and the reaction mixture was stirred at ambient temperature for 1 h. The reaction was complete as determined by LCMS analysis. The reaction mixture was quenched with saturated $NH_4Cl$. The reaction mixture was poured into 200 mL of $H_2O$ and extracted thrice with $CH_2Cl_2$. The organic layers were washed with brine, then dried over $Na2_SO_4$ and concentrated under vacuum. The resultant bright yellow solid was transferred into a filter funnel and the flask washed twice with a small volume of EtOAc to ensure complete transfer of the solid into the funnel. The liquid was filtered through. The solid was triturated twice with $Et_2O$ and dried under vacuum to afford the desired product as a cream-colored solid (8.09 g, 17.9 mmol, 70.5 % yield). $(M+H)^+$ = 452 m/e. $^1H$ NMR (400 MHz, $CHCl_3$-*d*) δ ppm 1.44 (s, 9 H) 7.49 - 7.54 (m, 1 H) 7.54 (d, *J*=1.77 Hz, 1 H) 8.03 (d, *J*=5.31 Hz, 1 H) 8.30 (d, *J*=2.53 Hz, 1 H) 8.37 (d, *J*=5.31 Hz, 1 H) 9.98 (s, 1 H).

Method B

A 50-L jacketed reactor was charged with 6-tert-butyl-8-fluorophthalazin-1(2H)-one (799 g, 3.63 mol) and THF (5.28 kg, 6.00 1). This yellow suspension was treated drop wise over 30 minutes with 1M solution in THF of LiHMDS (4.00 1, 4.00 mol) maintaining temp < 29 °C. After the addition was complete, the brown solution was stirred at ca. 22 °C for 40 minutes. The reaction was heated to 60 °C. Once the reaction was up to temperature, the slow addition of 2-fluoro-4-iodonicotinaldehyde (1.00 kg, 3.99 mol) in THF (5.28 kg, 6.00 1) started. Note: about 10 minutes into addition of the aldehyde solution, an orange-red suspension formed. Once the addition was complete, the reaction was a dark brown solution. The reaction was heated at 65 °C for 15 min. The heat was turned "OFF".

The reaction was allowed to cool to room temperature and agitate at room temp overnight. Water (6 L) was added in one portion to the reaction mixture and an exotherm (20-26 °C) was observed. The mixture agitated for about 50 min. Most of the THF (17 L) was removed under vacuum (bath temp: 42 °C) to yield an orange suspension. 2-Propanol (4.68 kg, 6.00 1) was added to the flask and the mixture was heated at 60 °C for 15 minutes. The reaction mixture cooled to room temperature while agitating, overnight. The product was filtered through a coarse fritted tabletop funnel and washed twice with 1.6 L of IPA (3.2 L) and dried by suction. A wet cake (2.155 kg of an orange solid) was dried in a vacuum oven at about 27 in Hg/50 °C with a nitrogen bleed to give 1.31 kg (79 %) of the desired product as a light brown solid.

Step 6. 2-(6-tert-Butyl-8-fluoro-1-oxo-1H-phthalazin-2-yl)-4-[1-methyl-5-(1'-methyl-1',2',3',4',5',6'-hexahydro-[3,4']bipyridinyl-6-ylamino)-6-oxo-1,6-dihydro-pyridazin-3-yl]-pyridine-3-carbaldehyde.

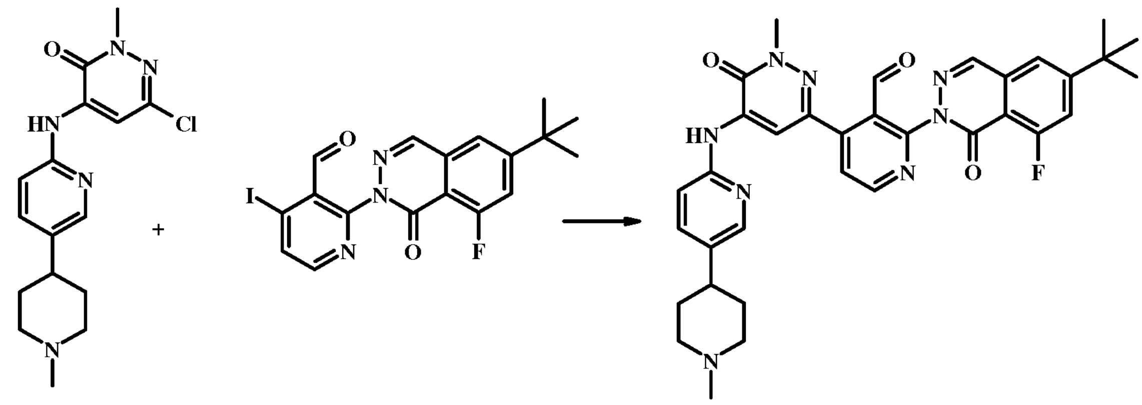

Method A

6-Chloro-2-methyl-4-(5-(1-methylpiperidin-4-yl)pyridin-2-ylamino)pyridazin-3(2H)-one (1.4 g, 4.19 mmol), bis(pinacolato)diboron (1.17 g, 4.61 mmol) and potassium acetate (1.23 g, 12.6 mmol) were suspended in dioxane (60 ml). The reaction mixture was degassed under argon. X-PHOS (300 mg, 629 μmol) and palladium(II) acetate (47.1 mg, 210 μmol) were added and the reaction mixture was stirred at 100 °C (external temperature) for 1 h under a nitrogen atmosphere. The reaction was monitored closely by LCMS by sampling an aliquot and dissolving it in methanol and looking for disappearance of starting chloride and concurrent appearance of the boronic acid (M+1 = 344) but being careful to minimize the amount of des-chlorinated side product (M+1 = 300). The reaction was complete after 1 h. The temperature of the heating bath was turned down to 80 °C and the flask was raised out of the heating bath, but continued stirring. 2-(6-*tert*-Butyl-8-fluoro-1-oxophthalazin-2(1H)-yl)-4-iodonicotinaldehyde (1.89 g, 4.19 mmol) and potassium carbonate (1.74 g, 12.6 mmol) were added, followed by water (6.00 ml). Tricyclohexylphosphine (118 mg, 419 μmol) and bis(dibenzylideneacetone)palladium (121 mg, 210 μmol) were added. The reaction mixture was heated with vigorous stirring at 80 °C and stirred 2 h and then the reaction mixture was cooled to ambient temperature. The reaction mixture was poured onto water and extracted with gentle shaking into EtOAc (2 x). The combined EtOAc extracts were washed with brine. The aqueous phase was extracted thrice with $CH_2Cl_2$. The $CH_2Cl_2$ and ethyl acetate layers were combined and the combined organic layers were dried over $Na_2SO_4$ and concentrated under vacuum. The crude material was slurried in 50 ml of $CH_2Cl_2$ and 200 ml of $Et_2O$ was added. The solid was filtered and washed with $Et_2O$. A second batch of solid precipitated out and was collected by filtration and washed with ether. Both batches had similar LCMS and $^1H$-NMR spectra, which were consistent with desired product, and they were combined to afford 1.62 g of product. $(M+H)^+$ = 623 m/e. $^1H$ NMR (300 MHz, $CHCl_3$-*d*) δ ppm 1.42 (s, 9 H) 1.88 (br. s., 3 H) 2.39 (br. s., 3 H) 2.46 - 2.64 (m, 1 H) 3.05 (br. s., 2 H) 3.89 (s, 3 H) 6.91 (d, *J*=8.31 Hz, 1 H) 7.38 - 7.66 (m, 3 H) 7.76 (d, *J*=5.29 Hz, 1 H) 8.19 - 8.38 (m, 3 H) 8.81 (s, 1 H) 8.87 (d, *J*=5.29 Hz, 1 H) 10.11 (s, 1 H).

Method B

The reactor was charged with 6-chloro-2-methyl-4-(5-(1-methylpiperidin-4-yl)pyridin-2-ylamino)pyridazin-3(2H)-one (450 g, 1.35 mol), 4,4,4',4',5,5,5',5'-octamethyl-2,2'-bi(1,3,2-dioxaborolane) (513 g, 2.02 mol), tricyclohexylphosphine (22.7 g, 80.9 mmol), $Pd(dba)_2$ (23.3 g, 40.4 mmol) and potassium acetate (265 g, 2.7 mol). 2-Methyltetrahydrofuran (10.01) was added with vacuum. The stirring was started, set at 10. A vacuum was pulled and the reactor was backfilled with nitrogen, twice. The reaction mixture was heated to 78 °C (inner) / 80 °C (cir.) under $N_2$ atmosphere. The reaction mixture was heated overnight. The reactor was cooled down to 10 °C. Once the reaction temperature went down to under 40 °C, the temperature was set to 30 °C. 2-(6-tert-Butyl-8-fluoro-1-oxophthalazin-2(1H)-yl)-4-iodonicotinaldehyde (578 g, 1.28 mol), potassium carbonate (373 g, 2.7 mol) and water (1.00 1) was added to the reaction mixture. The reaction was heated to 74°C (inner temp.)/78°C (cir.) to achieve a gentle reflux. The reaction mixture was heated at reflux, overnight. The reaction mixture was cooled down to 40°C (cir.)/38°C (reaction). To the reaction mixture was added (R)-2-acetamido-3-mercaptopropanoic acid (33.0 g, 202 mmol) solution in water (3.00 1). The reaction mixture stirred at 38°C (inner)/ 40°C (cir.) for 3 h. Water (6 L) was added, then 9.5 L of MeTHF was distilled (cir. temperature, no more than 40°C). IPA (3 L) was added, while maintaining the temperature of the mixture at 23°C with stirring. The solid was collected by filtration (Chem glass 50 L filter) after a very slow, overnight filtration. The reactor was charged with $H_2O$ (5 L). Then the filter cake was washed with the water from the reactor. The filtration remained slow. The filter cake was washed with IPA (6 L) during which, the filter was completely blinded with small particles. The material was transferred into a large table-top filter, and then continued IPA filtration. The solid was washed with nHeptane. Some product with small particle size got filtered through it, so those were recovered after also. The solid was air-dried overnight

to give the title compound (797 g) not fully dried, but used as such in Step 6, Method B.

Step 6. 6-tert-Butyl-8-fluoro-2-{3-hydroxymethyl-4-[1-methyl-5-(1'-methyl-1',2',3',4',5',6'-hexahydro-[3,4']bipyridinyl-6-ylamino)-6-oxo-1,6-dihydro-pyridazin-3-yl]-pyridin-2-yl}-2H-phthalazin-1-one.

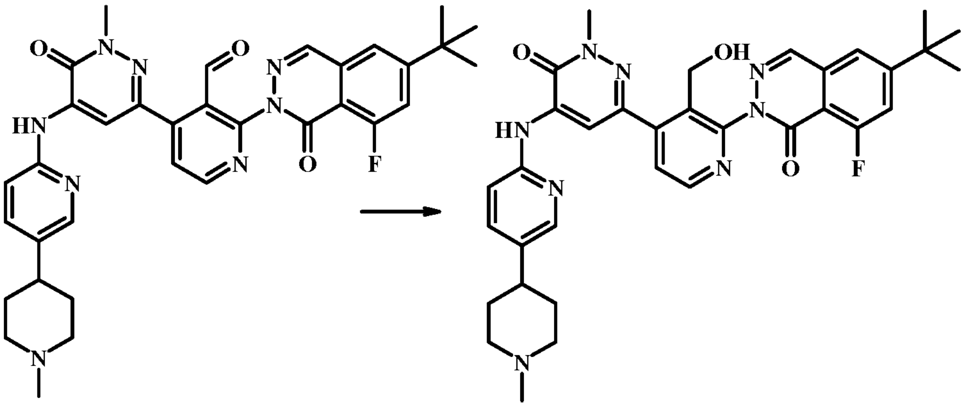

Method A

In a 250 mL round-bottomed flask, 2-(6-tert-butyl-8-fluoro-1-oxophthalazin-2(1H)-yl)-4-(1-methyl-5-(5-(1-methylpiperidin-4-yl)pyridin-2-ylamino)-6-oxo-1,6-dihydropyridazin-3-yl)nicotinaldehyde (1.62 g, 2.6 mmol) was combined with dry $CH_2Cl_2$ (45 ml) and dry MeOH (20 mL) to give a brown solution. Sodium borohydride (177 mg, 4.68 mmol) was added and the reaction was stirred at ambient temperature for 1 h before being quenched with saturated $NH_4Cl$. The reaction mixture was diluted with 50 mL $H_2O$ and extracted with $CH_2Cl_2$ (3 x 150 mL). The organic layers were dried over $Na_2SO_4$ and concentrated under vacuum. The crude material was purified by flash chromatography [silica gel, 80g, 0% to 50% (60:10:1 $CH_2Cl_2$:MeOH: $NH_4OH$) in $CH_2Cl_2$] to afford a slightly impure foam. The foam was slurried in 30 ml $Et_2O$ and 10 ml EtOAc and then stirred slowly with a heavy stir bar for 1 h resulting in a white solid. The solid was collected by filtration, dried under vacuum at 50 °C for 48 h. to afford the desired product as a white solid (880 mg). $(M+H)^+$ = 625 m/e. $^1H$ NMR (300 MHz, $CHCl_3$-*d*) δ ppm 1.44 (s, 9 H) 1.87 (br. s., 3 H) 2.15 (br. s., 2 H) 2.39 (br. s., 3 H) 2.52 (t, *J*=7.74 Hz, 1 H) 3.04 (br. s., 2 H) 3.82 - 3.91 (m, 1 H) 3.93 (s, 3 H) 4.46 - 4.63 (m, 2 H) 6.93 (d, *J*=8.69 Hz, 1 H) 7.42 - 7.59 (m, 3 H) 7.64 (d, *J*=4.91 Hz, 1 H) 8.15 - 8.39 (m, 3 H) 8.70 (s, 1 H) 8.73 (d, *J*=4.91 Hz, 1 H).

Method B

2-(6-tert-butyl-8-fluoro-1-oxophthalazin-2(1H)-yl)-4-(1-methyl-5-(5-(1-methylpiperidin-4-yl)pyridin-2-ylamino)-6-oxo-1,6-dihydropyridazin-3-yl)nicotinaldehyde (797 g, 1.28 mol) was dissolved in DCM (7.36 kg, 5.58 1) and MeOH (1.26 kg, 1.59 1). The solution was treated with activated carbon (10% wt), Celite™ (10% wt) and QuadraPure TU (5 wt%) at room temperature overnight. The mixture was filtered through a pad of Celite. $NaBH_4$ (24.2 g, 640 mmol) was added in 6 portions over 1 h to the solution, maintaining the temperature between 15-18 °C. The reaction was quenched with water (5 L). The mixture stirred for 30 min, and then the phases were separated. The aqueous layer was extracted once with DCM (2 L) and polish filtered giving a total of 9 L DCM solution. DCM was distilled at 30°C under vacuum, and at the same time, MEK (5 L) and MeOH (5 L) was added into the solution. After most of DCM was removed, temperature was increased to 40 °C to make sure there is no DCM left (chasing with MeOH at 40 °C); product was crystallizing out at this temperature. The temperature was increased to 80°C. Additional MEK (5 L) was added. The MeOH was removed under vacuum at 80°C. The mixture heated at 80°C for 3 hr, and then slowly cooled down to room temperature overnight. The solids were filtered, washed with MEK, and dried under vacuum over the weekend to give 6-tert-butyl-8-fluoro-2-(3-(hydroxymethyl)-4-(1-methyl-5-(5-(1-methylpiperidin-4-yl)pyridin-2-ylamino)-6-oxo-1,6-dihydropyridazin-3-yl)pyridin-2-yl)phthalazin-1(2H)-one (471 g, 754 mmol, 58.9 % yield). DSC showed the mixture of polymorph (anhydrous and MeOH solvate), and there was still borane complex (3%) left.

**Recrystallization**

6-tert-butyl-8-fluoro-2-(3-(hydroxymethyl)-4-(1-methyl-5-(5-(1-methylpiperidin-4-yl)pyridin-2-ylamino)-6-oxo-1,6-dihydropyridazin-3-yl)pyridin-2-yl)phthalazin-1(2H)-one (540 g, 864 mmol) (previous batch plus material from three smaller batches prepared in a similar manner as described in Method B above) was charged into a 12 L round bottomed flask and MeOH (2.14 kg, 2.7 1) and methyl ethyl ketone (5.4 1) were added. The suspension was heated to 64-66°C (inner) for 3 h to give a gentle MeOH reflux in order to break 3% of borane complex. After 3 h, only ~1% of borane complex left. The MeOH was distilled off from the solution; temperature went up to 68-70°C (inner)/ 80-85°C (heating mantle), the MeOH was chased with additional MEK. Aging at 68-70°C (inner) for 10 h, then slowly cooled down to room temperature. The slurry was heated for 5-6 h at 70-75 °C, DSC showed a mixture of forms. The slurry was heated again to 70-75°C (inner)/ 90-95°C (heating mantle) to remove the MeOH completely; adding more MEK. After inner temperature reached to 76-77°C, the distillation was stopped (final MEK, 6 vol. (~3 L)), and the slurry was aged at 75°C (inner) for 10 h; then cooled down to room temperature, slowly. After confirmation of the form by DSC, the material was collected by filtration, washed with MEK (400 mL), and dried under vacuum at 50°C overnight. NMR showed 0.23% of residual MEK. The solid was transferred to a drying dish, and dried under vacuum at 50°C overnight to give the title compound (503 g, 805 mmol, 6293.1 % yield) with 99.44% HPLC purity and 15 ppm residual Pd. $^1$H NMR (300 MHz, DMSO-$d_6$) δ ppm 1.38 (s, 9 H) 1.52 - 1.77 (m, 4 H) 1.87 - 2.01 (m, 2 H) 2.18 (s, 3 H) 2.33 - 2.46 (m, 1 H) 2.84 (d, *J*=11.33 Hz, 2 H) 3.79 (s, 3 H) 4.47 (dd, *J*=15.86, 5.29 Hz, 2 H) 4.76 - 4.88 (m, 1 H) 7.45 (d, *J*=8.69 Hz, 1 H) 7.58 - 7.66 (m, 2 H) 7.77 (dd, *J*=13.22, 1.51 Hz, 1 H) 7.89 (d, *J*=1.89 Hz, 1 H) 8.18 (d, *J*=2.27 Hz, 1 H) 8.53 (d, *J*=2.64 Hz, 1 H) 8.56 (s, 1 H) 8.63 (d, *J*=4.91 Hz, 1 H) 9.45 (s, 1 H).

Preparation of the fumarate salt of reference compound 1: 6-tert-butyl-8-fluoro-2-(3-(hydroxymethyl)-4-(1-methyl-5-(5-(1-methylpiperidin-4-yl)pyridin-2-ylamino)-6-oxo-1,6-dihydropyridazin-3-yl)pyridin-2-yl)phthalazin-1(2H)-one fumarate

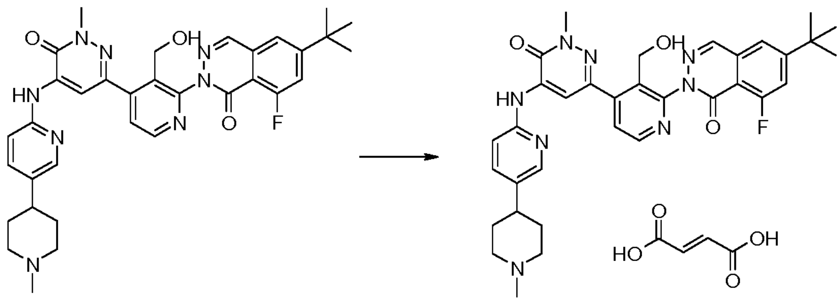

6-tert-butyl-8-fluoro-2-(3-(hydroxymethyl)-4-(1-methyl-5-(5-(1-methylpiperidin-4-yl)pyridin-2-ylamino)-6-oxo-1,6-dihydropyridazin-3-yl)pyridin-2-yl)phthalazin-1(2H)-one (639 mg, 1.02 mmol) was mixed with acetone (15 ml). To the slurry was added fumaric acid (125 mg, 1.07 mmol). The solution was stirred for 1 hr. The solid formed was filtered and then placed under vacuum in a vacuum oven at 50 °C overnight. A yellow solid was recovered (718.0 mg) and recrystallized from refluxing acetone (250 ml) to give 6-tert-butyl-8-fluoro-2-(3-(hydroxymethyl)-4-(1-methyl-5-(5-(1-methylpiperidin-4-yl)pyridin-2-ylamino)-6-oxo-1,6-dihydropyridazin-3-yl)pyridin-2-yl)phthalazin-1(2H)-one fumarate (718 mg, 969 μmol, 94.8 % yield) as a mostly amorphous solid. $(M+H)^+$ = 625 m/e.

**Preparation of reference Compound 2**

Step 1. Preparation of 2,4,6-trifluoro-N-(2-hydroxy-1,1-dimethyl-ethyl)-benzamide.

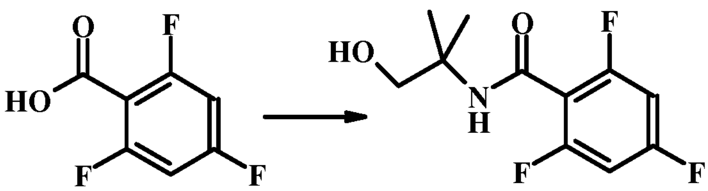

A flask, fitted with a calcium chloride drying tube, was charged with 2,4,6-trifluorobenzoic acid (25 g, 142 mmol) and taken up in dry dichloromethane (220 mL). The material was cooled to 0 °C (ice bath) and to this was added oxalyl

chloride (13.2 ml; 156 mmol) via syringe. Dry dimethylformamide (104 mg; 1.42 mmol) was next added and moderate bubbling was observed. After 15 minutes the cooling bath was removed and the mixture was stirred vigorously for 5 h. The volatiles were stripped (rotary evaporator) and the remainder was taken up in dry dichloromethane (150 ml) and cooled to 0 °C (ice bath). To this solution was added 2-amino-2-methyl-1-propanol (27.2 ml, 284 mmol) via slow drop-wise addition. After complete addition the cooling bath was removed and the mixture was warmed to ambient temperature overnight. The reaction, as described above was repeated on the same scale, and the combined reaction products were worked up as follows: The non-homogeneous mixture was suction filtered, rinsing with dichloromethane (approximately 300 ml). This first filtrate was set aside and the solid filtrated was rinsed a second time with dichloromethane (500 ml) using slow gravity filtration. The dichloromethane from the second filtration was stripped, which provided very pure product as a white crystalline solid (22.9 g). The dichloromethane solution from the first filtration was stripped to provide an impure brown colored remainder. This material was taken up in dichloromethane (200 ml) and water (250 ml) and shaken in a separatory funnel. The organic phase was collected and the aqueous phase back extracted with dichloromethane (2 x 120 ml). The dichloromethane phases were combined, dried (magnesium sulfate), filtered and stripped. The crude remainder was purified via trituration from hot dichloromethane / hexanes to provide additional desired product as a yellow solid (43.8 g). $(M+H)^+$ = 246 m/e.

Step 2. Preparation of 4,4-dimethyl-2-(2,4,6-trifluoro-phenyl)-4,5-dihydro-oxazole.

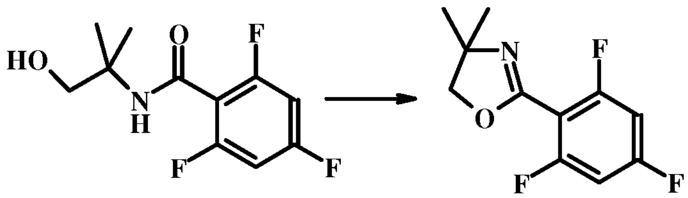

To a solution of 2,4,6-trifluoro-N-(2-hydroxy-1,1-dimethyl-ethyl)-benzamide (43.8 g, 177.1 mmol) in dry dichloromethane (400 mL) was added thionyl chloride (58.9 ml, 415 mmol) via slow drop-wise addition over 25 minutes (reaction flask was immersed in an ice bath partway through the addition). After complete addition the material was stirred to ambient temperature overnight. The material was next placed on a rotary evaporator and condensed (remove approximately 70% of the volume). The remainder was taken up in ether (200 ml) and a solid precipitate (39.94 g off-white solid) was collected by filtration. The ether filtrate was set aside and the solid material was taken up in water (120 ml) and treated with an aqueous solution of sodium hydroxide (2N, 55 ml). Ethyl acetate (120 ml) was added and the mixture was transferred to a separatory funnel and shaken. The organic phase was collected and washed with an equal volume of water. The ethyl acetate phase was collected and the aqueous phases were back extracted with ethyl acetate (2 x 100 ml). The combined organic phases were combined, dried (magnesium sulfate), filtered and stripped to provide the title compound as a pure off-white solid (33.98 g). $(M+H)^+$ = 230 m/e.

Step 3. Preparation of 2-(2,4-difluoro-6-methyl-phenyl)-4,4-dimethyl-4,5-dihydro-oxazole.

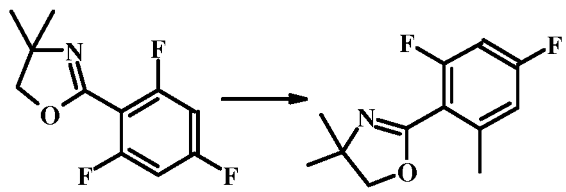

To a cooled (ice bath) solution of 4,4-dimethyl-2-(2,4,6-trifluorophenyl)-4,5-dihydrooxazole (16.8 g, 73.3 mmol) in dry tetrahydrofuran (150 ml) was added a solution of methyl magnesium bromide (73.3 ml, 3M in ether) via slow drop-wise addition. The mixture was stirred for 2 h at 0 °C and then warmed to ambient over 6 h. The reaction was carefully quenched via the addition a saturated aqueous solution of ammonium chloride (30 ml) and the material was taken up in water (200 ml) and ethyl acetate (150 ml), transferred to a separatory funnel and the organic phase was collected. The organic phase was washed with water (200 ml) and the ethyl acetate phase collected. The aqueous phases were

back extracted with ethyl acetate (2 x 120 ml) and the organic phases were combined, dried over magnesium sulfate, filtered and stripped to provide the desired product as a light yellow oil (16.31 g). $(M+H)^+$ = 226 m/e.

Step 4. Preparation of 2-[4-(4,4-dimethyl-4,5-dihydro-oxazol-2-yl)-3-fluoro-5-methyl-phenyl]-2-methyl-propionitrile.

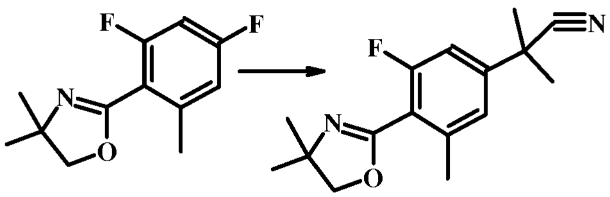

A flask containing a solution of 2-(2,4-difluoro-6-methylphenyl)-4,4-dimethyl-4,5-dihydrooxazole (14.84 g, 65.9 mmol) andobutyronitrile (9.11 g, 132 mmol) in dry tetrahydrofuran (130 ml) was cooled to -15 to -20 °C (acetonitrile/dry ice bath) under argon atmosphere. A solution of potassium bis(trimethylsily) amide (171 ml, 0.5M in toluene) was added via slow drop-wise addition. The mixture was stirred for 30 minutes at -15 °C and then gradually warmed to 15 °C over 1.5 h. The material was quenched via the addition of a saturated solution of aqueous ammonium chloride (100 ml). Water (80 ml) and diethyl ether (50 ml) were added and the material was transferred to a separatory funnel and the organic phase was collected. This was washed with an equal volume of water and the organic phase was collected. The aqueous phases were back extracted with ether (2 x 100 ml) and the combined organic phases were dried over magnesium sulfate, filtered and stripped. The material was purified by chromatography on silica gel eluting with 60% ethyl acetate / hexane to provide semi-pure product as a golden yellow oil (13.52 g, 75% pure). This material was used "as" in subsequent steps. $(M+H)^+$ = 275 m/e.

Step 5. Preparation of 2-(4-(2-cyanoporopyl-2-yl)-2-fluoro-6-methylphenyl)-3,4,4-trimethyl-4,5-dihydrooxazole-3-ium iodide.

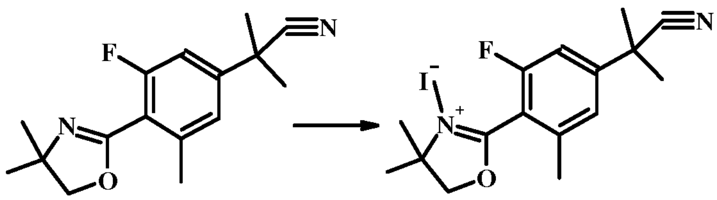

To a solution 2-[4-(4,4-dimethyl-4,5-dihydro-oxazol-2-yl)-3-fluoro-5-methyl-phenyl]-2-methylpropionitrile (23.86 g, 60% purity, 52.2 mmol) in dry acetonitrile (237 ml) was added methyl iodide (37 g, 261 mmol) via drop-wise addition over 10 minutes. The mixture was transferred to an oil bath heated to 63 °C and stirred overnight. The flask was cooled to ambient temperature and then in an ice bath. The solid precipitated product was collected by decantation to give the title compound as an off-white solid product which turned light yellow on standing (21.49 g) and was used without further purification in the next step.

Step 6. Preparation of 4-(cyano-dimethyl-methyl)-2-fluoro-6-methyl-benzoic acid

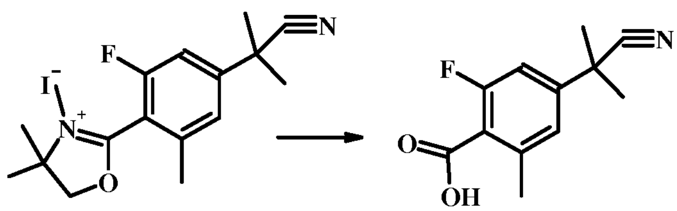

A flask was charged with 2-(4-(2-cyanoporopyl-2-yl)-2-fluoro-6-methylphenyl)-3,4,4-trimethyl-4,5-dihydrooxazole-3-ium iodide (21.9 g, 52.7 mmol ) and taken up in methanol (89 ml). To this slurry was added a solution of sodium hydroxide (10.5 g, 263 mmol) in water (178 ml) and the material was heated in an oil bath at 80 °C. The mixture was vigorously stirred for 60 minutes and then toluene (120 ml) was added. The mixture was stirred and shaken for 5 minutes in the oil bath. While still hot the material was transferred to a separatory funnel and the aqueous phase was collected. This was acidified with aqueous 1.5 N hydrochloric acid (to pH = 1). Ethyl acetate (25 ml) and water (5 ml) are added and the mixture was shaken in a separatory funnel. The organic phase was collected and the aqueous phase back extracted with ethyl acetate (2 x 40 ml). The combined organic phases were dried with magnesium sulfate, filtered and stripped to provide the title compound with impurities as a light yellow solid (5.4 g). (M-H)- = 220 m/e.

Step 7. Preparation of 4-(cyano-dimethyl-methyl)-2-fluoro-6-methyl-benzamide.

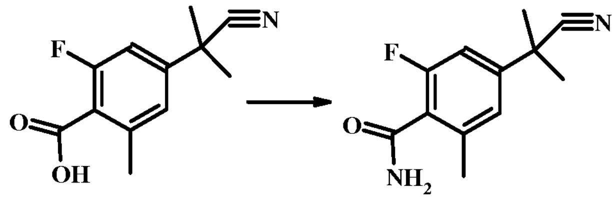

To 4-(2-cyanopropan-2-yl)-2-fluoro-6-methylbenzoic acid (14 g, 35 mmol, 75% pure) taken up in dry tetrahydrofuran (100 ml) was added 1,1'-carbonyldiimidazole (11.2 g, 69.1 mmol) in four equal portions over 15 minutes. The mixture was stirred for 2.5 h and then a 28% aqueous solution of ammonium hydroxide (20.4 ml) was added via dropwise addition. The material was stirred for 4 h and then concentrated under reduced pressure to remove 90% of the volatiles. The remainder was taken up in water (80 ml) and dichloromethane (80 ml) and shaken in a separatory funnel. The organic phase was collected and the aqueous phase was back extracted with dichloromethane (3 x 60 ml). The combined organic phase was dried (magnesium sulfate), filtered and stripped and the resultant semisolid was purified via trituration from hot dichloromethane / hexanes to provide the title compound with impurities as an off-white solid (10.71 g, 80% purity). $(M+H)^+$ = 221 m/e.

Step 8. Preparation 4-(cyano-dimethyl-methyl)-N-[1-dimethylamino-meth-(E)-ylidene]-2-fluoro-6-methyl-benzamide.

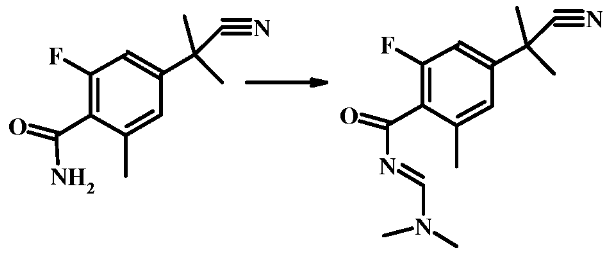

In a 250 ml round bottom flask was placed 4-(2-cyanopropan-2-yl)-2-fluoro-6-methylbenzamide (8.71 g, 31.6 mmol, 80% purity) and dimethylformamide dimethylacetal (7.26 ml, 51.4 mmol) in tetrahydrofuran (61 ml) to provide a non-homogeneous yellow suspension. The reaction mixture was heated to 63 °C (oil bath) and stirred for 3 h. The mixture was concentrated on the rotary evaporator and then taken up in hexane (80 ml). This was stirred vigorously for a few minutes until a white precipitate forms. The precipitate was collected by filtration, rinsing well with hexane to give the title compound as a white solid (7.02 g). $(M+H)^+$ = 276 m/e.

Step 9. Preparation of 2-(8-fluoro-1-oxo-1,2-dihydro-isoquinolin-6-yl)-2-methyl-propionitrile.

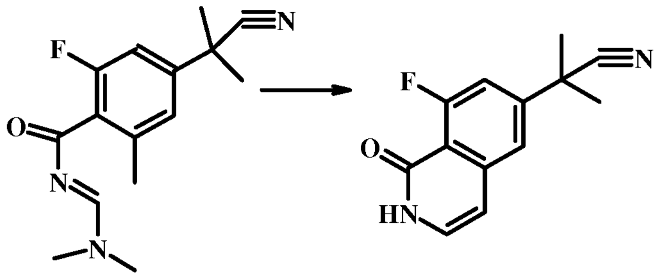

4-(Cyano-dimethyl-methyl)-N-[1-dimethylamino-meth-(E)-ylidene]-2-fluoro-6-methyl-benzamide. (10.66 g, 38.7 mmol) was taken up in dry tetrahydrofuran (100 ml) and placed in an oil bath heated to 55 °C. A solution of potassium tert-butoxide (58.1 ml, 1M in tetrahydrofuran) was added drop-wise from an addition funnel over 15 minutes. The reaction mixture was heated to 62 °C and stirred for 2 h. The solid mass which forms was cooled to ambient temperature and treated with concentrated hydrochloric acid (5.3 ml) via drop-wise addition. Water (30 ml) was added and the material was transferred to a separatory funnel. The organic phase was collected and washed with brine solution (25 ml). The aqueous phase was back extracted with ethyl acetate (25 ml) and the organics were combined, dried with magnesium sulfate, filtered and stripped. The remainder was crystallized from hot dichloromethane / hexanes to provide the desired product as an off-white solid (5.91 g). $(M+H)^+$ = 231 m/e.

Step 10. Preparation of 2-[2-(3-bromo-2-formyl-phenyl)-8-fluoro-1-oxo-1,2-dihydro-isoquinolin-6-yl]-2-methyl-propionitrile.

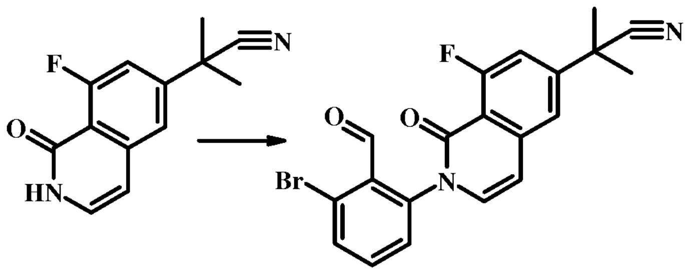

A solution of 2-(8-fluoro-1-oxo-1,2-dihydro-isoquinolin-6-yl)-2-methyl-propionitrile (250 mg, 1.09 mmol), 2,6-dibromobenzaldehyde (459 mg, 1.74 mmol) and sodium bicarbonate (182 mg, 2.17 mmol) in dry dimethylsulfoxide (8 ml) was placed under vacuum and back-filled with argon (repeat twice more). To this was added copper iodide (207 mg, 1.09 mmol) and the flask was evacuated and back-filled with argon (repeat twice more). The mixture was heated in an oil bath to 110 °C and stirred for 3.5 h. The flask was cooled to ambient and taken up in ethyl acetate (40 ml) and water (40 ml). The biphasic material was filtered through a plug of celite, rinsing well with ethyl acetate. The filtrate was transferred to a separatory funnel and the organic phase was collected. This was washed with an equal volume of 50% diluted brine solution and the ethyl acetate phase collected. The aqueous phases were back extracted with ethyl acetate (2 x 30 ml). The combined organic phase was dried with magnesium sulfate, filtered and stripped. The remainder was purified by HPLC on silica gel, eluting with 1% methanol / dichloromethane to provide the desired product as a light yellow solid (285 mg). $(M+H)^+$ = 413 / 415 m/e.

Step 11: Preparation of 5-Nitro-2H-pyrazole-3-carboxylic acid methyl ester

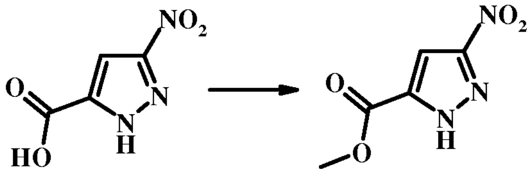

A 10mL single-neck round-bottomed flask was charged with 5-nitro-1H-pyrazole-3-carboxylic acid (4.0 g, 25.5 mmol) and anhydrous MeOH (40.0 ml). The reaction mixture was cooled to 0 °C in an ice/water cooling bath. To this mixture, thionyl chloride (7.88 g, 4.83 ml, 66.2 mmol) was added dropwise. After the addition was complete, the bath was removed and the reaction mixture was heated at reflux for 2 h. The reaction mixture was then concentrated to dryness under reduced pressure to give the desired product (4.36 g, 89.3%). $(M+Na)^+$ = 198.9 m/e

Step 12: Preparation of 2-Methyl-5-nitro-2H-pyrazole-3-carboxylic acid methyl ester

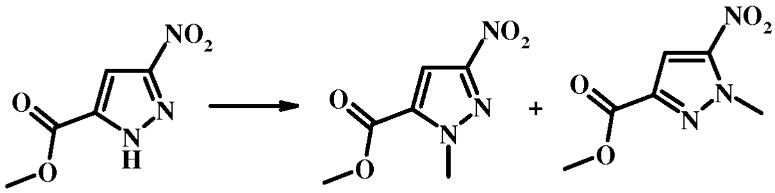

A 100-mL single-neck round-bottomed flask was charged with methyl 5-nitro-1H-pyrazole-3-carboxylate (3.89 g, 22.7 mmol), anhydrous DMF (30 ml), potassium carbonate (6.28 g, 45.5 mmol). MeI (4.19 g, 1.85 ml, 29.6 mmol) was added and the reaction mixture was stirred at room temperature for 18h. The mixture was then diluted with water (150 mL) and extracted with DCM (3 x 75mL). The combined organic layers were dried over $MgSO_4$ and concentrated under vacuum. The crude material was purified by flash chromatography (silica gel, AnaLogix system, SF40-240g column, 10% to 50% EtOAc in hexanes) to give the desired product as mixture of isomers (3.75 g). $M^+$ = 185.0 m/e

Step 13: Preparation of (2-methyl-5-nitro-2H-pyrazol-3-yl)-methanol

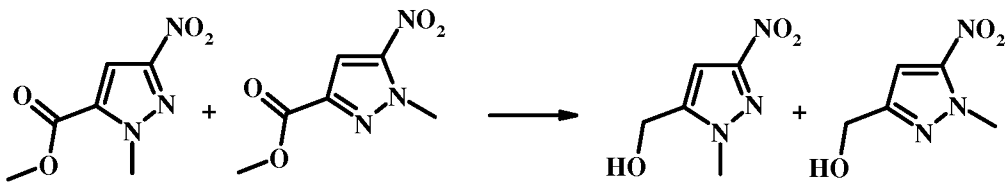

In a 250 mL three-necked flask equipped with a thermometer and nitrogen inlet, $LiBH_4$ (882 mg, 40.5 mmol) was combined with THF (30mL) to give a white suspension and cooled to 0 °C using an ice bath. To this mixture, methyl 1-methyl-3-nitro-1H-pyrazole-5-carboxylate (3.75g, 20.3 mmol) dissolved in THF (10mL) was slowly added keeping the internal temperature at 0 °C. After the addition was complete, the cooling bath was removed and the reaction mixture was stirred at room temperature for 1h. A few drops of MeOH were then added and the reaction mixture was stirred for 2h. The reaction was cooled to 0 °C using an ice bath and EtOAc (20 mL) was added followed by slow addition of water (100 mL). The layers were separated and the aqueous layer was extracted with EtOAc (3 x 100mL). The combined extracts were washed with brine (100 mL), dried over $MgSO_4$ and concentrated under vacuum. The crude material was purified by flash chromatography (silica gel, Analogix system SF40-240g, 30% to 60% EtOAc in hexanes) to give separately the title compound (2.09 g, 65.7 %) and it isomer (249 mg, 7.82 g). $(M+H)^+$ = 157.9 m/e

Step 14: Preparation of 5-bromomethyl-1-methyl-3-nitro-1H-pyrazole

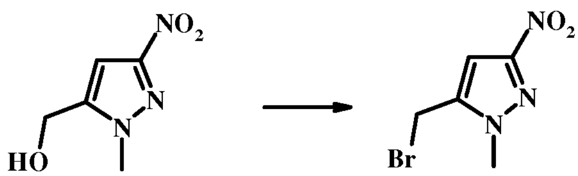

In a 250 mL round-bottomed flask (2-methyl-5-nitro-2H-pyrazol-3-yl)-methanol (2.09g, 13.3 mmol,) was combined with $CHCl_3$ (80mL) to give a white suspension. The reaction was cooled to 0 °C using an ice bath and $PBr_3$ (3.6 g, 1.25 mL, 13.3 mmol) was added dropwise. The cooling bath was removed and the reaction mixture was stirred at room temperature for 1h. The reaction was then cooled to 0 °C and diluted with DCM (100 mL). Saturated aqueous sodium bicarbonate was added (50 mL) until a pH of 8.5 was reached. The aqueous was separated and extracted with DCM (3 x 75 mL). The organic layers were combined and dried over $MgSO_4$ then concentrated under vacuum to give a crude material which was used as such for the next step (2.48 g, 84.7%). $^1H$ NMR (300 MHz, DMSO-$d_6$) $\delta$ ppm 3.96 (s, 3 H) 4.85 (s, 2 H) 7.15 (s, 1 H).

Step 15: Preparation of 5-Azetidin-1-ylmethyl-1-methyl-3-nitro-1H-pyrazole

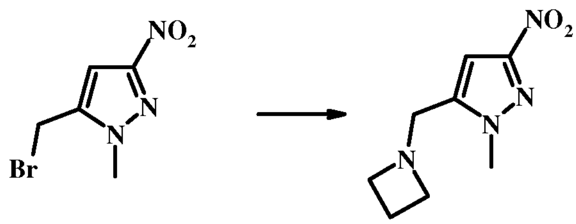

In a 50 mL round-bottomed flask, 5-(bromomethyl)-1-methyl-3-nitro-1H-pyrazole (2.48 g, 11.3 mmol) was combined with THF (60 ml) to give a light yellow solution. To this mixture were added azetidine (804 mg, 946 μl, 14.1 mmol) then DIPEA (1.75 g, 2.36 ml, 13.5 mmol) dropwise. The reaction mixture was stirred at room temperature for 24 h, and then it was diluted with EtOAc (100 mL) and washed with water (200 mL). The aqueous layer was back-extracted with EtOAc (2 x 75 mL). The combined organic layers were dried over $MgSO_4$ and concentrated under vacuum. The crude material was purified by flash chromatography (silica gel, 240 g, 1% to 5% MeOH in DCM) to give the title compound (2.21 g, 75.1 %). $(M)^+$ = 196.9 m/e.

Step 16: Preparation of 5-Azetidin-1-ylmethyl-1-methyl-1H-pyrazol-3-ylamine

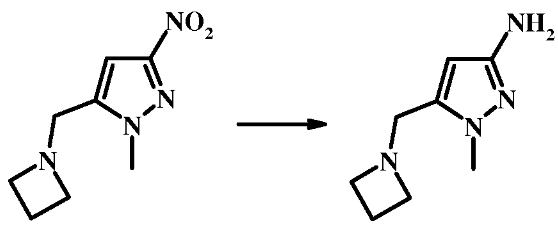

In a 100 mL round-bottomed flask,5-(azetidin-1-ylmethyl)-1-methyl-3-nitro-1H-pyrazole (1.66g, 8.46 mmol) was combined with EtOH (50mL) to give a white suspension. The reaction mixture was vacuum flushed thrice with argon then was stirred under a hydrogen atmosphere overnight. The reaction mixture was filtered through a celite pad, washed with ethanol and concentrated to give the title compound. $(M+H)^+$ = 166.9 m/e

Step 17: Preparation of 4-(5-Azetidin-1-ylmethyl-1-methyl-1H-pyrazol-3-ylamino)-6-chloro-2-methyl 2H-pyridazin-3-one

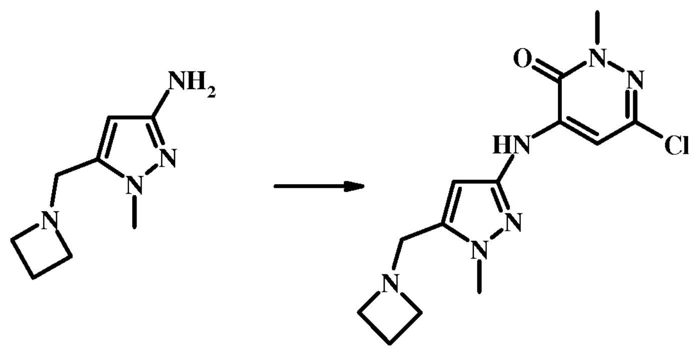

In a 25 mL round-bottomed flask, 5-(azetidin-1-ylmethyl)-1-methyl-1H-pyrazol-3-amine (1.0 g, 6.02 mmol), 4-bromo-6-chloro-2-methylpyridazin-3(2H)-one (1.34 g, 6.02 mmol), $Cs_2CO_3$ (6.86 g, 21.1 mmol) and 4,5-bis(diphenylphosphino)-9,9-dimethylxanthene (522 mg, 902 μmol) were combined with dioxane (50 ml). The resulting reaction mixture was vacuum flushed thrice with argon, then $Pd_2dba_3$ (413 mg, 451 μmol) was added and it was heated at 90 °C for 18 h. After cooling to room temperature it was diluted with 50mL of dichloromethane and water. The aqueous layer was back-extracted with DCM (2 x 25 mL). The organic layers were dried over $MgSO_4$ then concentrated under vacuum to near dryness. The precipitate was filtered off, washed with ether, then air dried overnight to give 0.93 g of the title compound as an off- white solid. The filtrate was concentrated and then purified by flash chromatography (silica gel, 24 g, 2% to 7% MeOH in DCM). The fractions containing the product were collected and combined, then concentrated to near dryness. The resulting solid was filtered off and washed with ether to give an extra 265 mg of the title compound (total 1.19 g, 64.1%). $^1H$ NMR (300 MHz, DMSO-$d_6$) δ ppm 1.96 (t, $J$=6.99 Hz, 2 H) 3.11 (t, $J$=6.99 Hz, 4 H) 3.48 (s, 2 H) 3.63 (s, 3 H) 3.71 (s, 3 H) 6.07 (s, 1 H) 7.68 (s, 1 H) 9.53 (s, 1 H).

Step 18. Preparation of 2-(2-{3-[5-(5-Azetidin-1-ylmethyl-1-methyl-1H-pyrazol-3-ylamino)-1-methyl-6-oxo-1,6-dihydropyridazin-3-yl]-2-formyl-phenyl}-8-fluoro-1-oxo-1,2-dihydro-isoquinolin-6-yl)-2-methyl-propionitrile

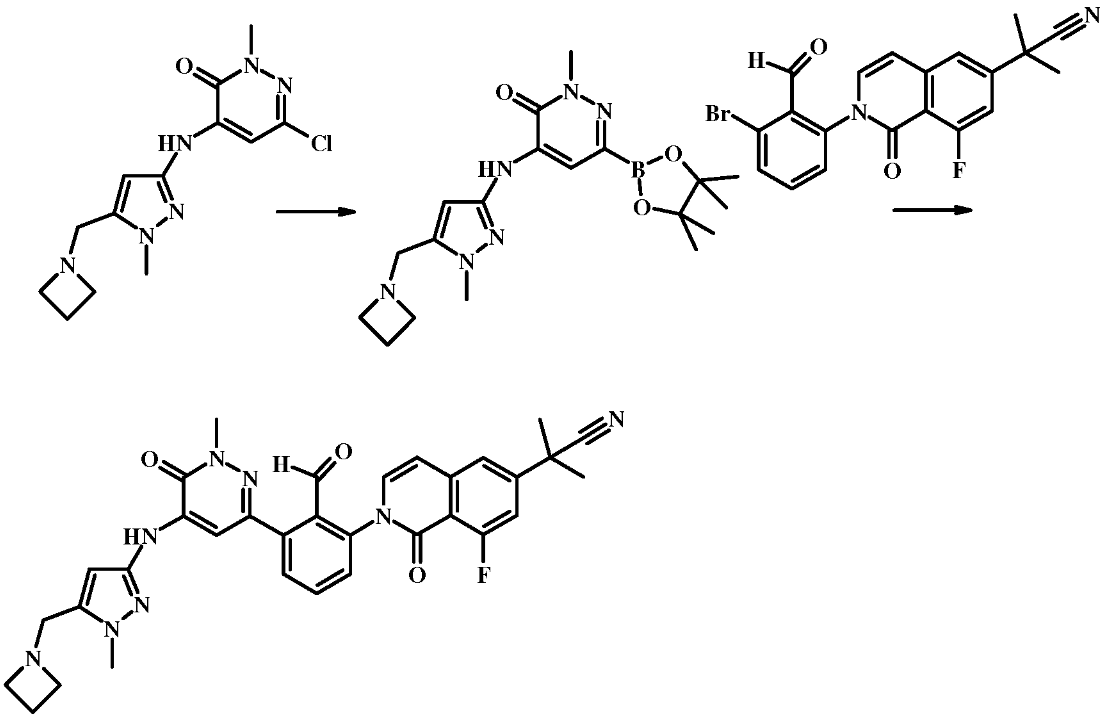

4-(5-azetidin-1-ylmethyl-1-methyl-1H-pyrazol-3-ylamino)-6-chloro-2-methyl-2H-pyridazin-3-one (219 mg, 0.71 mmol), bis(pinacolato)diboron (234 mg, 0.92 mmol), 2-dicyclohexylphosphino-2',4',6'-triisopropylbiphenyl (x-phos, 51 mg, 0.106 mmol) and potassium acetate (209 mg, 2.13 mmol) were taken up in dry dioxane (6.6 ml) and placed under

vacuum and back-filled with argon (step repeated five times). To this mixture was added palladium acetate (17.4 mg, 0.077 mmol) and the flask was evacuated and back-filled with argon (step repeated five times). The mixture was heated at 100 °C and stirred for 16 minutes. The flask was cooled to ambient and the crude contents of this flask were filtered (through celite, rinse through with 3 ml dioxane) into a second flask (immersed in a 110 °C oil bath under argon balloon) which contained a vacuum de-gassed solution of the following reagents: 2-(2-(3-bromo-2-formylphenyl)-8-fluoro-1-oxo-1,2-dihydroisoquinolin-6-yl)-2-methylpropanenitrile (275 mg, 0.665 mmol), potassium carbonate (460 mg, 3.3 mmol), tricyclohexylphosphine (57.1 mg, 0.204 mmol) and bis(dibenzylideneacetone)palladium (57.4 mg, 0.0998 mmol) in a mixture of n-butanol (0.825 ml), dioxane (3.44 ml) and water (2.58 ml). The flask was stirred and heated for 1 h and then cooled to ambient temperature. The crude was filtered through a short plug of celite, rinsing well with ethyl acetate (40 mL). To the filtrate was added water (40 ml) and the material was shaken in a separatory funnel. The organic phase was collected and the aqueous phase was back extracted with ethyl acetate (2 x 20 ml). The combined organic phases were dried with sodium sulfate, filtered and stripped. The resulting crude was purified by preparatory HPLC on silica gel (Analogix SF15-24 g column), eluting with 1% to 13% methanol / dichloromethane to provide the desired product as a yellow-brown solid (282 mg). $(M-H)^-$ = 646 m/e.

Step 19. Preparation of 2-(2-{3-[5-(5-azetidin-1-ylmethyl-1-methyl-1H-pyrazol-3-ylamino)-1-methyl-6-oxo-1,6-dihydro-pyridazin-3-yl]-2-hydroxymethyl-phenyl}-8-fluoro-1-oxo-1,2-dihydro-isoquinolin-6-yl)-2-methyl-propionitrile

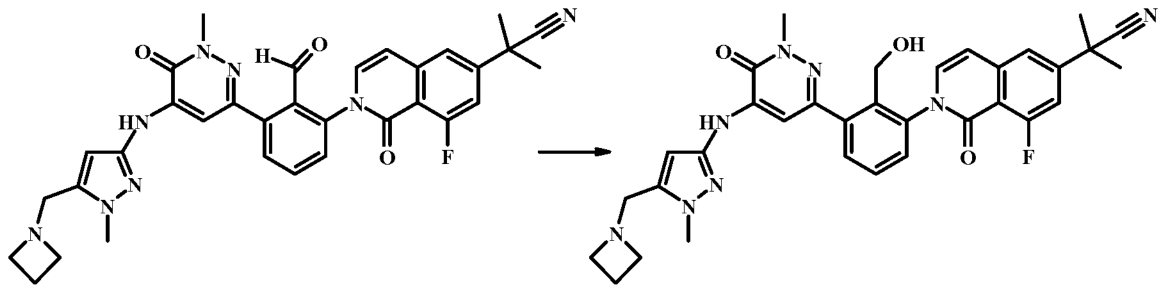

A solution of 2-(2-{3-[5-(5-Azetidin-1-ylmethyl-1-methyl-1H-pyrazol-3-ylamino)-1-methyl-6-oxo-1,6-dihydro-pyridazin-3-yl]-2-formyl-phenyl}-8-fluoro-1-oxo-1,2-dihydro-isoquinolin-6-yl)-2-methyl-propionitrile (792 mg, 1.31 mmol) in methanol (10 ml) and dichloromethane (10 ml) was cooled in an ice bath. To this yellow homogenous solution was added a solution of sodium borohydride (247 mg, 6.53 mmol) in water (1.5 ml). The mixture was stirred for 2 minutes then warmed up to ambient temperature. After stirring for about 5 mins, LC/MS showed complete conversion. Water (60 ml) and dichloromethane (50 ml) were added. The organic phase was collected, washed with an equal volume of 50% diluted brine solution. The aqueous phases were back extracted with dichloromethane (2 x 40 ml). The combined organic phases were dried with magnesium sulfate, filtered and stripped. The resulting crude material was purified by preparatory HPLC on silica gel (Analogix SF15-24 g column), eluting with 1% to 15% methanol / dichloromethane to provide a yellow foamy solid. Further crystallization fromo-propyl acetate/hexanes gave a product which was further triturating from 2:1 ether/iso-propyl acetate. o-propyl acetate was detected in the resulting product. Nanopure water (8-10 drops) was added and the resulting mixture was placed in a sonicator for 2 minutes, the volatile was stripped using a vacuum oven to provide 471 mg of the title compound as an off-white powder. $(M+H)^+$ = 609 m/e, $^1H$ NMR (300 MHz, $CDCl_3$-*d*) δ ppm 1.81 (s, 6 H) 2.12 (quin, J = 7.20 Hz, 2H) 3.27 (t, J = 7.0 Hz, 4 H) 3.55 (s, 2 H) 3.81 (s, 3 H) 3.88 (s, 3 H) 4.03 - 4.19 (m, 1 H) 4.22 - 4.44 (m, 2 H) 5.94 (s, 1 H) 6.61 (dd, *J*=7.36, 2.08 Hz, 1 H) 7.21 (dd, *J*=12.09, 1.89 Hz, 1 H) 7.35 (d, *J*=7.55 Hz, 1 H) 7.41 (dd, *J*=7.74, 1.32 Hz, 1 H) 7.52 (d, *J*=1.51 Hz, 1 H) 7.56 (t, *J*=7.74 Hz, 1 H) 7.65 (dd, *J*=7.55, 1.51 Hz, 1 H) 7.82 (s, 1 H) 7.91 (s, 1 H).

**Preparation of Compound 3**

Step 1. Preparation of (5-bromo-1-methyl-2-oxo-1,2-dihydro-pyridin-3-yl)-[5-(morpholine-4-carbonyl)-pyridin-2-yl]-carbamic acid tert-butyl ester.

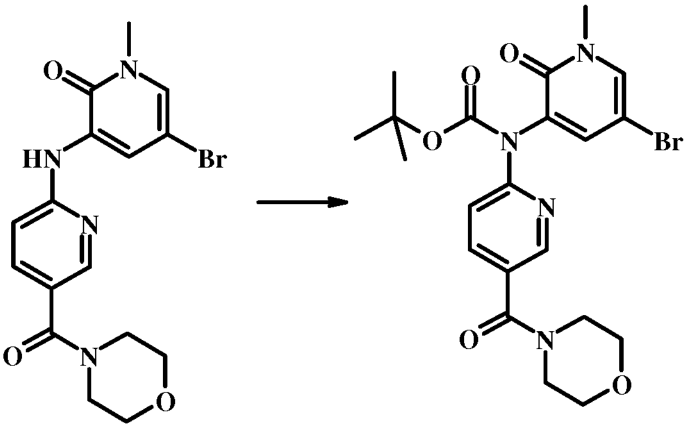

In a 250ml round-bottomed flask, 5-bromo-1-methyl-3-(5-(morpholine-4-carbonyl)pyridin-2-ylamino)pyridin-2(1H)-one (10 g, 25.4 mmol) was dissolved in THF (125 ml). Sodium hydride (1.12 g, 28.0 mmol) was added in portions over 10 minutes and stirred for 10 minutes. Then di-tert-butyl dicarbonate (6.12 g, 6.51 ml, 28.0 mmol) was added. Another 20 ml of THF were added because the red suspension did not stir. It was heated to 70 °C and stirred overnight. The suspension turned brown. The reaction was cooled to room temperature, then 125 ml of water and 125 ml of ethyl acetate were added and the layers were separated. The organic layer was washed with 100 ml of brine, dried over magnesium sulfate and concentrated to give 11.8 g of an orange-brown powder. 80 ml of ethyl acetate was added and the less soluble material was filtered off. The filtrate was evaporated to give a brown foam. The recovered powder on the filter funnel contained both the starting material and the product and so did the filtrate. The powder and filtrate were combined, dissolved in methylene chloride and evaporated to give the title compound alongside about 20% of starting material (10.71 g, 21.7 mmol, 85.4 % yield). $(M+H)^+$ = 494.9 m/e.

Step 2. Preparation of (5-bromo-1-methyl-2-oxo-1,2-dihydro-pyridin-3-yl)-(5-formyl-pyridin-2-yl)-carbamic acid tert-butyl ester

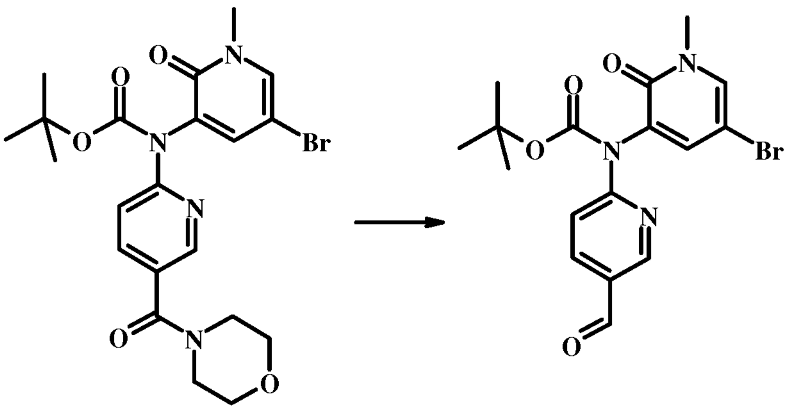

To a slurry of bis(cyclopentadienyl)zirconium chloride hydride (9.37 g, 36.3 mmol) in THF (180 ml) was added a solution of tert-butyl 5-bromo-1-methyl-2-oxo-1,2-dihydropyridin-3-yl(5-(morpholine-4-carbonyl)pyridin-2-yl)carbamate (13.7 g, 27.8 mmol) and tetrahydrofuran (350 ml) all at once. The orange solution was stirred at room temperature for 2 h. LCMS showed reaction almost completed after 1.5 h. The reaction mixture was poured onto 110 g of silica in 180 ml of ethyl acetate and stirred for 10 minutes. It was filtered and concentrated to give the title compound (10.7 g, 26.2 mmol, 94.4 % yield) as a light brown powder. $(M-BOC)^+$ = 309.9 m/e.

Step 3. Preparation of (5-bromo-1-methyl-2-oxo-1,2-dihydro-pyridin-3-yl)-{5-[(2-methoxy-ethylamino)-methyl]-pyridin-2-yl}-carbamic acid tert-butyl ester

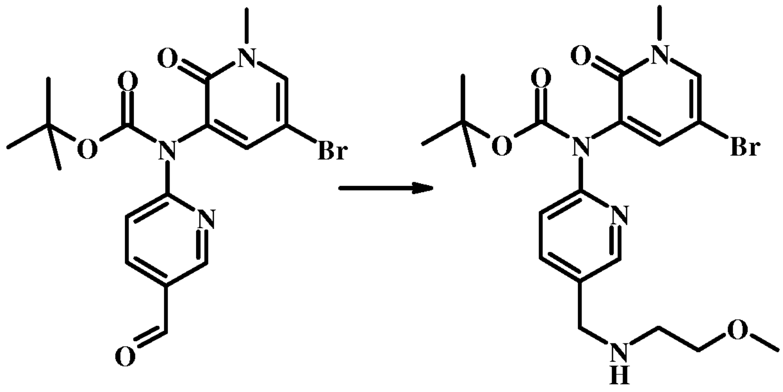

In a 250 ml round-bottomed flask tert-butyl 5-bromo-1-methyl-2-oxo-1,2-dihydropyridin-3-yl(5-formylpyridin-2-yl)carbamate (5 g, 12.2 mmol) was suspended in methylene chloride (122 ml) followed by 2-methoxyethanamine (2.3 g, 2.66 ml, 30.6 mmol), sodium triacetoxyborohydride (6.49 g, 30.6 mmol) and acetic acid (1.47 g, 1.4 ml, 24.5 mmol). The yellow cloudy solution was heated at 40°C overnight. It was foaming and turned dark green overnight (LCMS overnight showed reaction was complete). The reaction mixture was cooled to room temperature, and then transferred into a separation funnel with 50 ml methylene chloride and extracted with 100ml of sat. $NaHCO_3$ The organic layer was separated, dried over $MgSO_4$, filtered and concentrated to give 6.1 g of a dark green oil, which was dissolved with methylene chloride and methanol and concentrated onto Celite. Purification by flash chromatography (AnaLogix Intelli-Flash 280, 50 g silica gel column, 10-75% "magic base" (14%MeOH in DCM+0.14%$NH_4OH$)/methylene chloride) afforded 1.04 g of a green-blue foam, which was re-dissolved in methanol three times and then concentrated and finally dried on the high vacuum pump overnight to give the title compound (1.0 g, 2.14 mmol, 17.5 % yield) as a green-blue foam. The mixed fractions 10-27 were also evaporated to give 2.5 g of a dark green oil, which was again dissolved with methylene chloride and methanol, concentrated onto Celite. Further purification by flash chromatography (AnaLogix IntelliFlash 280, 80 g silica gel column, 1-10% methanol/ methylene chloride, then 75% "magic base" (14%MeOH in DCM+0.14%$NH_4OH$)/DCM) afforded another 1.2 g of the title compound as a green-blue oil. $(M+H)^+$ = 469.0 m/e.

Step 4. Preparation of 5-bromo-3-{5-[(2-methoxy-ethylamino)-methyl]-pyridin-2-ylamino}-1-methyl- 1H-pyridin-2-one

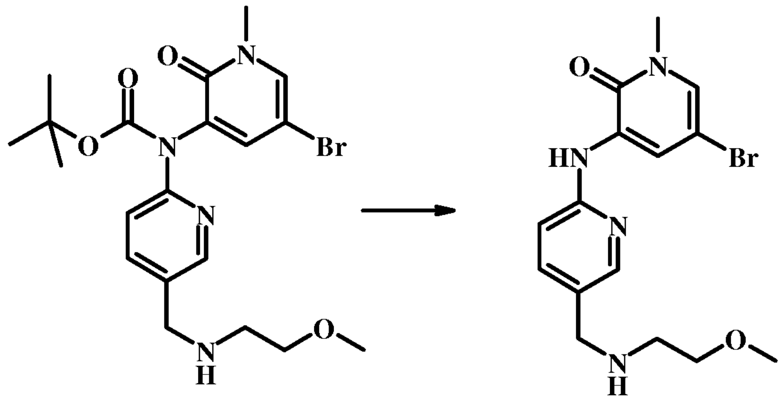

To tert-butyl 5-bromo-1-methyl-2-oxo-1,2-dihydropyridin-3-yl(5-((2-methoxyethylamino)methyl)pyridin-2-yl)carbamate (2.2 g, 4.71 mmol) in a 250 ml round-bottom flask containing DCM (25 ml) was added TFA (7.4 g, 5 ml, 64.9 mmol). The reaction was stirred for 2 h. LCMS showed the reaction to be incomplete. The reaction continued stirring for an additional 1.5 h. LCMS showed reaction was still incomplete, but making progress. After stirring for 3 more hours, the reaction was complete. The solvent was evaporated, the residue placed under vacuum overnight then dissolved in DCM. Aq. sat $NaHCO_3$ was added and the mixture was stirred vigorously. A solid formed. The mixture was stirred until all gas evolution had ceased. The solid had re-dissolved in the organic layer. The layers were separated. The aqueous layer was extracted once with DCM. The organic layers were combined, dried over $Na_2SO_4$ then concentrated under vacuum to give the title compound (1.8 g, 4.9 mmol). $(M)^+$ = 366.9 m/e.

Step 5. Preparation of 2-(6-tert-Butyl-1-oxo-3,4-dihydro-1H-isoquinolin-2-yl)-6-chloro-benzaldehyde one

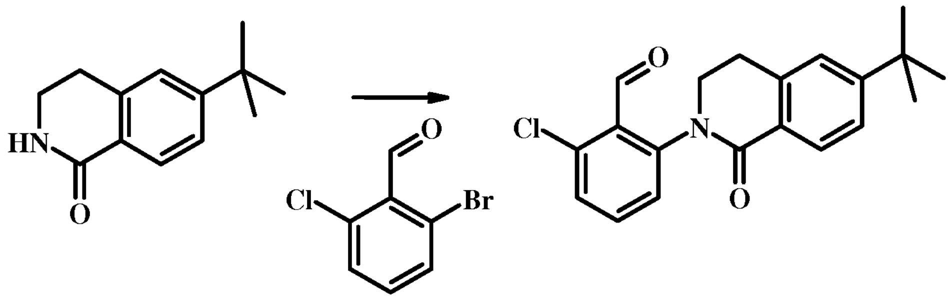

Method A

6-tert-butyl-3,4-dihydroisoquinolin-1(2H)-one (10.81 g, 53.2 mmol), 2-bromo-6-chlorobenzaldehyde (15.2 g, 69.1 mmol), Xantphos (3.08 g, 5.32 mmol) and cesium carbonate (43.3 g, 133 mmol) were suspended in dioxane (216 ml). The reaction mixture was degassed with argon. Finally bis(dibenzylideneacetone)palladium (2.29 g, 3.99 mmol) was added. The reaction mixture was stirred at 100 °C (external temperature) for 3.5 h. The whole reaction mixture was cooled to room temperature and filtered through a plug of celite. It was washed with dioxane (100 ml) and the filtrate was concentrated. The crude was treated with ethyl acetate: the precipitate was filtered off to give 13.8 g of a yellow solid and the filtrate was concentrated to give 19.46 g of an orange solid. The yellow solid (filter cake) was suspended in dichloromethane then filtered and washed with dichloromethane (100 ml). Filtrate was concentrated under vacuum to give the title compound (11.68 g) as a yellow solid. $(M)^+$ = 341.9 m/e.

Method B

A 1L Atlas reactor was charged with 6-tert-butyl-3,4-dihydroisoquinolin-1(2H)-one (80 g, 394 mmol), 2-bromo-6-chlorobenzaldehyde (90.7 g, 413 mmol), palladium(II) acetate (1.77 g, 7.87 mmol), Xantphos (6.83 g, 11.8 mmol) and $K_2CO_3$ (109 g, 787 mmol). Then the reactor was evacuated and backfilled with nitrogen. This sequence was repeated three times. DMF (604 g, 640 ml) was added then the reactor was heated to 90 °C for 16.5 h then cooled down to ~70°C. Water (3 vol.; 240 mL) was added to crush out the product. After aging at 70°C for 2 h, the reactor was cooled down to room temperature (aqueous layer with salt was observed.). The material was collected by filtration, washed with water to remove some inorganics then water/IPA and then air-dried over the weekend to give 2-(6-tert-butyl-1-oxo-3,4-dihydroisoquinolin-2(1H)-yl)-6-chlorobenzaldehyde (123.5 g, 361 mmol, 91.8 % yield) as a yellowish solid with 97% HPLC purity. An analysis showed Pd residues in the material. The material was dissolved in 650 mL DMF while heating to 80°C then a 240 mL aqueous solution containing 16 g of N-acetyl-L-cysteine was added slowly. The mixture was stirred for an additional 5 hr at 80°C; crystallization was observed during this process. The mixture was cooled down to ambient temperature slowly. The material was collected by filtration, washed with water/IPA, and then dried in a vacuum oven at 80 °C overnight. 109 g of the title compound were recovered with a 99.5+% HPLC purity.

Step 6. Preparation of 6-tert-butyl-2-(3-chloro-2-hydroxymethyl-phenyl)-3,4-dihydro-2H-isoquinolin-1-one

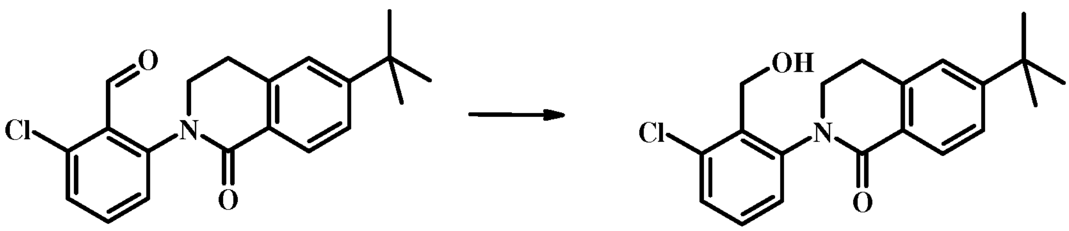

Method A

2-(6-tert-butyl-1-oxo-3,4-dihydroisoquinolin-2(1H)-yl)-6-chlorobenzaldehyde (3.04 g, 8.89 mmol) was dissolved in THF (46.1 ml) and MeOH (4.61 ml). The mixture was cooled to -40°C (external temperature). Sodium borohydride (404 mg, 10.7 mmol) was added in portions (3 x 50 mg, then 25 mg). The reaction mixture was allowed to warm up to 0°C then stirred 30 min at 0°C. An ammonium chloride solution was added and the reaction mixture was extracted with EtOAc. The organic phase was washed with water, brine and then dried over sodium sulfate. After filtrate, it was concentrated under vacuum to give the title compound 2.38 g as a light yellow foam. $(M)^+$ = 344.0 m/e.

Method B

A 1L Atlas reactor was charged with 2-(6-tert-butyl-1-oxo-3,4-dihydroisoquinolin-2(1H)-yl)-6-chlorobenzaldehyde (80 g, 234 mmol) and the material was dissolved with DCM (634 g, 480 ml) at room temperature. IPA (250 g, 320 ml) was added to the solution, which was subsequently cooled down to ~2°C. $NaBH_4$ (4.43 g, 117 mmol) was added portion wise for 15 min, during which time the temperature jumped to ~ 9°C. The reaction mixture was stirred for 1 hr at ~2°C to give a clean homogeneous solution, which was then quenched with water (320 g, 320 ml). The DCM was distilled off with heating at ~60-80 °C. After complete removal of DCM, the product was crystallized out from an IPA/$H_2O$ solution (1/1, ~8 vol.) with no need for phase separation. After aging for 3-4 hrs at 80 °C, the mixture was cooled down to ambient temperature slowly and stirred overnight. The material was collected by filtration, washed with IPA/$H_2O$ (1/1), and dried in a vacuum oven at 80°C over the weekend to give the title compound (77.7 g, 226 mmol) with 99.4% purity by HPLC.

Step 7. Preparation of acetic acid 2-(6-tert-butyl-1-oxo-3,4-dihydro-1H-isoquinolin-2-yl)-6-chloro-benzyl ester

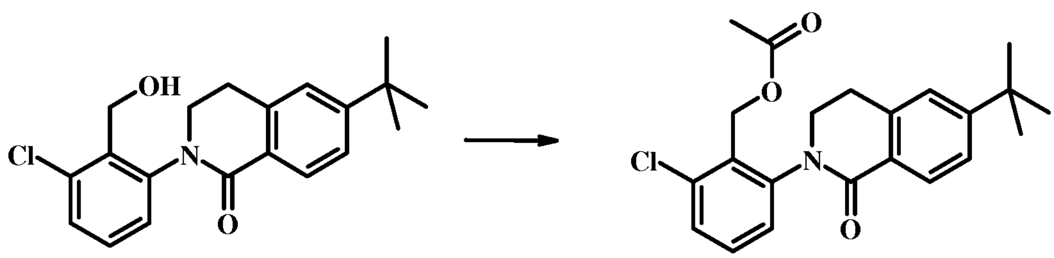

In a 250 mL round-bottomed flask, 6-tert-butyl-2-(3-chloro-2-(hydroxymethyl)phenyl)-3,4-dihydroisoquinolin-1(2H)-one (21.9 g, 63.7 mmol) was combined with acetic anhydride (32.5 g, 30.0 ml, 318 mmol) and pyridine (15.1 g, 15.5 ml, 191 mmol) in DCM (600 ml) to give a colorless solution. The reaction mixture was stirred overnight. The crude reaction mixture was concentrated under vacuum to give a tan oil. The residue was dissolved in DCM then washed with water, dried $MgSO_4$, evaporated and purified by column chromatography (0% to 25% EtOAc/Hex over 10 min, then hold at 25% for 20 min) to give the title compound (22.9 g, 59.3 mmol). $^1H$ NMR (400 MHz, $CDCl_3$) $\delta$ ppm 1.32 - 1.45 (m, 9 H) 2.06 (s, 3 H) 3.05 (dt, J=15.73, 4.77 Hz, 1 H) 3.35 (ddd, J=15.98, 10.93, 5.43 Hz, 1 H) 3.79 (dt, J=11.94, 5.27 Hz, 1 H) 3.95 - 4.14 (m, 1 H) 5.07 - 5.39 (m, 2 H) 7.21 (dd, J=7.71, 1.39 Hz, 1 H) 7.27 (d, J=1.77 Hz, 1 H) 7.36 - 7.47 (m, 3 H) 8.06 (d, J=8.08 Hz, 1 H).

Step 8. Preparation of acetic acid 2-(6-tert-butyl-1-oxo-3,4-dihydro-1H-isoquinolin-2-yl)-6-(4,4,5,5-tetramethyl-[1,3,2]dioxaborolan-2-yl)-benzyl ester

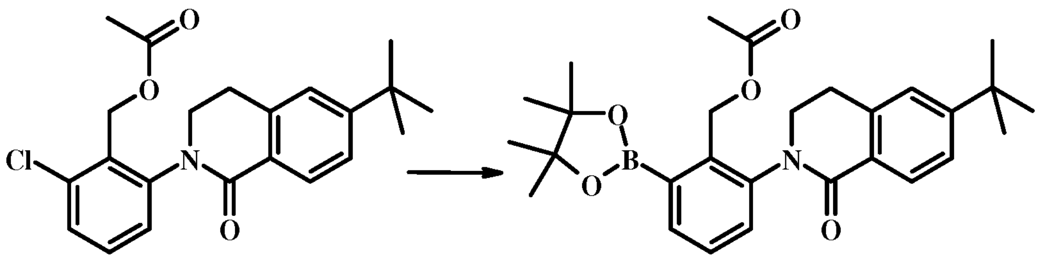

2-(6-tert-butyl-1-oxo-3,4-dihydroisoquinolin-2(1H)-yl)-6-chlorobenzyl acetate (5 g, 13.0 mmol) was dissolved in dioxane (50.0 ml) while heating and X-PHOS (618 mg, 1.3 mmol), potassium acetate (2.6 g, 26.5 mmol), bis(pinacolato)diboron (4.28 g, 16.8 mmol) were added. The suspension was degassed again by sonicating under vacuum and back filling with argon. Finally palladium (II) acetate (145 mg, 648 μmol) was added and the mixture was stirred at 75 °C (external temperature) for 2 hr. Reaction was not complete after 2 h. Additional aliquots of palladium (II) acetate and X-Phos were added. The reaction was stirred at 75 °C for 3 more hours. LCMS showed some remaining starting material and some des-chloro by-product. Heating at 75 °C was continued overnight, and then the reaction mixture was cooled to room temperature. The reaction mixture was filtered through celite and washed with dioxane. The filtrate was concentrated under vacuum to give a brown gum, which was dissolved in dichloromethane (5 ml) and purified by flash chromatography (silica gel, 220 G, 10% to 100% EtOAc in hexanes [Hold at 10% for 5 min, then to 100% over 15 min.]) to give a yellow gum containing about 60% of the title compound and about 40% of the des-chloro by-product, which was used as such in the next step. Product $(M+H)^+$ = 478.1 m/e. Des-chloro by-product $(M+H)^+$ = 452.0 m/e.

Step 9. Preparation of acetic acid 2-(6-tert-butyl-1-oxo-3,4-dihydro-1H-isoquinolin-2-yl)-6-( 5-{5-[(2-methoxy-ethylamino)-methyl]-pyridin-2-ylamino}-6-oxo-1,6-dihydro-pyridin-3-yl)-benzyl ester

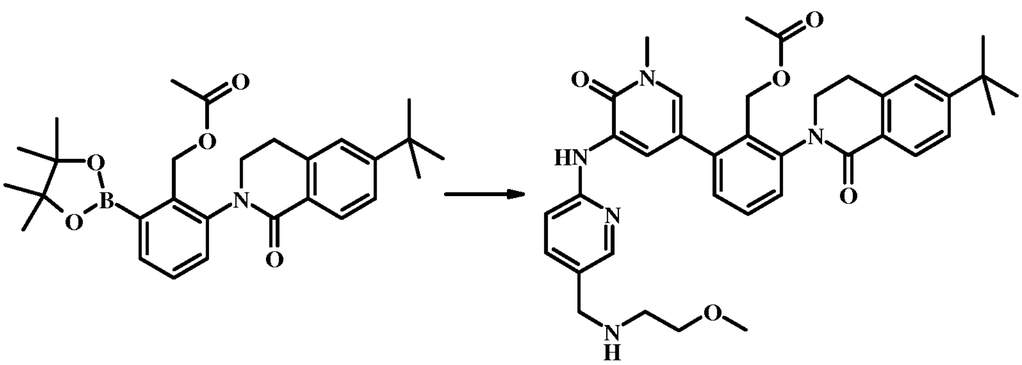

2-(6-tert-butyl-1-oxo-3,4-dihydroisoquinolin-2(1H)-yl)-6-(4,4,5,5-tetramethyl-1,3,2-dioxaborolan-2-yl)benzyl acetate (5.8 g, 7.29 mmol) and 5-bromo-3-(5-((2-methoxyethylamino)methyl)pyridin-2-ylamino)-1-methylpyridin-2(1H)-one (2.68 g, 7.29 mmol) were combined in a 250 ml round bottom flask. Potassium phosphate tribasic (3.09 g, 14.6 mmol) was added, followed by X-PHOS (348 mg, 729 μmol) and bis(dibenzylideneacetone)palladium (210 mg, 364 μmol). The solvent solution, BuOH (60 ml) and water (15.0 ml) were added. The reaction mixture was flushed twice with argon. The vessel was closed and heated to 100 °C, (external temperature) for 1.5h. DCM and water were added and the DCM phase was separated, filtered through celite and purified by chromatography using a gradient of 0% to 5% methanol in DCM over 20 min, followed by 50% Magic Base in DCM for 30 more min) to give the title compound alongside the des-acetate and some impurities. The mixture was used as such in the next step. Product $(M+H)^+$ = 638.1 m/e. Des-acetate by-product $(M+H)^+$ = 596.1 m/e.

Step 10. Preparation of 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one

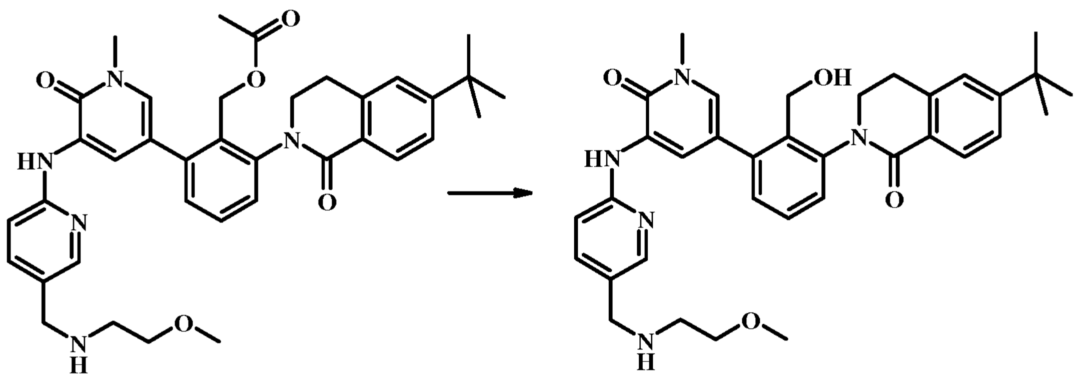

To 2-(6-tert-butyl-1-oxo-3,4-dihydroisoquinolin-2(1H)-yl)-6-(5-(5-((2-methoxyethylamino)methyl)pyridin-2-ylamino)-1-methyl-6-oxo-1,6-dihydropyridin-3-yl)benzyl acetate (3.18 g, 4.99 mmol) dissolved in THF was added sodium hydroxide (24.9 ml, 24.9 mmol) at room temperature. The reaction mixture was heated at 60 °C for two hrs. LCMS showed starting material still present. The reaction mixture was stirred at 60 °C, overnight and LCMS showed reaction was complete. It was cooled to room temperature, diluted with sat NaHCO3 and DCM. The layers were separated. The aqueous layer was extracted three times with DCM. The organics were dried over $Na_2SO_4$ then concentrated under vacuum. The residue was triturated with ether, which turned into a gum. The solvent was evaporated then EtOAc was added until the residue was dissolved. $Et_2O$ was added. A solid formed, and then turned to gum. The solvent was evaporated to give a crude yield of 3.18 g. The solid was placed in a round-bottom flask with a stirring bar placed at 50 °C (external temp). 30 ml of acetone were slowly added. All went into solution. The vessel was closed with a Teflon stopper. The reaction mixture was stirred for about 30 min, and then a solid started to form. The mixture was stirred at 50 °C for 72 h. After 72 h, the thick suspension was cooled to room temperature. The solid was filtered, washed with ice cold acetone and place in a vacuum oven at 50 °C for 2 h then at room temperature overnight to give the title compound. $(M+H)^+$ =596.1 m/e.

$^1$H NMR (400 MHz, $CHCl_3$-d) δ ppm 1.39 (s, 9 H) 2.70 - 2.90 (m, 2 H) 3.11 - 3.22 (m, 1 H) 3.25 - 3.34 (m, 1 H) 3.37 (s, 3 H) 3.51 - 3.60 (m, 2 H) 3.72 (s, 3 H) 3.78 (s, 2 H) 3.81 - 3.89 (m, 1 H) 4.12 (ddd, J=12.25, 9.85, 4.67 Hz, 1 H) 4.28 - 4.47 (m, 2 H) 4.63 (d, J=10.61 Hz, 1 H) 6.83 (d, J=8.59 Hz, 1 H) 7.27 (dd, J=7.45, 1.64 Hz, 2 H) 7.31 (d, J=1.52 Hz, 1 H) 7.41 - 7.53 (m, 4 H) 7.60 (dd, J=8.59, 2.27 Hz, 1 H) 7.93 (s, 1 H) 8.11 (d, J=8.08 Hz, 1 H) 8.18 (d, J=2.02 Hz, 1 H) 8.70 (d, J=2.27 Hz, 1 H).

Formulation Data

**Formulation of reference compound 1 for PD experiments (mOVA36)**

10.6 g of reference compound 1 was micronized in a Jet-O-Mizer jet mill at feed and grinding pressures of 90 psi. The micronized material thus obtained was analyzed by powder X-ray diffraction pattern and particle size distribution as shown in Figures 1 and 2 respectively. No change in polymorphic form was observed during the micronization process. Eighty percent of the particles were of size 5 μm or less with a mean particle size (d50% = 2.88 μm) in the range suitable for inhalation delivery. The material was used for the in vivo experiment mOVA36. See Figures 1 and 2.

**Formulation of reference compound 1 for PD experiments (mOVA 40, mPolyIC-3-12 and mPolyIC-3-12)**

8.6 g of 2-(6-tert-butyl-8-fluoro-1-oxophthalazin-2(1H)-yl)-4-(1-methyl-5-(5-(1-methylpiperidin-4-yl)pyridin-2-ylamino)-6-oxo-1,6-dihydropyridazin-3-yl)nicotinaldehyde was micronized in a Jet-O-Mizer jet mill at 80 psi. The mean particle size (d50%) was 3.05 μm with 90% particles ≤ 6 μm (Figure 3). This material (30% w/w) and lactose (Lactohale LH 300, 70% w/w) were mixed in a Turbula mixer at 22 rpm for 15 minutes. The d50% and d90% particle size of the lactose ingredient were < 5 μm and ≤ 10 μm, respectively (DFE Pharma specifications (# 001/October 2011). The blend thus obtained was used for the in vivo experiment mOVA40, mPolyIC-3-12 and mPolyIC-5-12. See Figure 3.

**Formulation of reference compound 1 for PD experiment mOVA 20**

Mixtures of reference compound 1 in a buffer at two different concentrations (0.15 and 1.5 mg/mL) were prepared as follows

Preparation of a 10 mL of a reference compound 1 solution of concentration 1.5 mg/mL

The fumarate salt of reference compound 1 (17.8 mg) was mixed with 2 mL of propylene glycol (PG). The preparation

was sonicated for about 20-30 minutes until the drug was fully dissolved. A sufficient quantity of purified water was added to 10.0 g. The resulting mixture was stirred by vortex to get a clear, homogeneous solution (pH 4.4).
Preparation of a 10 mL of a reference compound 1 solution of concentration 0.15 mg/mL
1.0 g of the 1.5 mg/mL solution prepared as above was placed into a vial. A sufficient quantity of vehicle (20% PG/80% Water) was added to 10.0 g. The resulting mixture was stirred by vortex.

**Formulation of reference compound 2 for PD experiment mOVA 50**

Mixtures of reference compound 2 in a buffer at three different concentrations (0.15, 0.5 and 1.5 mg/mL) were prepared as follows:

Preparation of a reference compound 2 solution of concentration 1.5 mg/mL
Reference compound 2 (1.65 mg) was placed in a vial. A sufficient quantity of vehicle (20% PG/80% water) was added to 1.1 g. The preparation was sonicated for about 2 minutes. A fine suspension was achieved (pH 4.833).

Preparation of a reference compound 2solution of concentration 0.5 mg/mL
0.1740 g of the 1.5 mg/mL suspension prepared as above was placed into a vial. A sufficient quantity of vehicle (20% PG/80% Water) was added to 0.521 g and the preparation was mixed.

Preparation of a reference compound 2 solution of concentration 0.15 mg/mL
0.07716 g of the 1.5 mg/mL suspension prepared as above was placed into a vial. A sufficient quantity of vehicle (20% PG/80% Water) was added to 0.7717 g. The pH of the resulting preparation was 4.71.

**Formulation of compound 3 for PD experiment mOVA 49**

Mixtures of compound 3 in a buffer at three different concentrations (0.15, 0.5 and 1.5 mg/mL) were prepared as follows

Preparation of vehicle (30 mL)

| **Ingredients for vehicle preparation** | | |
|---|---|---|
| | **% w/w** | **g per 30 mL** |
| Citric Acid anhydrous | 0.080 | 0.0240 |
| Sodium Citrate dihydrate | 0.171 | 0.0513 |
| Sodium Chloride | 0.90 | 0.270 |
| Polysorbate 80 | 0.20 | 0.060 |
| 1. In a beaker, weigh directly 0.06 g Polysorbate 80.<br>2. Add about 26 g of water and stir until dissolved.<br>3. Add citric acid, sodium citrate and sodium chloride. . Stir until dissolved. QS to 30 mL<br>5. Take pH = 4.68. | | |

Preparation of a compound 3 solution of concentration 1.5 mg/mL

Compound 3 (1.59) was placed in a vial. A sufficient quantity of the vehicle as prepared above was added to 1.06 g. The preparation was sonicated for about 2 minutes. A fine suspension was achieved (pH 4.85).

Preparation of a reference compound 2 solution of concentration 0.5 mg/mL

0.16773 g of the 1.5 mg/mL suspension prepared as above was placed into a vial. A sufficient quantity of the vehicle as prepared above was added to 0.50735 g and the preparation was mixed.

Preparation of a reference compound 2 solution of concentration 0.15 mg/mL

0.06675 g of the 1.5 mg/mL suspension prepared as above was placed into a vial. A sufficient quantity of the vehicle as prepared above was added to 0.65608 g. The pH of the resulting preparation was 4.70.

Biological Examples

**Btk Biacore binding assay**

Analyses were performed on a Biacore T-100. Btk kinase was avi-tagged and co-expressed in baculovirus with biotin ligase to generate single site biotinylated Btk (Avidity, LLC). Biotinylated target was captured on Biacore streptavidin sensor chips (Series S Sensor Chip SA) at densities of ~10000 resonance units (lru ~1pg protein/mm2). Test compounds were solubilized from powders in 100% DMSO for 10mM stocks and diluted in an 8 point, 2-fold series ranging from 0.78nM to 100nM. Compounds were injected for 100 seconds association time and dissociation was followed for 20 min. Experiments were performed at 25°C and no regeneration buffer was utilized and therefore no more than two high affinity compounds were analyzed per chip. The running buffer consisted of 50mM Hepes pH 7.2, 150mM NaCl, 10mM MgCl2, 2mM MnC12, 1mM TCEP, 1%PEG 3350, 5%DMSO. Kinetic analysis was performed using Biacore BIAevaluation software using a simple model for 1:1 (Langmuir) binding. Data from these experiments indicated that the off rate of reference Compound 1, reference Compound 2 and Compound 3 were very slow and the obtained KDs agreed very well with the FRET assay (Table 1).

**Bruton's tyrosine kinase (BTK) inhibition TR-FRET (Time resolved FRET) assay**

This BTK competition assay measures compound potency ($IC_{50}$) for the inactivated state of Bruton's Tyrosine Kinase using FRET (Förster/ Fluorescence Resonance Energy Transfer) technology. The BTK - Eu complex was incubated on ice one hour prior to use at a starting concentration of 50 nM BTK-Bioease$^{Tm}$ : 10 nM Eu-streptavidin (Perkin-Elmer Catalog# AD0062). The assay buffer consisted of 20 mM HEPES (pH 7.15), 0.1mM DTT, 10mM $MgCl_2$, 0.5 mg/ml BSA with 3% Kinase Stabilizer (Fremont Biosolutions, Catalog # STB-K02). After 1h, the reaction mixture from above was diluted 10 fold in assay buffer to make 5 nM BTK: 1nM Eu-Streptavidin complex (donor fluorophore). 18μl of a mixture of 0.11 nM BTK-Eu and 0.11 nM Kinase Tracer 178 (Invitrogen, Catalog # PV5593,) with BTK-Eu alone as no negative control, was then dispensed into 384-well flat bottom plates (Greiner, 784076). Compounds to be tested in assay were prepared as 10x concentrations and serial dilution in half-log increments was performed in DMSO so as to generate 10 point curves. To initiate the FRET reaction, compounds prepared as 10x stock in DMSO was added to the plates and the plates were incubated 18-24h at 14°C.

After the incubation the plates were read on a BMG Pherastar Fluorescent plate reader (or equivalent) and used to measure the emission energy from the europium donor fluorophore (620 nm emission) and the FRET (665 nm emission). The negative control well values were averaged to obtain the mean minimum. The positive "no inhibitor" control wells were averaged to obtain the mean maximum. Percent of maximal FRET was calculated using following equation:

$$\%\ \text{max FRET} = 100 \text{ x } [(\text{FSR}_{\text{cmpd}} - \text{FSR}_{\text{mean min}}) / (\text{FSR}_{\text{mean max}} - \text{FSR}_{\text{mean min}})]$$

where FSR = FRET Signal ratio. % Max FRET curves were plotted in Activity Base (Excel) and the IC50 (%), hill slope, z' and %CV were determined. The mean $IC_{50}$ and standard deviation will be derived from duplicate curves (singlet inhibition curves from two independent dilutions) using Microsoft Excel.

Data from FRET indicated that reference Compound 1, reference Compound 2 and Compound 3 were very potent Btk inhibitors (Table 1).

**Table 1.** Biacore and FRET data for Compounds 1-3.

| | Biacore | | | FRET |
|---|---|---|---|---|
| **Compound** | **Ka (1/Ms)** | **Kd (1/s)** | **KD (M)** | **$IC_{50}$ (M)** |
| Compound 1 (reference) | $8.86 \times 10^5$ | $3.61 \times 10^{-4}$ | $4.08 \times 10^{-10}$ | $4.00 \times 10^{-10}$ |
| Compound 2 (reference) | $4.53 \times 10^5$ | $6.70 \times 10^{-4}$ | $1.48 \times 10^{-9}$ | $1.10 \times 10^{-9}$ |
| Compound 3 | $1.07 \times 10^6$ | $2.33 \times 10^{-4}$ | $1.19 \times 10^{-10}$ | $2.00 \times 10^{-10}$ |

**Effects of Compounds 1-3 on human mast cell activation**

One million human cord blood derived CD34+ hematopoietic stem cells (HSCs) from different donors (AllCells #CB008F-S, Emeryville, CA) were cultured for eight weeks in a serum-free complete medium (StemPro-34 with supplements; Invitrogen, Carlsbad, CA), with recombinant h-SCF (100 ng/ml) and h-IL6 (50 ng/ml). During the first week of culturing, recombinant h-IL3 (10 ng/ml) was also included to support HSCs differentiation. After 8 weeks of culture, cells were stimulated with recombinant h-IL-4 (10 ng/ml) for 5 days. Confirmation of the mast cell differentiation process was routinely done by FACS to check for c-kit and FcεRI expression; differentiated cells were routinely more than 90% c-kit positive, FcεRI positive.

Differentiated mast cells were sensitized with 0.1 μg/ml anti-NP IgE (Serotec, Raleigh, NC) overnight at 37°C. Cells were washed and then treated with Compound 1 for 1 hour at 37°C. After treatment, cells were cross-linked with 1 μg/ml NP(30)-BSA (Biosearch Technologies, Novato CA) for 30 minutes (for histamine and lipid mediator assessment) or overnight (for cytokine assessment). Culture supernatants were collected and assayed for histamine (Oxford Biomedical Research, Rochester Hills, MI), PGD2 and LTC4 (Cayman Chemical Company, Ann Arbor MI), and IL-5 and IL-13 (Bio-Rad Bio-Plex Pro cytokine quantification kit) release as per kits' instructions. Reference Compound 1, reference Compound 2 and Compound 3 reduced the release of these mediators into the supernatant in a concentration dependent manner up to 100% efficacy and the $IC_{50}$ values are reported in Table 2. Interestingly, treating the cells with a glucocorticoid did not attenuate any of these responses.

**Table 2.** $IC_{50}$ values for Compounds 1-3 effects on mast cell mediators released after high-affinity Fc epsilon receptor (FCεRI) activation.

| Compound | Histamine | PGD2 | LTC4 | IL-5 | 1L-13 | GM-CSF |
|---|---|---|---|---|---|---|
| Compound 1 (reference) | $1.47 \times 10^{-8}$ M | $6.31 \times 10^{-9}$ M | $4.28 \times 10^{-9}$ M | $7.32\times 10^{-9}$ M | $1.16 \times 10^{-8}$M | $3.77 \times 10^{-9}$ M |
| Compound 2 (reference) | $9.60 \times 10^{-9}$ M | $3.09 \times 10^{-9}$ M | N/A | $1.32 \times 10^{-8}$ M | $9.19 \times 10^{-8}$ M | $2.60 \times 10^{-8}$ M |
| Compound 3 | $2.17 \times 10^{-8}$ M | $5.31 \times 10^{-9}$ M | N/A | $8.67 \times 10^{-8}$ M | $9.74 \times 10^{-8}$ M | $2.86 \times 10^{-8}$ M |
| *N = 3 donors* | | | | | | |

## Effects of Compounds 1-3 on human B cell antibody production

Human total B cells were enriched with RosetteSep human B cell enrichment cocktail (#28921, Vancouver, BC) from buffy coat leukocyte packs (New York Blood Center) following manufacturer's protocol. Enriched B cell purity (around 80%) was checked by FACS with CD19+ staining. B cells were suspended (0.1 million cells/well/100 μl) in RPMI-1640 based conditional medium (50 ng/ml IL-2, 50ng/ml IL-10, and 1 μg/ml anti-IgD for the activation of B cells to produce IgG/IgM; 10 ng/ml IL-4, 10 ng/ml IL-10, 25 ng/ml IL-21, and 1 μg/ml anti-CD40 for the activation of B cells to produce IgE) together with reference Compound 1 (1 nM - 10 μM). Cells were cultured for 10 days (for IgG/IgM production) or 14 days (for IgE production) at 37°C. Culture supernatants were collected for IgM, IgG, and IgE analysis following Bethyl Laboratory's protocol (#E80-104, #E80-100, #E80-108, Montgomery, TX). Reference Compound 1 reduced the levels of IgM, IgG, and IgE measured in the supernatant in a concentration dependent manner up to 100% efficacy and the IC50 values are reported in Table X. Interestingly, treating the cells with a glucocorticoid did not attenuate the IgM or IgG responses and actually induced a significant increase in IgE levels in the supernatant consistent with previous reports [Zieg et al. JACI, 1994, 94: 222; Hemady et al. JACI 1985, 75:304; Wu et al. JCI 1991, 87: 870]. Reference Compound 2 and Compound 3 had similar inhibitory effects on IgM and IgG production (Table 3).

**Table 3.** $IC_{50}$ values for Effects of Compounds 1-3 on B cell antibody production after B cell receptor (BCR) activation.

| Compound | IgM | IgG | IgE |
|---|---|---|---|
| Compound 1 (reference) | $1.21 \times 10^{-9}$ M | $4.15 \times 10^{-9}$ M | $1.47 \times 10^{-9}$ M |
| Compound 2 (reference) | $3.28 \times 10^{-9}$ M | $9.26 \times 10^{-9}$ M | N/A |
| Compound 3 | $4.76 \times 10^{-9}$ M | $3.17 \times 10^{-9}$ M | N/A |
| *N = 3 donors* | | | |

## Effects of Compound 1-3 in a model of allergic airway disease

The mouse model of ovalbumin-induced allergic airway disease was employed to assess the effects of Compound 1 on allergen-induced bronchoconstriction and allergen-induced airway inflammation. Briefly, mice (male; BALB/c; 7 -9 weeks of age) were immunized i.p. with 10 μg of ovalbumin (OVA) in 0.2 ml of Alum (2% Al(OH)3 in water, Serva, Heidelberg, Germany) on days 0 and 14. Control animals received Alum only. On days 21-23, animals were exposed for 20 min to a nebulized (Proneb Ultra II, PARI Respiratory Equipment, Midlothian, VA,) aerosol of 1% OVA (10 mg/ml) in phosphate-buffered saline (PBS), to establish the inflammatory process within the lung; or PBS alone as a control. For studies where intranasal dosing was used, mice were anesthetized with isoflurane and intranasally (i.n.) administered vehicle (20% propylene glycol) or reference Compound 1 (0.3 mg/kg) one hour prior to each aerosolized OVA challenge.

For studies using inhalation delivery, reference Compound 1 was micronized by a jet mill (MC One Jet Mill, Jetpharma USA Inc., South Plainfield, NJ) and the dry powder aerosol was generated using a Wright dust feed dry powder aerosol generator. The micronized drug powder was packed into a cylindrical reservoir using a hydraulic press at approximately 1000 psi to produce compacted cakes of powder used as input by the Wright dust feed. The dust feed creates and aerosol that is passed through a sonic nozzle for de-agglomeration and into a cyclone to remove non-respirable particles and agglomerates. The cyclone output passed into the TSE nose-only rodent exposure system. Dose groups of mice were exposed to the aerosol test atmosphere for 5, 15, or 45 minutes. The TSE had 24 ports for animals and 3 sampling ports for sampling: real time aerosol concentration (Microdust pro), gravimetric aerosol concentration (absolute filter), and particle size (cascade impactor). During exposure the animals were restrained in glass tubes designed for the anatomy of mice. The exposure system was qualified for spatial uniformity and temporal stability by an air mass balance. The air flow to each port and the port to port flow rates varied by less than 2% with the input flow rate set at 17 liters per minute. The particle size distribution for each exposure was evaluated by collecting aerosol samples with an eight stage cascade impactor. Aerosol was sampled by the cascade impactor at an airflow rate of 1 liter per minute for the 45 minutes. Impactor data were mathematically evaluated to determine the Mass Median Aerodynamic Diameter (MMAD) and Geometric Standard Deviation (GSD) of 6-tert-Butyl-2-[2-hydroxymethyl-3-(5-{5-[(2-methoxy-ethylamino)-methyl]-pyridin-2-ylamino}-1-methyl-6-oxo-1,6-dihydro-pyridin-3-yl)-phenyl]-3,4-dihydro-2H-isoquinolin-1-one using graphical analysis of an assumed lognormal distribution. The estimated deposited dose of reference Compound 1 was calculated using a minute volume for the mouse of 0.026 liters per minute and the pulmonary deposition fraction was determined from the MMAD. Mice were dosed 45 minutes prior to exposure to aerosolized or intravenously administered OVA.

To assess reference Compound 1's effects on acute bronchoconstriction, control (Alum injected and saline challenged) and OVA sensitized and challenged mice were anesthetized with 150 mg/kg pentobarbital i.p., surgically prepared with a tracheal cannula, and mechanically ventilated on a computer controlled respirator (flexiVent, SCIREQ Inc.; tidal volume = 10 ml/kg; respiratory rate of 150 breaths/min; 3 cmH2O positive end-expiratory pressure). Respiratory system resistance (*R*) was measured (SnapShot150 perturbation) before and every 10 seconds for 7 minutes after injection into the tail vein of 100 μl of 7.5 mg/ml (30 mg/kg) ovalbumin in 0.9% saline. Data was digitally recorded using flexiWare software (version 7.1). The difference between baseline and peak antigen-induced *R* was calculated as the percent increase in *R,* and reported in Table 2 as a percent reduction of OVA-induced acute bronchoconstriction. In the two studies reported here, i.v. administration of OVA induced a ~60-80% increase in *R*, which was effectively inhibited by reference Compound 1 when administered i.n. or delivered as a dry powder formulation by inhalation.

**Table 4.** Effects of Compounds 1-3 on allergen-induced bronchoconstriction.

| Compound | Route of administration | Delivered Dose (mg/kg) | Deposited Dose (mg/kg) | % reduction in airway resistance (*R*) | Exp. No |
|---|---|---|---|---|---|
| Compound 1 (reference) | Intranasal | 0.3 | 0.3 | 85* | mOVA20 |
| Compound 1 (reference) | Inhaled | 1.0 | 0.03 | 44 | mOVA36 |
| Compound 1 (reference) | Inhaled | 2.7 | 0.1 | 59* | mOVA36 |
| Compound 1 (reference) | Inhaled | 8.6 | 0.3 | 86* | mOVA36 |
| Compound 2 (reference) | Intranasal | 0.3 | 0.3 | 53 | mOVA50 |
| Compound 2 (reference) | Intranasal | 1.0 | 1.0 | 64* | mOVA50 |

(continued)

| Compound | Route of administration | Delivered Dose (mg/kg) | Deposited Dose (mg/kg) | % reduction in airway resistance (*R*) | Exp. No |
|---|---|---|---|---|---|
| Compound 2 (reference) | Intranasal | 3.0 | 3.0 | 105* | mOVA50 |
| Compound 3 | Intranasal | 0.3 | 0.3 | 77* | mOVA49 |
| Compound 3 | Intranasal | 1.0 | 1.0 | 83* | mOVA49 |
| Compound 3 | Intranasal | 3.0 | 3.0 | 93* | mOVA49 |

To study the effects of reference Compound 1 on airway inflammation, after the three 1% OVA challenges, a final OVA challenge was given as an aerosol solution of 5% OVA in PBS (50 mg/ml) or PBS alone for 20 min. Mice are anesthetized with pentobarbital (75 μl of 50 mg/ml solutions: 150 mg/kg) 24 hours after the 5% OVA challenge, and surgically prepared with a tracheal cannula (18 gauge Angiocath, Becton Dickinson, Sandy, Utah, USA). The lungs were lavaged and airway inflammation was evaluated as previously described by performing total and differential cell counts [Harris et al., 2012, Mucosal Immunology]. Data are reported in Table 3 as a percent reduction of OVA-induced airway eosinophilia. In the study reported here, aerosolized OVA induced a 288% increase in the numbers of eosinophils recovered in the lavage fluid, which was effectively inhibited by reference Compound 1 delivered as a dry powder formulation by inhalation.

**Table 5.** Effect of reference Compound 1 on allergen-induced airway eosinophilia.

| Compound | Route of administration | Delivered Dose (mg/kg) | Deposited Dose (mg/kg) | % reduction in airway eosinophilia | Exp. no |
|---|---|---|---|---|---|
| Compound 1 (reference) | Inhaled | 0.09 | .003 | 82* | mOVA40 |
| Compound 1 (reference) | Inhaled | 0.27 | .010 | 83* | mOVA40 |
| Compound 1 (reference) | Inhaled | 0.81 | .030 | 84* | mOVA40 |

**Reference Compound 1's effect in a model of steroid-resistant airway inflammation**

Reference Compound 1's effects on airway inflammation induced by a synthetic form of doublestranded RNA, polyinosine-polycytidylic acid (Poly I:C) were assessed. The model employed is meant to mimic the effects of an acute viral infection, which has been shown to elicit a steroid-resistant airway neutrophilia [Harris et al., 2012, Mucosal Immunology]. To perform this experiment, mice (male; BALB/c; 7 -9 weeks of age) were anesthetized with isoflurane and intranasally (i.n.) administered saline or poly I:C (30 μg) and airway inflammation was evaluated 24 hours later as previously described [Harris et al., 2012, Mucosal Immunology]. Compound 1 inhibited poly I:C-induced airway neutrophilia in a dose-dependent (Table 6). These reductions in neutrophils were consistent with reductions in the levels of neutrophil chemoattractants in the lavage fluid (Table 4).

**Table 6.** Effect of reference Compound 1 on Poly I:C acid induced airway neutrophilia.

| Compound | Route of administration | Delivered Dose (mg/kg) | Deposited Dose (mg/kg) | % reduction in airway neutrophilia | Exp. no |
|---|---|---|---|---|---|
| Compound 1 (reference) | Inhaled | 0.09 | .003 | 17 | mPolyIC-3-12 |
| Compound 1 (reference) | Inhaled | 0.27 | .010 | 22 | mPolyIC-3-12 |
| Compound 1 (reference) | Inhaled | 0.81 | .030 | 67* | mPolyIC-3-12 |

(continued)

| Compound | Route of administration | Delivered Dose (mg/kg) | Deposited Dose (mg/kg) | % reduction in airway neutrophilia | Exp. no |
|---|---|---|---|---|---|
| Compound 1 (reference) | Inhaled | 8.6 | 0.3 | 83* | mPolyIC-5-12 |

DMPK Data

The pharmacokinetics of reference Compound 1 in rats, dogs and cynomolgus monkeys (cynos) were characterized by low oral bioavailability and high plasma clearance (Table 7). In mice, it was characterized by moderate plasma clearance and low oral bioavailability. The pharmacokinetics of reference Compound 2 and compound 3 in rats and dogs were characterized by low oral bioavailability and high plasma clearance (Table 8 and 9).

**Table 7. Key** pharmacokinetic data for reference Compound 1

| Parameter | Rat | Mouse | Dog | Cyno |
|---|---|---|---|---|
| CL (mL/min/kg) | 75.8 | 26 | 28.3 | 38.9 |
| Oral Bioavailability (%) | 2 | 12 | 1.5 | 8 |
| Doses (mg/kg) | 1.0 (iv), 2.0 (po) | 1.0 (iv), 10 (po) | 1.0 (iv), 1.0 (po) | 0.3 (iv), 0.9 (po) |

**Table 8. Key** pharmacokinetic data for reference Compound 2

| Parameter | Rat | Dog |
|---|---|---|
| CL (mL/min/kg) | 84.9 | 35 |
| Oral Bioavailability (%) | <2 | 4 |
| Doses (mg/kg) | 0.5 (iv), 2 (po) | 1.0 (iv), 1.0 (po) |

**Table 9. Key** pharmacokinetic data for Compound 3

| Parameter | Rat | Dog |
|---|---|---|
| CL (mL/min/kg) | 111.2 | 31.4 |
| Oral Bioavailability (%) | 7 | 8 |
| Doses (mg/kg) | 0.5 (iv), 2 (po) | 1.0 (iv), 1.0 (po) |

The foregoing application has been described in some detail by way of illustration and example, for purposes of clarity and understanding.

## Claims

1. A formulation comprising micronized 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isoquinolin-1-one and micronized lactose.

2. A compound of Formula I,

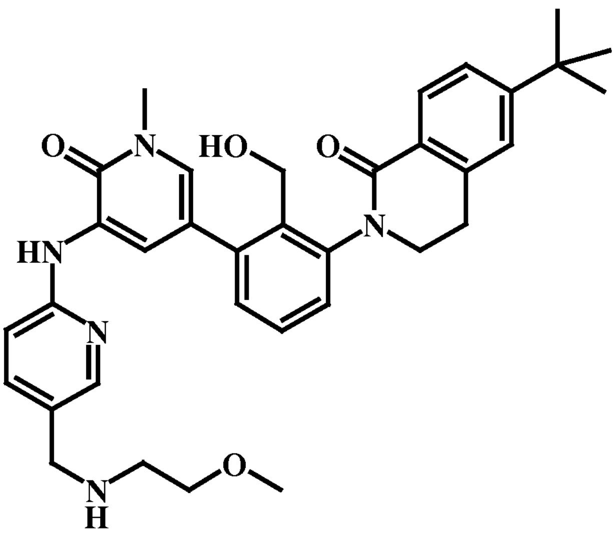

I

or a pharmaceutically acceptable salt thereof.

## Patentansprüche

1. Formulierung umfassend mikronisiertes 6-tert-Butyl-2-[2-hydroxymethyl-3-(5-{5-[(2-methoxy-ethylamino)-methyl]-pyridin-2-ylamino}-6-oxo-1,6-dihydro-pyridin-3-yl)-phenyl]-3,4-dihydro-2H-isochinolin-1-on und mikronisierte Laktose.

2. Verbindung der Formel I

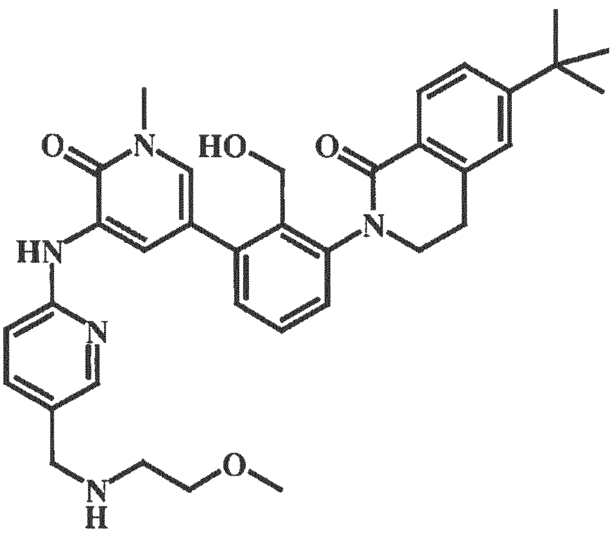

I

oder ein pharmazeutisch annehmbares Salz davon.

## Revendications

1. Formulation comprenant de la 6-tert-butyl-2-[2-hydroxyméthyl-3-(5-{5-[(2-méthoxy-éthylamino)-méthyl]-pyridin-2-ylamino}-6-oxo-1,6-dihydro-pyridin-3-yl)-phényl]-3,4-dihydro-2H-isoquinoléin-1-one micronisée et du lactose micronisé.

2. Composé de formule I,

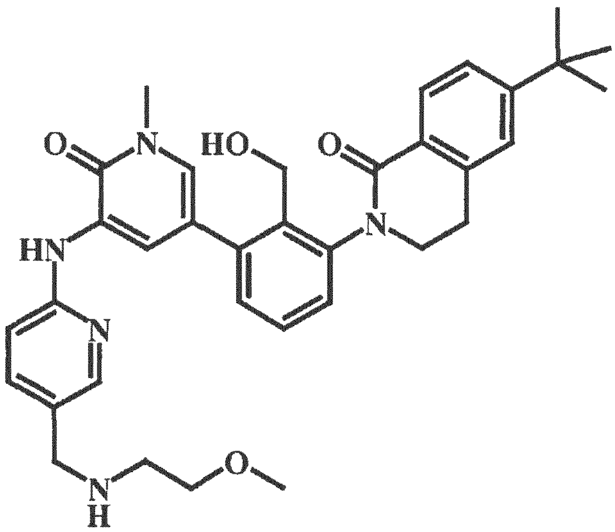

I

ou un sel pharmaceutiquement acceptable de celui-ci.

Figure 1

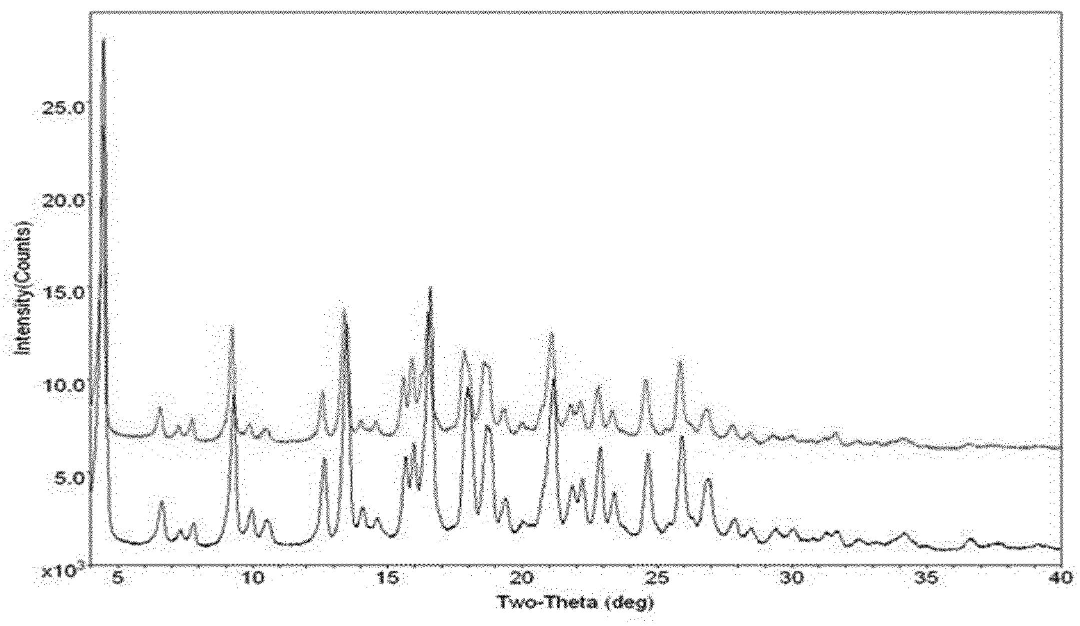
25.0
20.0
15.0
10.0
5.0
x10^3
Intensity(Counts)
5
10
15
20
25
30
35
40
Two-Theta (deg)

Figure 2

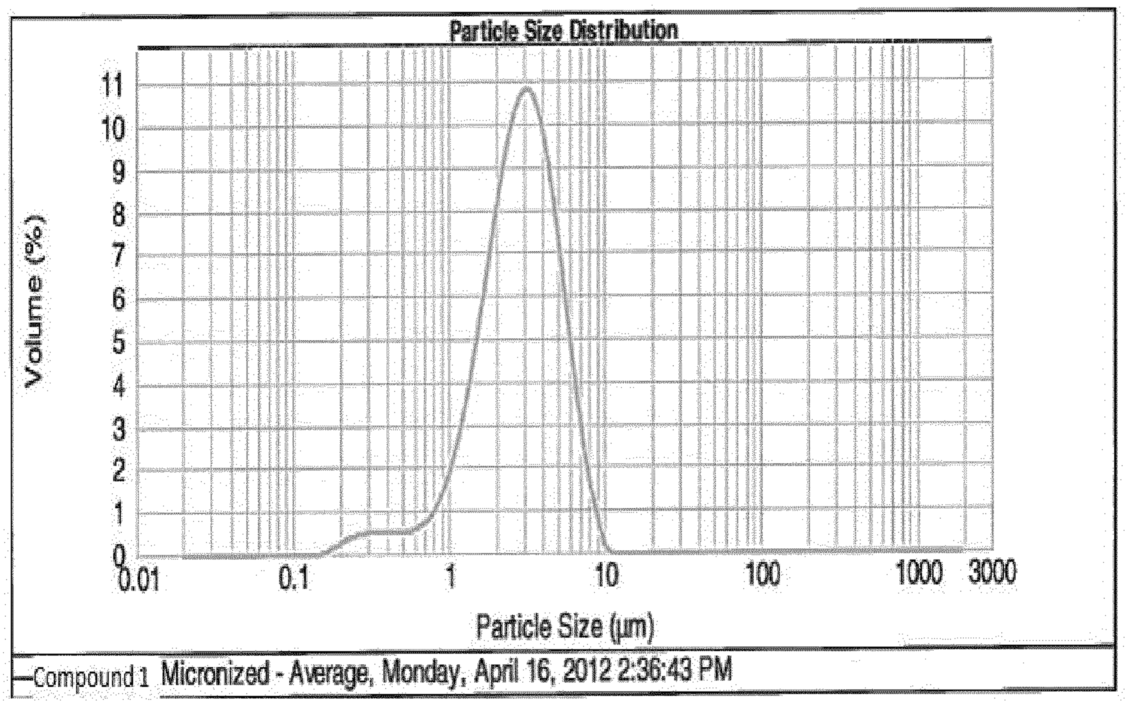
Particle Size Distribution
Volume (%)
11
10
9
8
7
6
5
4
3
2
1
0
0.01
0.1
1
10
100
1000
3000
Particle Size (µm)
Compound 1 Micronized - Average, Monday, April 16, 2012 2:36:43 PM

**Figure 3**

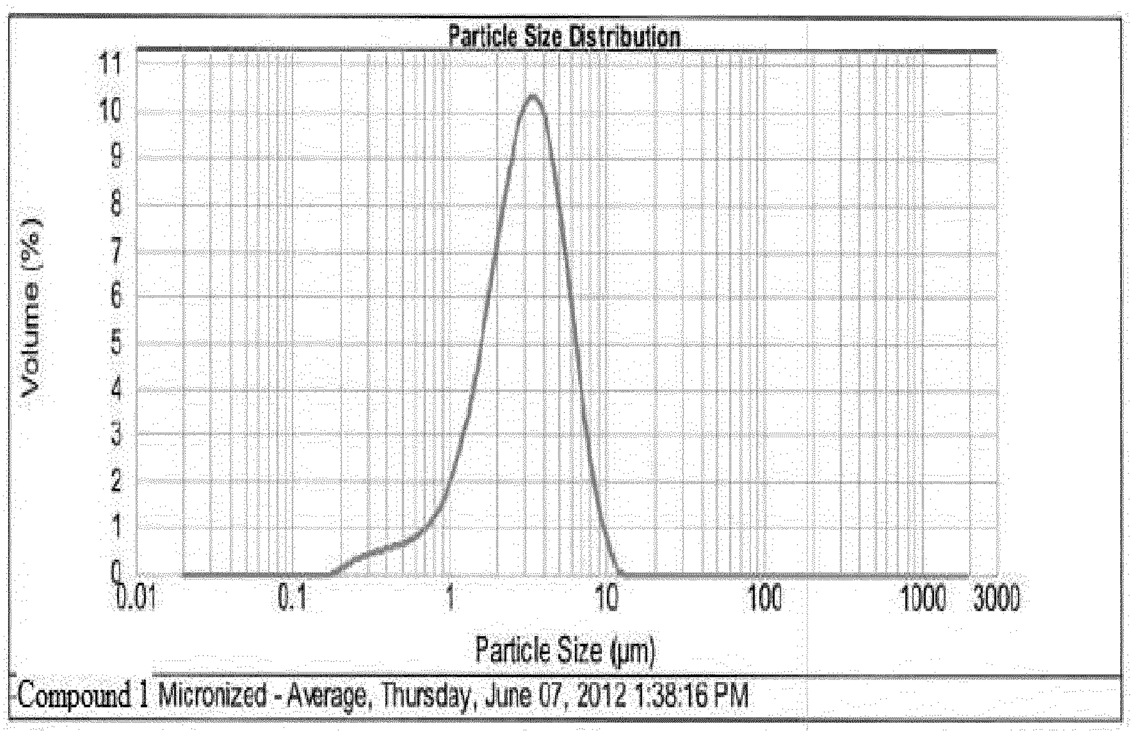
Particle Size Distribution
Volume (%)
0
1
2
3
4
5
6
7
8
9
10
11
0.01
0.1
1
10
100
1000
3000
Particle Size (µm)
Compound 1 Micronized - Average, Thursday, June 07, 2012 1:38:16 PM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011140488 A1
- WO 9632099 A
- US 6119853 A
- US 6179118 B
- US 6315112 B
- US 6352152 B
- US 6390291 B
- US 6679374 B
- US 6360739 B
- US 6431168 B


### Non-patent literature cited in the description


- **T. HUNTER.** *Cell,* 1987, vol. 50, 823-829
- **RASTETTER et al.** *Annu Rev Med,* 2004, vol. 55, 477
- **KHAN et al.** *Immunity,* 1995, vol. 3, 283
- **ELLMEIER et al.** *J. Exp. Med.,* 2000, vol. 192, 1611
- **ROSEN et al.** *New Eng. J. Med.,* 1995, vol. 333, 431
- **LINDVALL et al.** *Immunol. Rev.,* 2005, vol. 203, 200
- **JANSSON ; HOLMDAHL.** *Clin. Exp. Immunol.,* 1993, vol. 94, 459
- **Z. PAN et al.** *Chem. Med Chem.,* 2007, vol. 2, 58-61
- **IWAKI et al.** *J. Biol. Chem.,* 2005, vol. 280, 40261
- **IYER et al.** *J. Biol. Chem.,* 2011, vol. 286, 9503-13
- **HATA et al.** *J. Exp. Med.,* 1998, vol. 187, 1235-47
- **MACGLASHAN et al.** *Int. Immunopharmacol,* 2011, vol. 11, 475-9
- **WU et al.** *Am J Respir Crit Care Med,* 2008, vol. 178, 1123-1129
- **JOHNSTON et al.** *Br. Med. J.,* 1995, vol. 310, 1225-1229
- **HAMMAD et al.** *Nat Med.,* 2009, vol. 15 (4), 410-6
- **LIU et al.** *Nat Immunol.,* 2011, vol. 12 (5), 416-24
- **LEE et al.** *Proc Natl Acad Sci U S A.,* 2012, vol. 109 (15), 5791-6
- **HORWOOD et al.** *J Exp Med,* 2003, vol. 197, 1603
- **ISLAM ; SMITH.** *Immunol. Rev.,* 2000, vol. 178, 49
- **FELDHAHN et al.** *J. Exp. Med.,* 2005, vol. 201, 1837
- **GOODMAN ; GILMAN'S.** The Pharmacological Basis of Therapeutics. McGraw Hill Companies Inc, 2001
- **TAYAB et al.** *Expert Opin. Drug Deliv.,* 2005, vol. 2 (3), 519-532
- **SELBY et al.** *Future Med. Chem.,* 2011, vol. 3 (13), 1679-1701
- **FORBES et al.** *Adv Drug Del. Rev,* 2011, vol. 63, 69-87
- Determination of the deposition of the emitted dose in pressurized inhalations using apparatus A. *British Pharmacopaeia,* 1988, 204-207
- Nomenclature in Organic Chemistry. **J. RIGAUDY ; D. P. KLESNEY.** IUPAC. Pergamon Press, 1979
- **ZIEG et al.** *JACI,* 1994, vol. 94, 222
- **HEMADY et al.** *JACI,* 1985, vol. 75, 304
- **WU et al.** *JCI,* 1991, vol. 87, 870
- **HARRIS et al.** *Mucosal Immunology,* 2012